# EUROPEAN PATENT APPLICATION

(11) **EP 4 461 274 A1**
(43) Date of publication of application: **13.11.2024**
(21) Application number: 22918201.9
(22) Date of filing: 31.08.2022
(51) Int. Cl.: A61F 5/08, A61M 16/06, A61M 16/00

(54) **NASAL DILATOR HAVING EXPIRATORY AIRWAY POSITIVE PRESSURE VALVE MEMBER**

(30) Priority: 06.01.2022 US 202263296865 P
(71) Applicant: Somnics, Inc., Zhubei, Hsinchu 30261 (TW)
(72) Inventor: CHEN, Chung-Chu, Zhubei Hsinchu, Taiwan 30261 (CN); LEE, Chih-Jung, Zhubei Hsinchu, Taiwan 30261 (CN); YOUNG, Chieh-Neng, Zhubei Hsinchu, Taiwan 30261 (CN); YU, Tung-Ming, Zhubei Hsinchu, Taiwan 30261 (CN)
(74) Representative: Charrier Rapp & Liebau
(86) International application number: PCT/CN2022/116223
(87) International publication number: WO 2023/130746

(57) **Abstract**

A nasal dilator for treating breathing problem is provided, which includes a hollow nasal dilator body and a one-way flow resistance unit. The hollow nasal dilator body has a first end opening and a second end opening opposing the first end opening. The hollow nasal dilator body longitudinally extends from the first end opening to the second end opening and being adapted for insertion into a user's nostril with the second end opening placed within the user's nasal passage so that the hollow nasal dilator body supports and dilates the nasal passage. The one-way flow resistance unit is assembled to the hollow nasal dilator body and in proximity to the first end opening and away from the second end opening. The one-way flow resistance unit creates an exhalation flow resistance higher than an inhalation flow resistance such that an expiratory positive airway pressure is generated within the nasal passage.

## Description

### Technical Field

This invention relates to a device for treating sleep apnea and snoring, and more particularly to a device for generating expiratory positive airway pressure.

### Technical Background

Sleep apnea is a condition in which repeated collapses in the patient's airway during inhalation causes a cessation of breathing during sleep. During inhalation, air pressure in the lungs and respiratory passages is reduced. If during this time, the tone of the muscles in the upper-airway is reduced, the airway tends to collapse. As the airway begins to occlude prior to an apnea episode, the patient often begins to snore. Snoring is an effort to try to combat the collapsed airway. Obstructive sleep apnea is the most common type of sleep apnea. When the patient sleeps, the muscles of the airway relax and collapse, blocking the airway, making breathing difficult or apnea. Patients will interrupt their sleep due to apnea, resulting in poor sleep quality, which will affect their mental status during the day. Patients will feel drowsiness, fatigue and inability to concentrate during the day, which may even cause traffic accidents.

Expiratory positive airway pressure (EPAP) is an emerging treatment for sleep apnea. It works similarly to continuous positive airway pressure (CPAP) but only provides positive pressure on the exhale. Unlike CPAP requiring a bulky and noisy machinery, EPAP utilizes a small and lightweight device with a disposable one-way valve affixed over the nostrils with adhesive. The valve allows air entering the nasal cavity on inhaling while preventing the air inside the nasal cavity from passing through it on exhaling to cause an exhalation flow resistance higher than an inhalation flow resistance such that an expiratory positive airway pressure is generated within the nasal passage to open the upper airway. Therefore, the user's upper airway could maintain patency during sleep. EPAP device does not need electricity to work, but it controls the air flow direction inside the nasal passage via a one-way valve to help a positive pressure naturally generated inside the user's passage on exhaling. The positive pressure helps the user's upper airway keep patency on a subsequent inhaling. Therefore, the user could have easy breathing during sleep.

This invention aims to providing a nasal dilator with an EPAP valve insert to practice the technical means for producing an expiratory positive airway pressure.

### Summary of the Invention

According to one object of this invention, a nasal dilator with an EPAP valve insert is provided. The nasal dilator includes a hollow nasal dilator body and a one-way flow resistance unit. The hollow nasal dilator body has a first end opening and a second end opening opposing the first end opening and longitudinally extending from the first end opening to the second end opening. The hollow nasal dilator body is adapted for insertion into a user's nostril with the second end opening placed within the user's nasal passage so that the hollow nasal dilator body supports and dilates the nasal passage. The one-way flow resistance unit is assembled to the hollow nasal dilator body in proximity to the first end opening and away from the second end opening. The one-way flow resistance unit creates an exhalation flow resistance higher than an inhalation flow resistance such that an expiratory positive airway pressure is generated within the nasal passage.

In an implementation of this invention, the one-way flow resistance unit of the nasal dilator with an EPAP valve insert is removable and interchangeable from the nasal dilator.

In an implementation of this invention, the one-way flow resistance unit of the nasal dilator with an EPAP valve insert is able to adjust a ratio of the exhalation flow resistance to the inhalation flow resistance.

In an implementation of this invention, the one-way flow resistance unit of the nasal dilator with an EPAP valve insert comprises a one-way flow resistance valve and a valve seat, the one-way flow resistance valve is fastened in the valve seat, and the valve seat is assembled to the hollow nasal dilator body.

In an implementation of this invention, the one-way flow resistance valve of the nasal dilator with an EPAP valve insert has at least one radial cut.

In an implementation of this invention, the one-way flow resistance valve of the nasal dilator with an EPAP valve insert has at least one vent spaced from the radial cut.

In an implementation of this invention, the one-way flow resistance valve of the nasal dilator with an EPAP valve insert has at least one slit vent in a radial direction.

In an implementation of this invention, the one-way flow resistance valve of the nasal dilator with an EPAP valve insert has at least one slit vent in a radial direction and a cut between the slit vent and a periphery of the one-way flow resistance valve.

In an implementation of this invention, the one-way flow resistance valve of the nasal dilator with an EPAP valve insert has a multiple of slit vents spaced from each other and arranged along at least one radial direction.

In an implementation of this invention, the one-way flow resistance valve of the nasal dilator with an EPAP valve insert has a multiple of slit vents spaced from each other and arranged along at least one radial direction and a cut between at least one pair of the neighboring slit vents.

In an implementation of this invention, both of the one-way flow resistance valve and the valve seat of the nasal dilator with an EPAP valve insert are in a shape of a three-dimensional trapezoid.

In an implementation of this invention, the hollow nasal dilator body of the nasal dilator with an EPAP valve insert has at least one lateral vent in proximity to the first end opening.

In an implementation of this invention, the hollow nasal dilator body of the nasal dilator with an EPAP valve insert has a flow resistance structure unit in proximity to the first end opening.

In an implementation of this invention, the hollow nasal dilator body of the nasal dilator with an EPAP valve insert has a flow resistance structure unit in proximity to the first end opening, and the valve seat of the nasal dilator has a rotatable flow resistance adjustment unit with capability to change relative orientation between the flow resistance adjustment unit and the flow resistance structure unit to adjust the exhalation flow resistance.

In an implementation of this invention, the nasal dilator with an EPAP valve insert further has a rotatable baffle assembled between the valve seat and the one-way flow resistance valve for blocking a partial area of the vent.

In an implementation of this invention, the nasal dilator with an EPAP valve insert further has at least one stopper positioned at an inner surface of the hollow nasal dilator body in proximity to the first end opening to confine a bending angle of the one-way flow resistance valve upon a force from an inhalation flow.

In an implementation of this invention, the nasal dilator with an EPAP valve insert further has a stent assembled under the one-way flow resistance valve and connected to an inner surrounding wall of the hollow nasal dilator body, and the stent crosses a part or the whole of an inner radius of the hollow nasal dilator body.

In an implementation of this invention, the stent of the nasal dilator with an EPAP valve insert has one of the following configurations: cross shape, X shape, T shape and a singular rib.

In an implementation of this invention, the one-way flow resistance unit having its individual flow resistance is made with an individual color, or mapping to an individual color code.

In an implementation of this invention, an easy-to-remove means is provided along an inner surrounding wall of the hollow nasal dilator body in proximity to the second end opening to facilitate removal of a part of the hollow nasal dilator body via the easy-to-remove means.

In an implementation of this invention, the nasal dilator with an EPAP valve insert has at least one sensing unit for detecting temperature, humidity, air flow pressure, breathing pattern, vibration, snoring and chemical materials inside the user's nasal passage.

In one aspect, this invention provides a nasal dilator assembly comprising a pair of present nasal dilators and a jointing piece connected between the pair of nasal dilators.

In an implementation of this invention, the one-way flow resistance unit of each of the nasal dilators is able to adjust a ratio of the exhalation flow resistance to the inhalation flow resistance.

In one another aspect, this invention provides a negative pressure sleep therapy system comprising the present nasal dilator assembly, an electric console, an oral interface, and a sensing unit; wherein the nasal dilator assembly is worn by a user's nose in order that an expiratory positive airway pressure is generated inside the user's nasal passage, the electric console provides a negative pressure via the oral interface delivering to the user's oral cavity such that a negative pressure environment is generated inside the user's oral cavity, the sensing unit detects at least one user's physiological signal including temperature, humidity, air flow pressure, breathing pattern, vibration, snoring and chemical materials inside the user's nasal passage, and the sensing unit transmits the detected physiological signal to the electric console for computation with an algorithm, and the electric console accordingly adjusts the negative pressure provided and then via the oral interface to control the pressure inside the user's oral cavity.

### Brief Description of the Drawings

The implementation of this invention is not confined to sizes and dimension ratios of the structures shown in the drawings.
Fig. 1A through Fig. 1C are various schematic views of a nasal dilator with an EPAP valve insert according to a first embodiment of this invention.
Fig. 2 schematically shows a use state of the nasal dilator with an EPAP valve insert according to the first embodiment of this invention.
Fig. 3A and Fig. 3B respectively show an inhalation flow direction and an exhalation flow direction of the nasal dilator with an EPAP valve insert of the first embodiment of this invention.
Fig. 4A through Fig. 4H are various schematic views of a nasal dilator with an EPAP valve insert according to a second embodiment of this invention.
Fig. 5A and Fig. 5B respectively show an inhalation flow direction and an exhalation flow direction of the nasal dilator with an EPAP valve insert of the second embodiment of this invention.
Fig. 5C is a schematic partial cross-sectional perspective view of the nasal dilator with an EPAP valve insert according to the second embodiment of this invention showing an exhalation flow direction.
Fig. 6A through Fig. 6H are various schematic views of a nasal dilator with an EPAP valve insert according to a third embodiment of this invention.
Fig. 7A shows an inhalation flow direction of the nasal dilator with an EPAP valve insert of the third embodiment of this invention; Fig. 7B and Fig. 7C show an exhalation flow direction of the nasal dilator with an EPAP valve insert of the third embodiment of this invention.
Fig. 8A shows another schematic perspective view of the nasal dilator with an EPAP valve insert of the third embodiment of this invention; Fig. 8B shows schematic perspective traverse sectional view of the nasal dilator with an EPAP valve insert of the third embodiment of this invention.
Fig. 9A through Fig. 9H are various schematic views of a nasal dilator with an EPAP valve insert according to a fourth embodiment of this invention.
Fig. 10A through Fig. 10H are various schematic views of a nasal dilator with an EPAP valve insert according to a fifth embodiment of this invention.
Fig. 11A through Fig. 11G are various schematic views of a nasal dilator with an EPAP valve insert according to a sixth embodiment of this invention.
Fig. 12A through Fig. 12H are various schematic views of a nasal dilator with an EPAP valve insert according to a seventh embodiment of this invention.
Fig. 13A through Fig. 13H are various schematic views of a nasal dilator with an EPAP valve insert according to an eighth embodiment of this invention.
Fig. 14A and Fig. 14B show respective variances of the nasal dilator with an EPAP valve insert of the fifth embodiment of this invention.
Fig. 15A shows a variance of the vale seat and the one-way flow resistance valve of the second embodiment of this invention; Fig. 15B shows a schematic perspective view of the valve seat of the variance; Fig. 15 shows a variance of the one-way flow resistance valve of the second embodiment of this invention; Fig. 15D shows a schematic assembled view of the one-way flow resistance valve and the valve seat of Fig. 15C.
Fig. 16A shows a variance of the valve seat and the one-way flow resistance valve of the nasal dilator with an EPAP valve insert according to the fifth embodiment of this invention.
Fig. 16B is a schematic view of a one-way flow resistance valve of Fig. 16A and Fig. 16D is a schematic bottom view of the one-way flow resistance valve of Fig. 16B.
Fig. 16C, Fig. 16E and Fig. 16F show respective variances of the one-way flow resistance valve of Fig. 16B.
Fig. 17A and Fig. 17B illustrate respective use states of the rotatable baffle of the fourth embodiment and fifth embodiment of this invention.
Fig. 18A and Fig. 18B illustrate an error-proofing mechanism of the nasal dilator with an EPAP valve insert of this invention.
Fig. 19 illustrates the nasal dilator with an EPAP valve insert of this invention having different materials.
Fig. 20A through Fig. 20H are various schematic views of a nasal dilator with an EPAP valve insert according to a ninth embodiment of this invention.
Fig. 21A through Fig. 21G show various schematic views of a first variance of the ninth embodiment of this invention.
Fig. 22A through Fig. 22H are various schematic views of a nasal dilator with an EPAP valve insert according to a tenth embodiment of this invention.
Fig. 22I shows a schematic assembled perspective view of the valve seat and one-way flow resistance valve of the nasal dilator with an EPAP valve insert of the tenth embodiment of this invention.
Fig. 23A shows an inhalation flow direction of the nasal dilator with an EPAP valve insert of the tenth embodiment of this invention; Fig. 23B and Fig. 23C show an exhalation flow direction of the nasal dilator with an EPAP valve insert of the tenth embodiment of this invention.
Fig. 24A through Fig. 24F show various schematic views of a second variance of the ninth embodiment of this invention.
Fig. 24G shows an inhalation flow direction of the second variance of the ninth embodiment.
Fig. 25A through Fig. 25F show various schematic views of a third variance of the ninth embodiment of this invention.
Fig. 25G shows an inhalation flow direction of the third variance of the ninth embodiment.
Fig. 26A through Fig. 26F show various schematic views of a fourth variance of the ninth embodiment of this invention.
Fig. 26G shows an inhalation flow direction of the fourth variance of the ninth embodiment.
Fig. 27A shows a schematic cross-sectional view of a nasal dilator with an EPAP valve insert according to an eleventh embodiment of this invention; Fig. 27B shows a schematic exploded view of Fig. 27A.
Fig. 27C shows a schematic view of the use state of the nasal dilator with an EPAP valve insert according to the eleventh embodiment of this invention.
Fig. 28A shows a schematic cross-sectional view of a variance of the eleventh embodiment; Fig. 28B shows a schematic exploded view of Fig. 28A.
Fig. 29A and Fig. 29B show applications of the nasal dilator with an EPAP valve insert of this invention.

### Numeral Reference Description:

1, 2, 3, 4, 5, 6, 7, 8, 9, 10, 11, 12, 130 nasal dilator with an EPAP valve insert
100, 200, 250, 300, 400, 600, 900 nasal dilator unit
110a, 120a first hollow nasal dilator body
110b, 120b second hollow nasal dilator body
111, 121 rear cover
112a first one-way flow resistance valve
112b second one-way flow resistance valve
113, 123, 1100, 1410a, 1410b, 1500, 1540, 1600, 820, 2100, 2510, 3100, 4100, 6100, 9100 valve seat
114, 124 filter
115, 125 front cover
116, 126 mesh cover
117a, 127a first exit flow adjuster
117b, 127b second exit flow adjuster
122, 1200, 1420a, 1420b, 1520, 1550, 1620, 1640, 1660, 1680, 2200, 2520, 3200, 4200, 6200, 9200, 9200b, 9200c, 9200d one-way flow resistance valve
1001, 2001, 2501, 3001, 4001, 5001, 9001, 9001a, 9001b, 9001d first end opening
1001a, 1103, 2004d, 3004d step surface
1002, 2002, 2502, 3002, 4002, 5002, 9002, 9002a, 9002b, 9002d second end opening
1003, 1554, 2004a, 2004b, 2515, 3007, 3120b, 3120c, 4202, 5202, 6102a, 6152a through hole
1004, 2004, 3004 valve seat supporting portion
1004a, 1101a gap
1100a, 2101a, 2530a, 2540a, 2540b, 3110a, 4110a, 4120a recess portion
1101 hollow protrusion
1102 accommodating space
1104, 1402, 1603, 2101b, 3101b opening
1105, 1604, 2103, 2514, 3103, 4140, 9104, 9104b valve connecting portion
1300, 2300, 2600, 3300, 4300, 6300, 9300 jointing piece
1401, 6001, 7001, 8001, 9102 top opening
1402a first recess
1404a second recess
1405, 2505, 4005, 9005b, 9005c, 9005d stent
1406a annular step surface
1412a first protrusion
1414a second protrusion
1502a, 1502b, 4110b stopper
1522a, 1522b, 1648, 2201, 3201, 4201, 6201 cut
1622 , 1646 , 9222 slit vent
1622a, 1646a, 9222a curved middle portion
1644, 9224, 9224b central positioning post
1662, 1664, 1666, 1682, 1684, 1686 sub-slit vent
1682a, 1682b V-shaped cut
1840 engaging mechanism
1900a, 1900b hollow nasal dilator body portion
2000a, 3000a, 4003, 6310a, 6310b, 6310c, 6310d flange
2003, 3003 stopper part
2004c, 3004c, 4006, 4007 engaging block
2101 upper portion of valve seat
2102 lower portion of valve seat
2500a, 5010, 9010, 9010a, 9010b, 9100c, 9100d first annular flange
2500b, 5020, 9020, 9020a, 9020b second annular flange
2504 annular step surface
2511, 2521 top side
2512, 2522 bottom side
2513 three-dimensional conical middle portion
2523 hollow conical middle portion
2524 trapezoidal opening
2513a trapezoidal through hole
2516a, 2516b second vent
2518 plug
2530, 4110 first hollow annular portion
2540, 4120 second hollow annular portion
3005 flow resistance structure unit
3006 slit channel
3006a first end
3006b second end
3110 top annular portion
3120 flow resistance adjustment unit
4100a common opening
4130 hollow middle portion
4210, 5210 rotatable baffle
4210a, 4210b rib end
6002, 7002, 8002 bottom of hollow carved body
6102, 6152 periphery
6104 , 6154 cavity of hollow carved body
6150 flow resistance adjustment part
6156 rotation portion
6310 supporting post
7003, 8003 annular flange
9003 perforation
9004 easy-to-remove means
9015a, 9016a first rib
9015b, 9016b second rib
9030b third annular flange
9102, 9102b through hole
9104a middle positioning post

### Embodiments

The nasal dilator with an expiratory positive airway pressure (EPAP) valve insert provided by this invention is described in detail through the following specific embodiments in conjunction with the accompanying drawings.

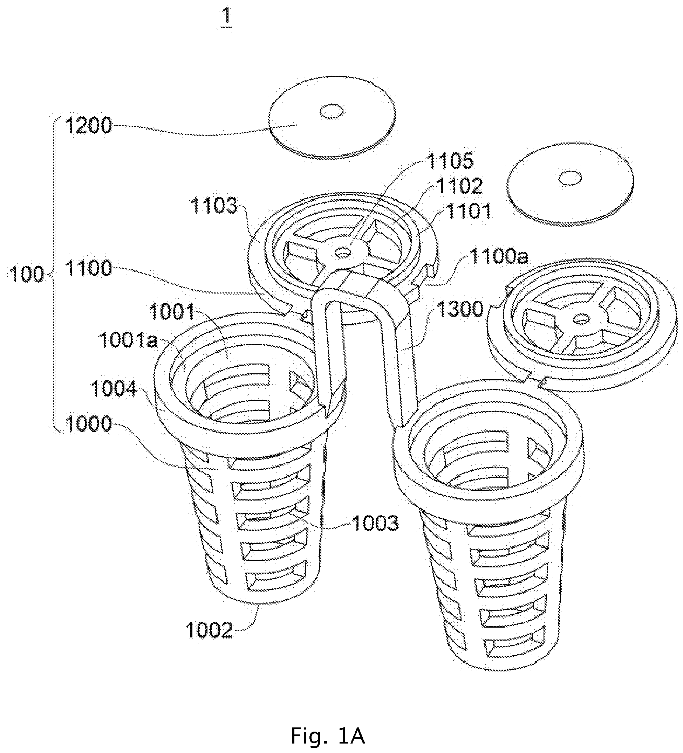

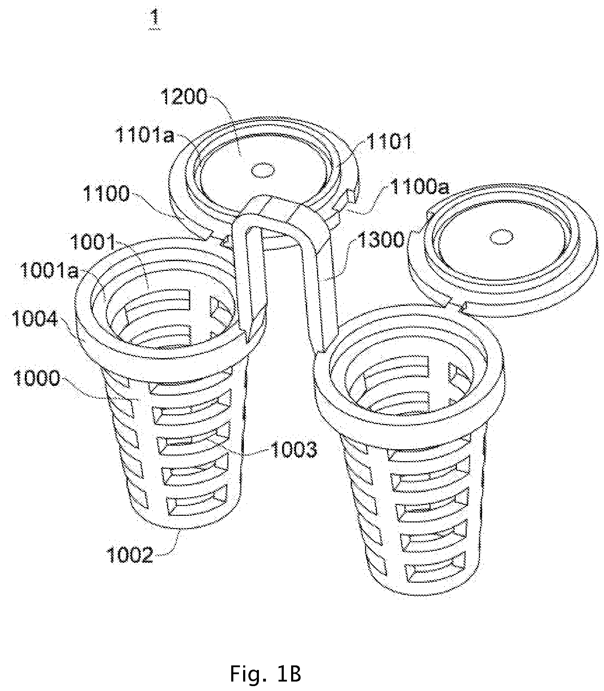

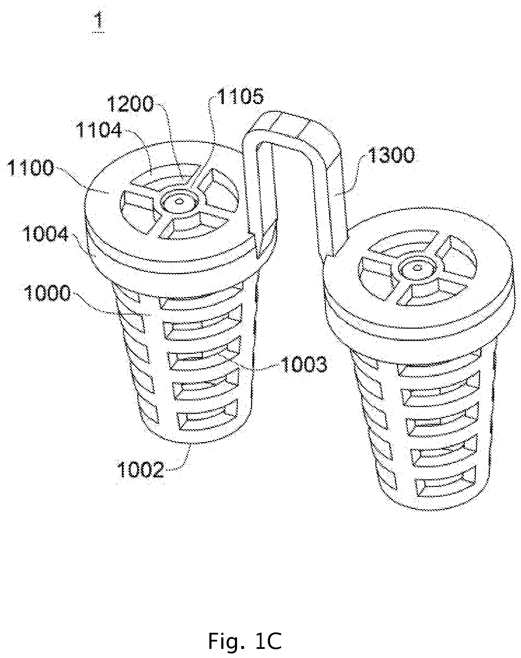

Fig. 1A through Fig. 1C are various schematic perspective views of a nasal dilator with an EPAP valve insert 1 according to a first embodiment of this invention. Fig. 1A is a schematic perspective exploded view of the nasal dilator with an EPAP valve insert 1 of the first embodiment, Fig. 1B is a schematic perspective view of the nasal dilator with an EPAP valve insert 1 of the first embodiment at its open state, Fig. 1C is a schematic perspective view of the nasal dilator with an EPAP valve insert 1 of the first embodiment after assembled. According to the first embodiment, the nasal dilator with an EPAP valve insert 1 has a pair of nasal dilator unit 100, each of the pair of nasal dilator unit 100 includes a hollow nasal dilator body 1000, a valve seat 1100 and a one-way flow resistance valve 1200. The nasal dilator with an EPAP valve insert 1 further includes a jointing piece 1300 for connecting the pair of nasal dilator unit 100. The hollow nasal dilator body 1000 has a first end opening 1001 and a second end opening 1002 opposing to the first end opening 1001, the first end opening 1001 is larger than the second end opening 1002. The hollow nasal dilator body 1000 is substantially cylinder shape longitudinally extending from the first end opening 1001 to the second end opening 1002, an outer diameter of the hollow nasal dilator body 1000 gradually decreases from the first end opening 1001 to the second end opening 1002 to facilitate an outer profile of the hollow nasal dilator body 1000 mates with a user's nasal cavity. A surrounding wall of the hollow nasal dilator body 1000 extending between the first end opening 1001 and the second end opening 1002 is provided with a plurality of through hole 1003 such that the hollow nasal dilator body 1000 become hollow carved and a weight of the hollow nasal dilator body 1000 may decrease. Without increasing the manufacturing complexity, these through holes 1003 may be any shape to keep structure strength and light weight of the hollow nasal dilator body 1000. A valve seat supporting portion 1004 is provided at the first end opening 1001 of the hollow nasal dilator body 1000 for assembling the vale seat 1100. A step surface 1001a is provided at the first end opening 1001 between the valve seat supporting portion 1004 and the hollow nasal dilator body 1000, and the valve seat 1100 is assembled unto the step surface 1001a. The valve seat 1100 is pivotally assembled to the valve seat supporting portion 1004 of the hollow nasal dilator body 1000 to reduce a risk of the valve seat 1100 may easily drop off. A hollow protrusion 1101 is formed at one side of the valve seat 1100 toward the hollow nasal dilator body 1000, the hollow protrusion 1101 has an accommodating space 1102 for positioning the one-way flow resistance valve 1200. An outer diameter of the hollow protrusion 1101 is smaller than an outer diameter of the valve seat 1100 so that a step surface 1103 is formed between them. When the valve seat 1100 is assembled to the hollow nasal dilator body 1000, the step surface 1103 resists against one side of the valve seat supporting portion 1004 opposing to the first end opening 1001, the hollow protrusion 1101 is received inside the valve seat supporting portion 1004 over the step surface 1001a. Please see Fig. 1C, there is an opening 1104 provided at one side of the valve seat 1100 away from the hollow nasal dilator body 1000 to fluidly communicate with the hollow protrusion 1101 of the valve seat 1100. The valve seat 1100 has a valve connecting portion 1105 provided at the opening 1104 for assembling and fixing the one-way flow resistance valve 1200 to the valve seat 1100. Please see Fig. 1B, an outer diameter of the one-way flow resistance valve 1200 is smaller than an inner diameter of the hollow protrusion 1101 of the valve seat 1100 in order that a gap is provided between the one-way flow resistance valve 1200 and the hollow protrusion 1101 to serve as a fluid channel once the one-way flow resistance valve 1200 is assembled to the valve seat 1100. The one-way flow resistance valve 1200 may be practiced by a check valve made of a flexible material so that the one-way flow resistance valve 1200 may be deformed when air flow pressure acts at the one-way flow resistance valve 1200. A recess portion 1100a is provided at a location of an outer surrounding wall of the valve seat 1100 corresponding to the jointing piece 1300. When the valve seat 1100 is assembled to the hollow nasal dilator body 1000, the jointing piece 1300 engages the recess portion 1100a of the outer surrounding wall of the valve seat 1100 to enhance assembling between the valve seat 110 and the hollow nasal dilator body 1000.

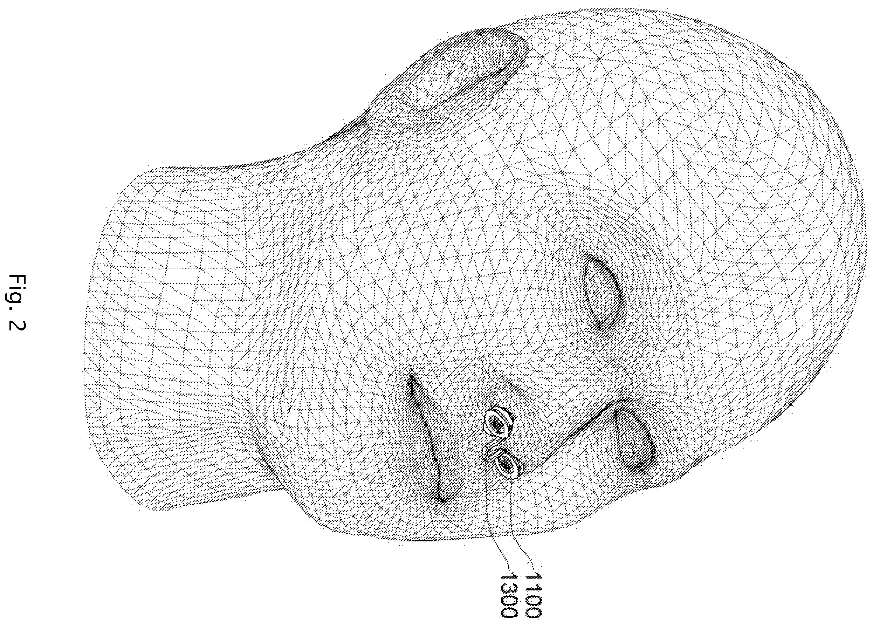

Fig. 2 shows a use state of the nasal dilator with an EPAP valve insert 1 worn on a user. It may be seen that the hollow nasal dilator body 1000 inserts the nasal nostril of the user, supporting and dilating the nasal passage, while both the jointing piece 1300 and the side of the valve seat 1100 having the opening 1104 are outside the nasal nostrils, and engaging against them. The jointing piece 1300 may prevent the nasal dilator with an EPAP valve insert 1 from lost and also helping the user easily wear and take off the nasal dilator with an EPAP valve insert 1.

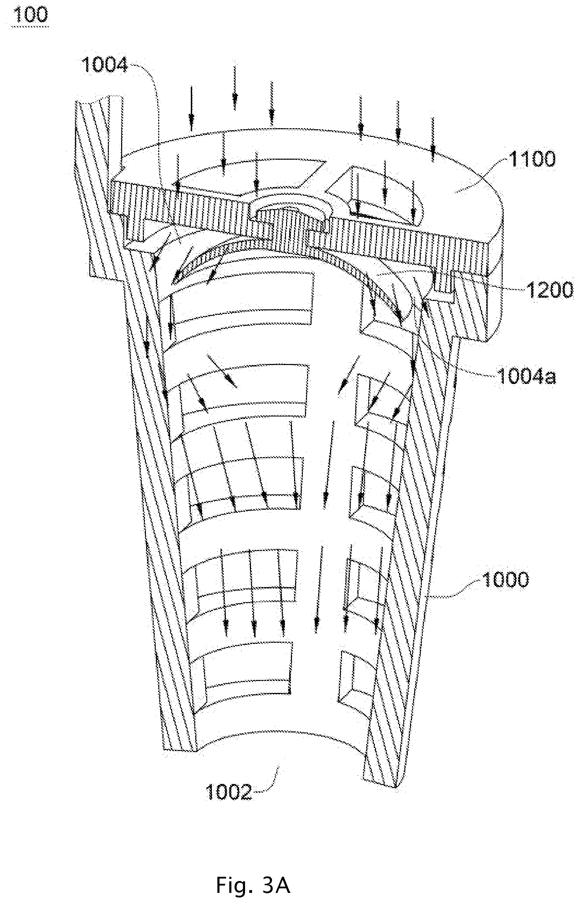

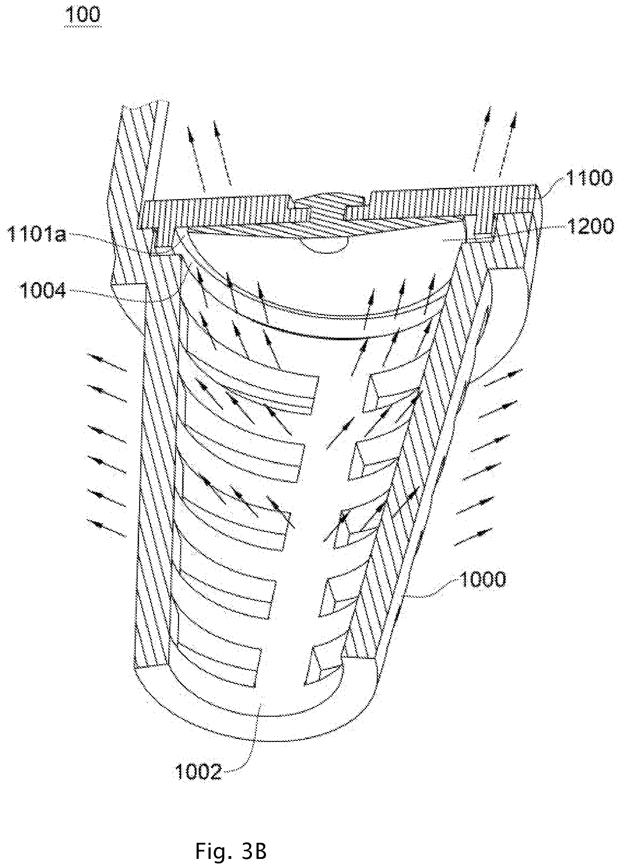

Please refer to Fig. 3A, during the inhalation of the user, air flow directions are represented by arrows. The one-way flow resistance valve 1200 is in an open state to allow air flow from outside entering the hollow nasal dilator body 1000, and meanwhile air flow pressure acts at the one-way flow resistance valve 1200 to force the one-way flow resistance valve 1200 toward the second end opening 1002 deformed and becoming an umbellar configuration, so that a gap 1004a is provided between the one-way flow resistance valve 1200 and the inner surrounding wall of the hollow nasal dilator body 1000 to facilitate air flow pass through the one-way flow resistance valve 1200 and the gap 1004a and entering the hollow nasal dilator body 1000 to let the user easily breathe. Please refer to Fig. 3B, during the exhalation of the user, air flow directions are represented by arrows, the air flow from the second end opening 1002 of the hollow nasal dilator body 1000 flows to the first end opening 1001, and the one-way flow resistance valve 1200 is in a close state to prevent air flow from passing through, and air flow pressure forces the one-way flow resistance valve 1200 returning to the original configuration from the umbrella configuration. Meanwhile, air flow inside the hollow nasal dilator body 1000 only can pass the gap 1101a between the one-way flow resistance valve 1200 and the valve seat 1100, so that the flow resistance of the air flow represented by the arrows increases relative to the flow resistance of the air flow when the user inhales. As a consequence, a positive pressure is generated inside the upper airway of the user to facilitate the upper airway keep patency to stabilize soft tissue inside the oral cavity of the user to prevent the soft tissue from collapsing.

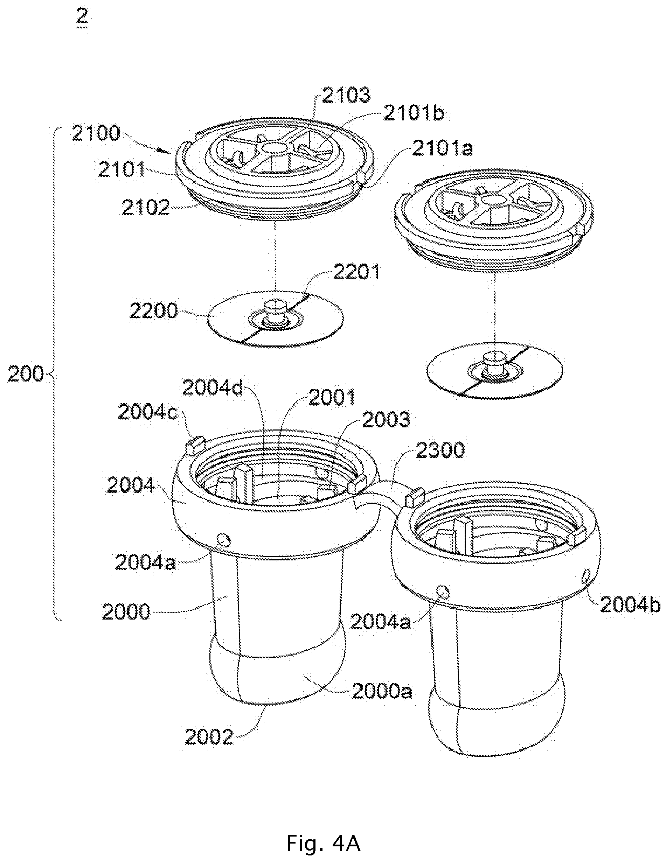

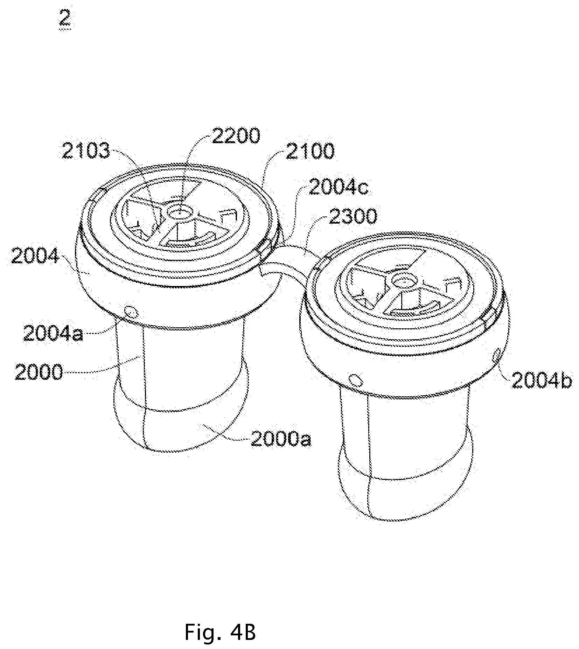

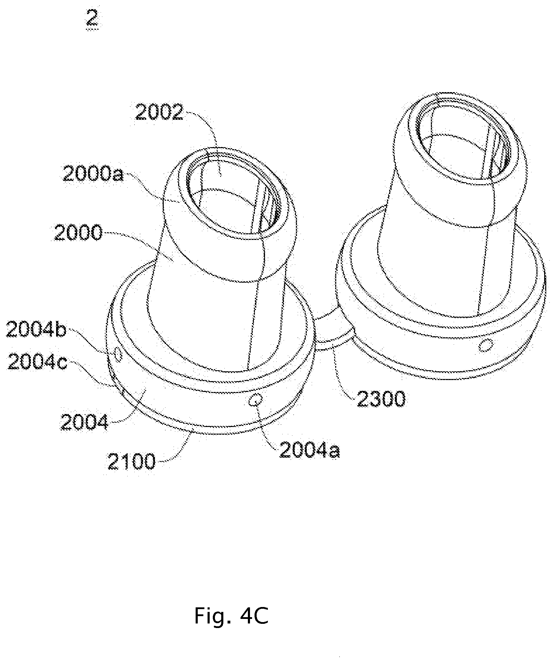

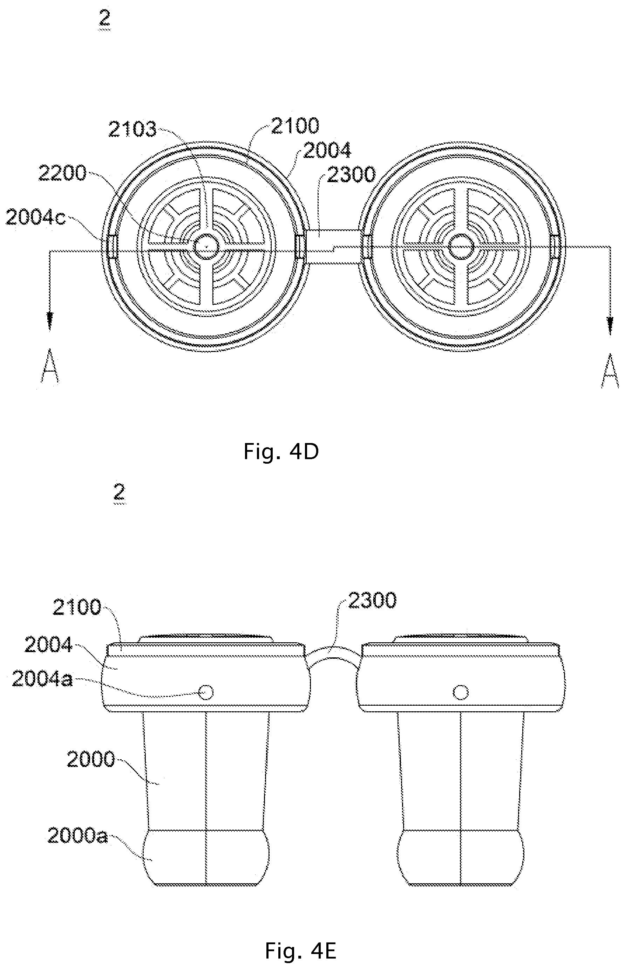

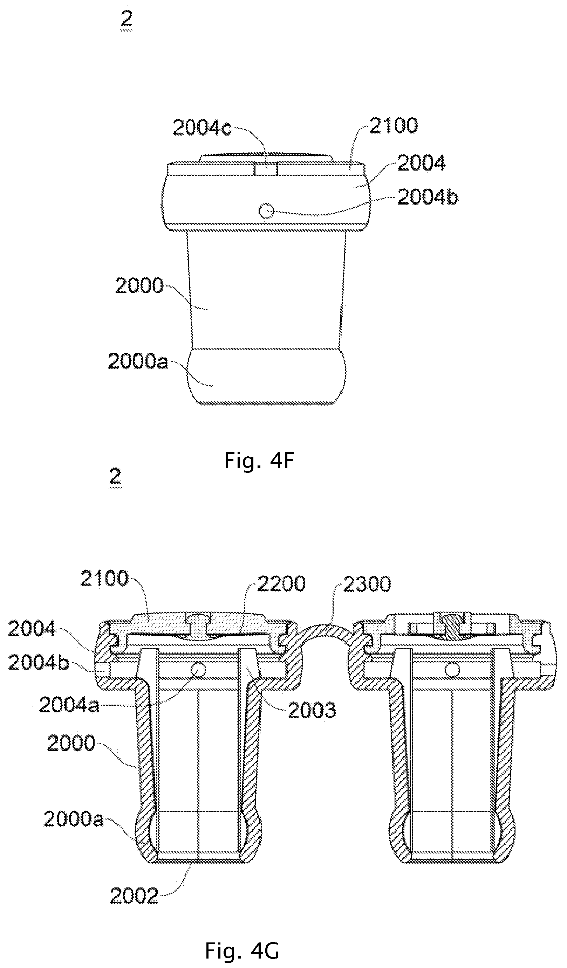

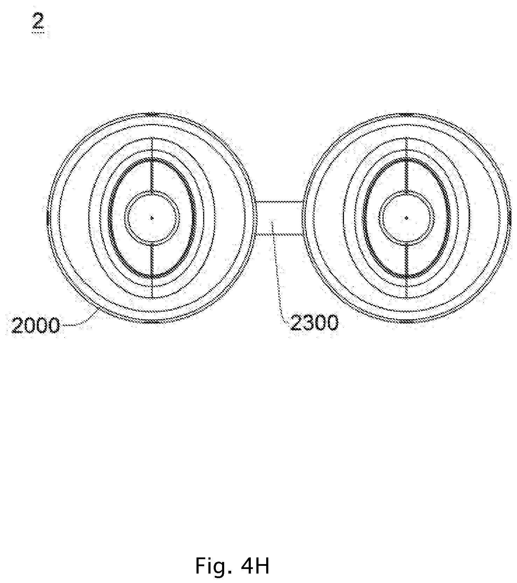

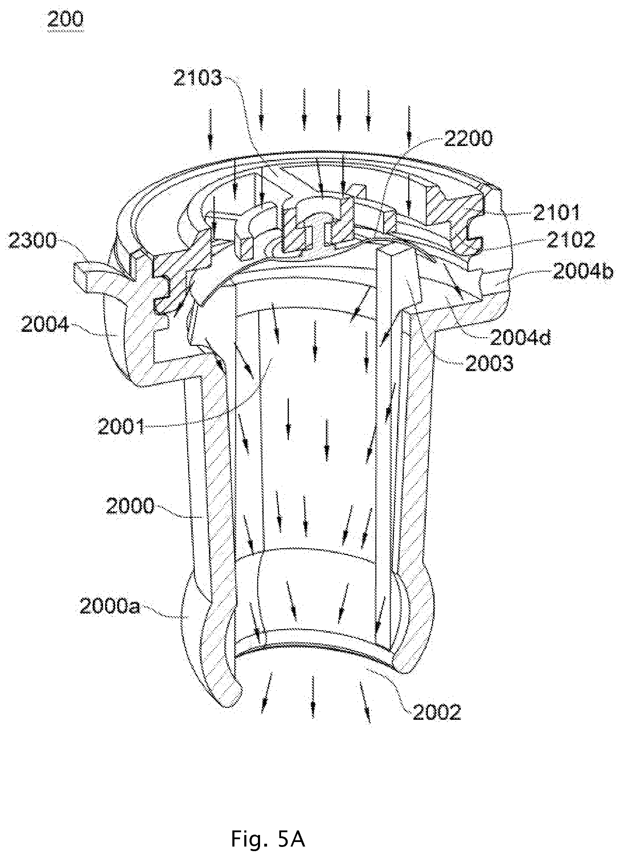

Fig. 4A through Fig. 4H are various schematic views of a nasal dilator with an EPAP valve insert 2 according to a second embodiment of this invention. Fig. 4A is a schematic perspective exploded view, Fig. 4B is a schematic perspective front view, Fig. 4C is a schematic perspective bottom view, Fig. 4D is a schematic top view, Fig. 4E is a schematic front view, Fig. 4F is a schematic side view, Fig. 4G is a schematic cross-sectional view along the A-A cutting line of Fig. 4D, Fig. 4H is a schematic bottom view. The nasal dilator with an EPAP valve insert 2 of the second embodiment includes a pair of nasal dilator unit 200, each of the pair of the nasal dilator unit 200 includes a hollow nasal dilator body 2000, a valve seat 2100 and a one-way flow resistance valve 2200. The nasal dilator with an EPAP valve insert 2 further comprises a jointing piece 2300 for connecting the pair of nasal dilator unit 200. The hollow nasal dilator body 2000 has a first end opening 2001 and a second end opening 2002 opposite to the first end opening 2001. The hollow nasal dilator body 2000 extends longitudinally from the first end opening 2001 to the second end opening 2002. The outer surrounding wall of the hollow nasal dilator body 2000 at the second end opening 2002 is provided with a flange 2000a to improve the degree of engagement between the nasal dilator with an EPAP valve insert 2 and the nasal cavity, and dilating the nasal passage of the user. A hollow valve seat supporting portion 2004 is provided at the first end opening 2001 of the hollow nasal dilator body 2000 to assemble the valve seat 2100. Please refer to Fig. 5A, an inner diameter of the hollow valve seat supporting portion 2004 is larger than the diameter of the first end opening 2001 of the hollow nasal dilator body 2000 so that a step surface 2004d may be provided at the first end opening 2001 of the hollow nasal dilator body 2000 and connecting the hollow nasal dilator body 2000. At least a pair of opposing stoppers 2003 is provided at the inner surrounding wall of the hollow nasal dilator body 2000. The stoppers 2003 along the inner surrounding wall of the hollow nasal dilator body 2000 pass the first end opening 2001 and the step surface 2004d to longitudinally extend inside the hollow valve seat supporting portion 2004 and corresponding to the jointing piece 2300. A pair of opposing through holes 2004a pass through the wall of the hollow valve seat supporting portion 2004 and fluidly communicates inside of the hollow valve seat supporting portion 2004, and the pair of through holes 2004a are spaced from the stoppers 2003. Another through hole 2004b passes through the wall of the hollow valve seat supporting portion 2004 and is positioned at where near the stopper 2003 away from the jointing piece 2300. The through hole 2004b fluidly communicates inside of the hollow valve seat supporting portion 2004. A pair of opposing engaging block 2004c is disposed at the top surface of the hollow valve seat supporting portion 2004 to engage the valve seat 2100 so that the parts of the nasal dilator unit 200 may be assembled together firmly. The valve seat 2100 has a hollow upper portion 2101 and a hollow lower portion 2102 fluidly communicating with each other. The outer periphery of the hollow upper portion 2101 has a pair of opposing recess 2101a (see Fig. 4A) to engage the pair of engaging block 2004c of the hollow valve seat supporting portion 2004. The hollow lower portion 2102 is a hollow jointing protrusion toward the first end opening 2001 of the hollow nasal dilator body 2000 to engage the inner wall portion of the hollow valve seat supporting portion 2004 above the through holes 2004a and 2004b such that a space above the step surface 2004d of the hollow valve seat supporting portion 2004 is provided and fluidly communicating with the first end opening 2001 and the through holes 2004a, 2004b. The hollow upper portion 2101 of the valve seat 2100 has an opening 2101b and a valve jointing portion 2103 provided at the opening 2101b to join and fasten the one-way flow resistance valve 2200 unto the valve seat 2100. The one-way flow resistance valve 2200 has at least one radial cut 2201. The one-way flow resistance valve 2200 may be practiced by a check valve and made of flexible material. When air flow pressure acts at the one-way flow resistance valve 2200, it may be deformed. Also, the radial cut 2201 assists the deformation of the one-way flow resistance valve 2200 along the air flow direction when the air flow pressure acts at the one-way flow resistance valve 2200.

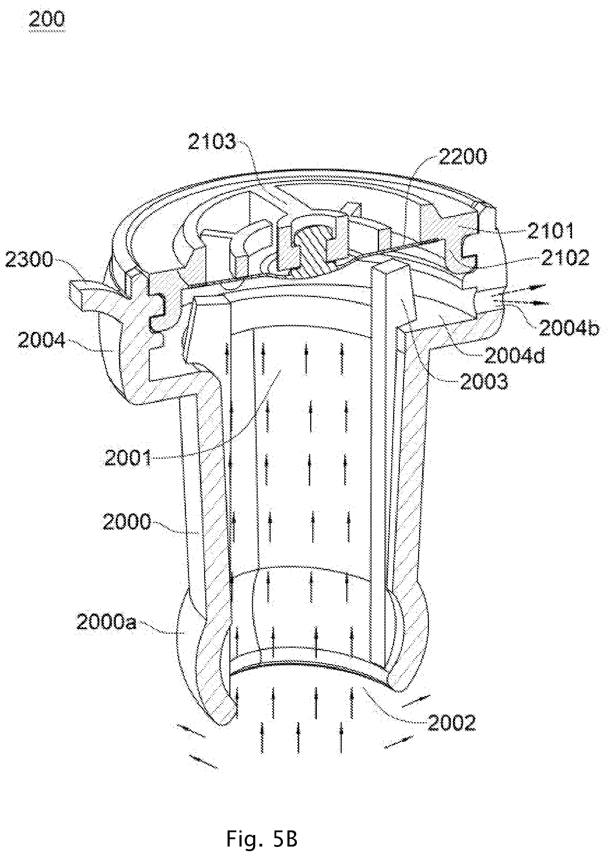

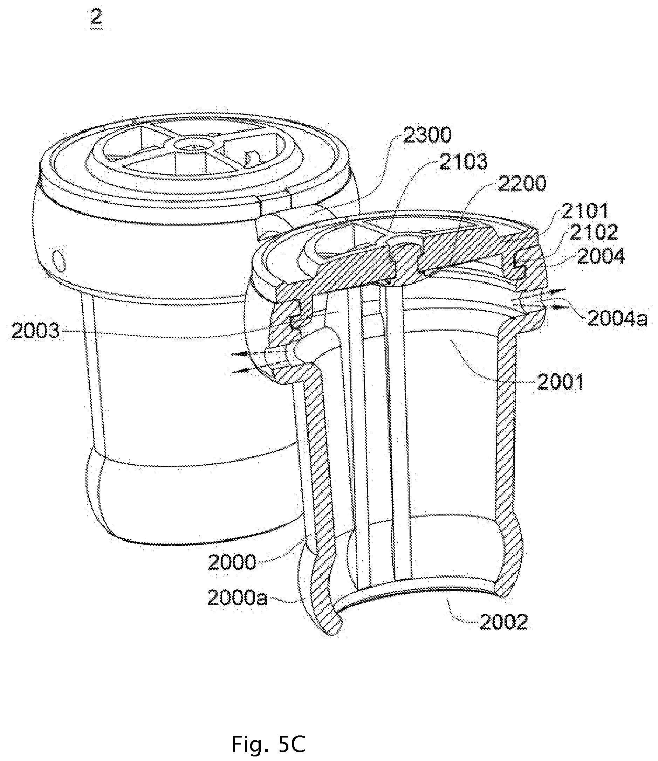

Please refer to Fig. 5A, during the inhalation of the user, air flow directions are represented by arrows. The one-way flow resistance valve 2200 is in an open state to allow air flow entering the hollow nasal dilator body 2000 to let the user easily breathe. Meanwhile, air flow pressure acts at the one-way flow resistance valve 2200 to force the one-way flow resistance valve 2200 deformed to become an umbrella configuration toward the second end opening 2022 and resisting against the stoppers 2003. The height of the stoppers 2003 protruding the step surface 2004d may be varied so as to adjust an opening angle of the umbrella configuration of the one-way flow resistance valve 2200 to change a size of fluid channels between the one-way flow resistance valve 2200 and the hollow valve seat supporting portion 2004. Please refer to Fig. 5B, during the exhalation of the user, air flow directions are represented by arrows, the air flow from the second end opening 2002 of the hollow nasal dilator body 2000 flows to the first end opening 2001, and the one-way flow resistance valve 2200 is in a closed state to prevent the air flow from passing through it, and air flow pressure forces the one-way flow resistance valve 2200 returning to the original state from the umbrella configuration and resisting against the bottom of the hollow upper portion 2101 so as to close fluid channels between the valve seat 2100 and outside environment. Please refer to Fig. 5B and Fig. 5C, meanwhile, air flow inside the hollow nasal dilator body 2000 only can pass through the through holes 2004a, 2004b of the hollow valve seat supporting portion 2004 to outside. As a consequence, flow resistance of air flow represented by arrows is increased relative to the flow resistance of air flow during the inhalation of the user. Therefore, a positive pressure is generated inside the upper airway of the user to facilitate the upper airway keep patency to stabilize soft tissue inside the oral cavity of the user to prevent the soft tissue from collapsing.

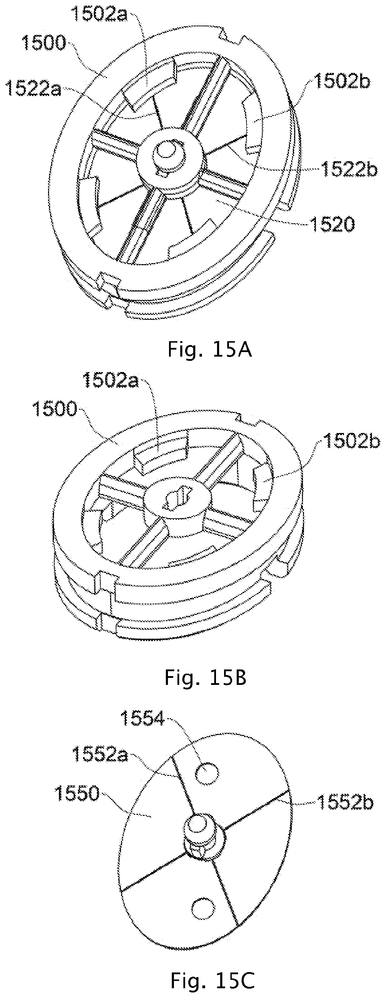

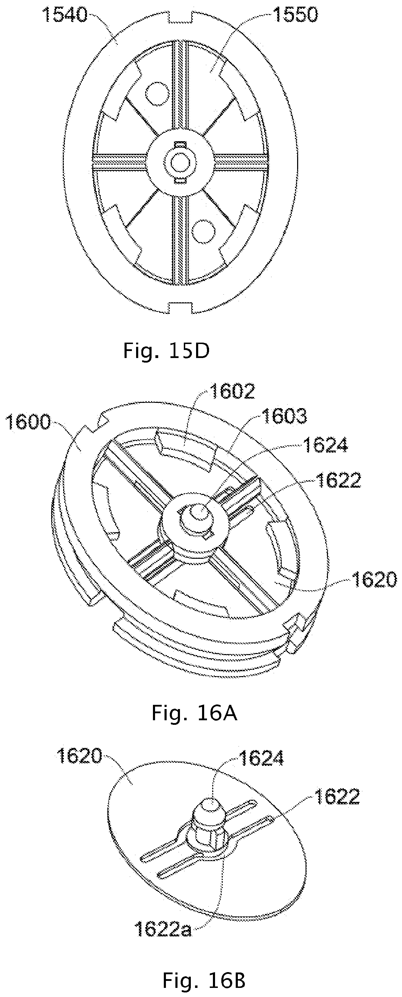

Fig. 15A shows a schematic perspective assembled view of a valve seat 1500 and a one-way flow resistance valve 1520, which is a variance of the valve seat 2100 and the one-way flow resistance valve 2200 of the second embodiment. Fig. 15B is a schematic perspective view of the valve seat 1500. The one-way flow resistance valve 1520 has two radial cuts 1522a, 1522b in a cross configuration. Alternately, the two radial cuts 1522a, 1522b may be in an X configuration (not shown). The inner surrounding wall of the valve seat 1500 is provided with blocks 1502a, 1502b corresponding to the radial cuts 1522a, 1522b so that the blocks 1502a, 1502b may block the one-way flow resistance valve 1520 to prevent air leakage from the hollow nasal dilator body during the exhalation of the user. Moreover, one skilled artisan in the field would understand a valve seat 1540 and a one-way flow resistance valve 1550 shown in Fig. 15D may be another variance of the valve seat 2100 and the one-way flow resistance valve 2200 of the second embodiment. As Fig. 15C shows, the one-way flow resistance valve 1550 has two radial cuts 1522a, 1522b in a cross configuration and a pair of air vent 1554 spaced from the radial cuts 1522a, 1522b. Also, the two radial cuts 1522a, 1522b may be in an X configuration (not shown).

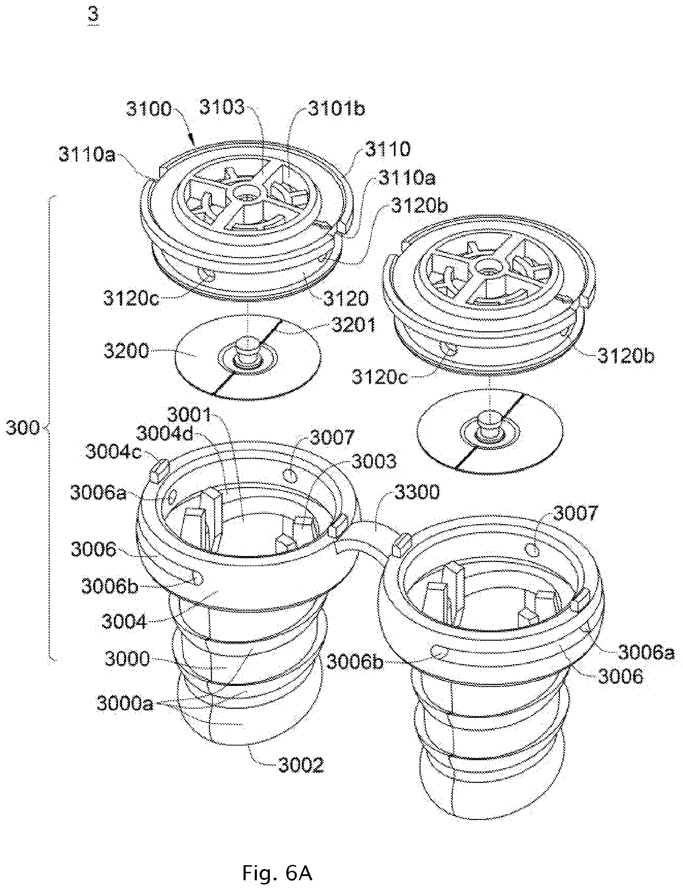

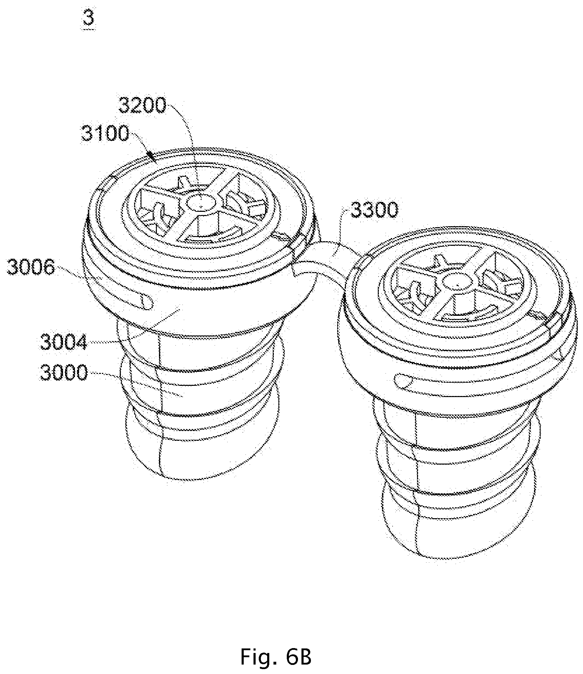

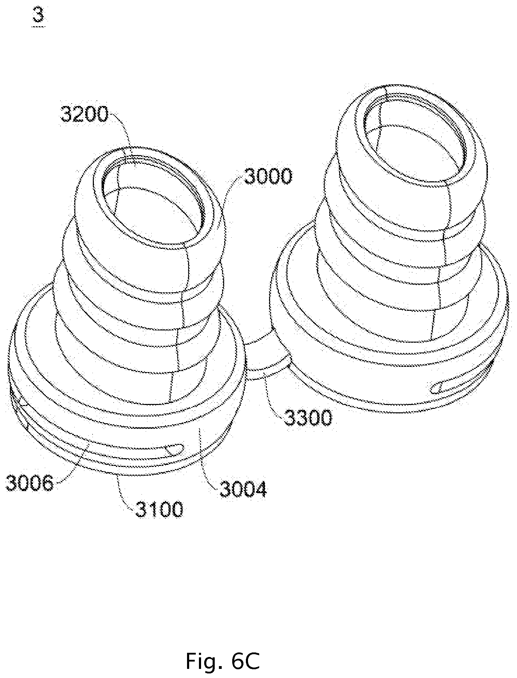

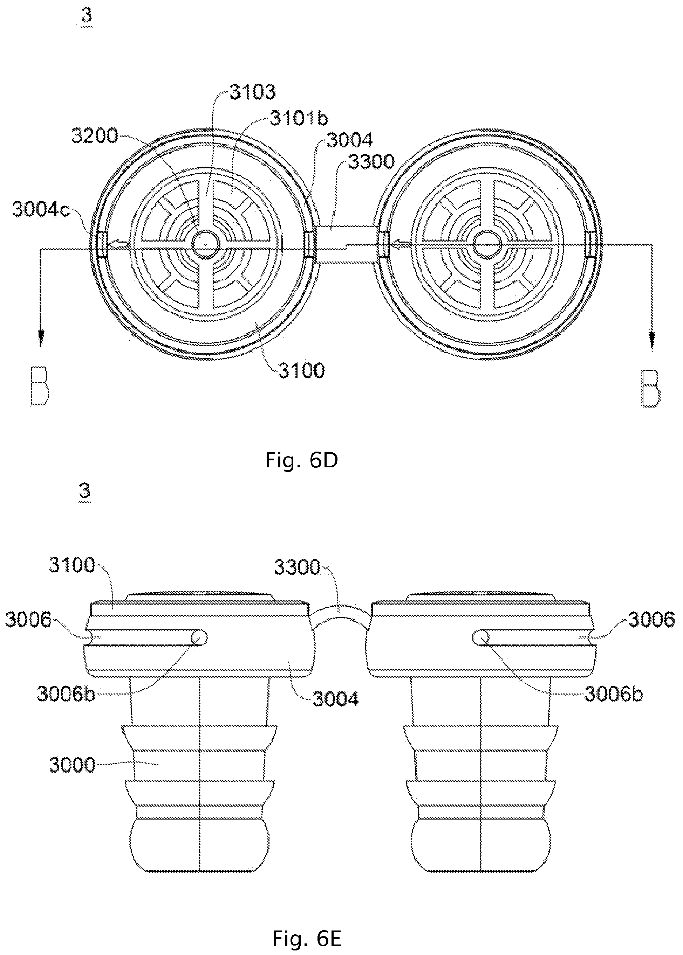

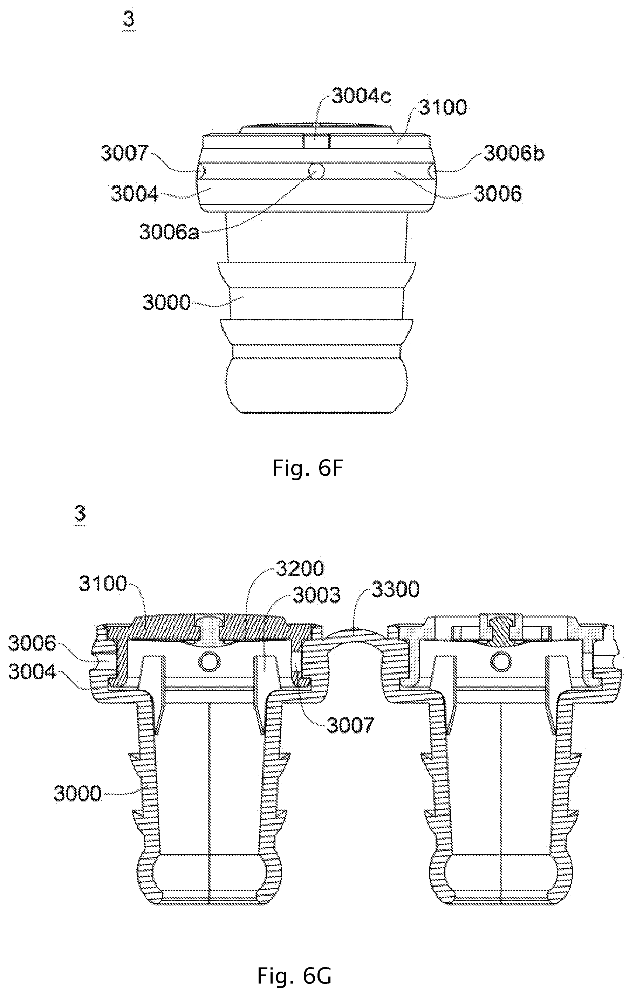

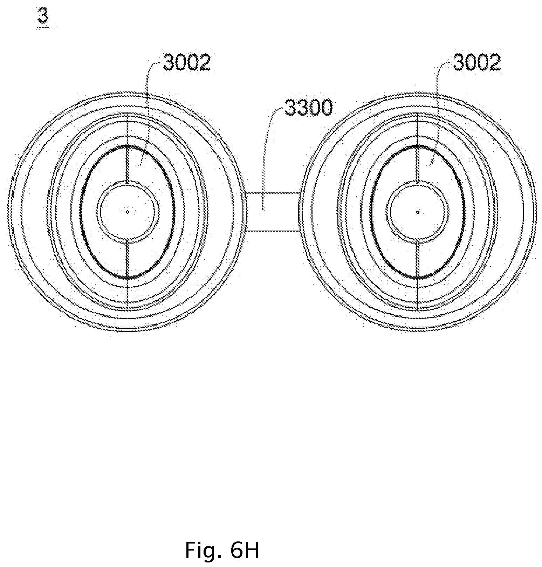

Fig. 6A through Fig. 6H are various schematic views of a nasal dilator with an EPAP valve insert 3 according to a third embodiment of this invention. Fig. 6A is a schematic perspective exploded view, Fig. 6B is a schematic perspective front view, Fig. 6C is a schematic perspective bottom view, Fig. 6D is a schematic top view, Fig. 6E is a schematic front view, Fig. 6F is a schematic side view, Fig. 6G is a schematic cross-sectional view of Fig. 6D along the B-B cutting line, and Fig. 6H is a schematic bottom view. The difference between the third embodiment and second embodiment mainly resides in a flow resistance adjustment unit and a flow resistance structure unit provided in the nasal dilator with an EPAP valve insert 3 of the third embodiment. A number of air flow exit and a size of the air flow exit of the nasal dilator with an EPAP valve insert 3 may be varied by changing the position of the flow resistance adjustment unit relative to the flow resistance structure unit so as to adjust the exhalation air flow resistance. As a consequence, a positive pressure inside the nasal cavity of the user may be changed/adjusted during the exhalation of the user.

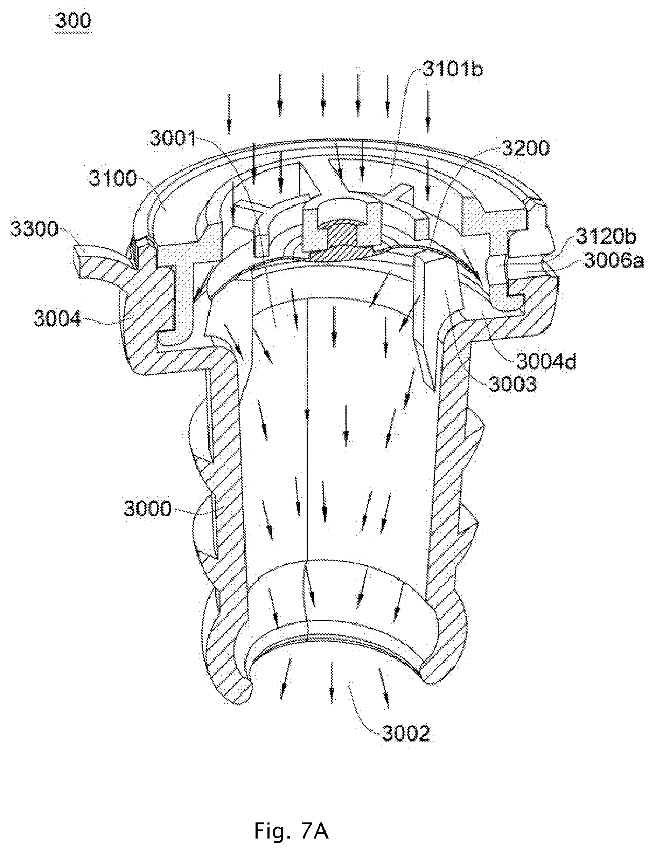

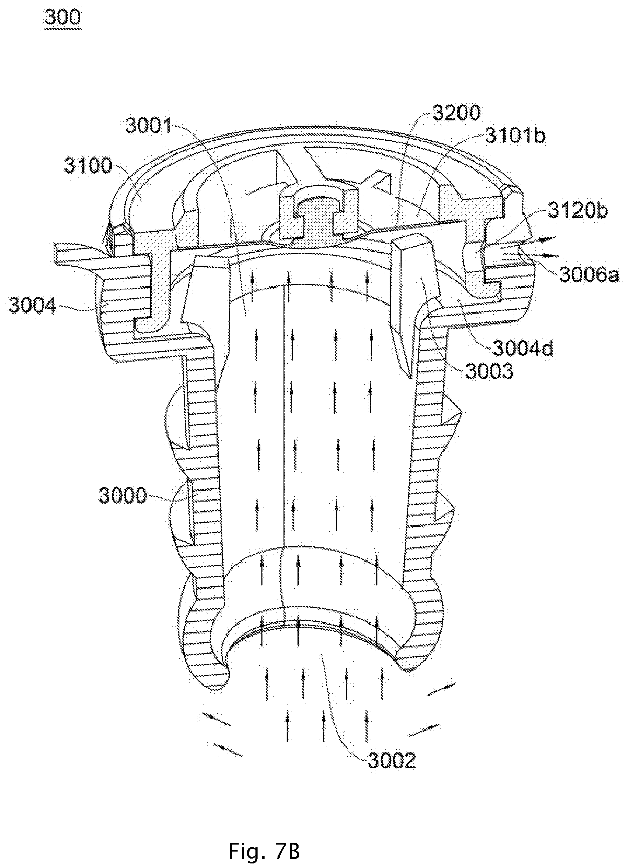

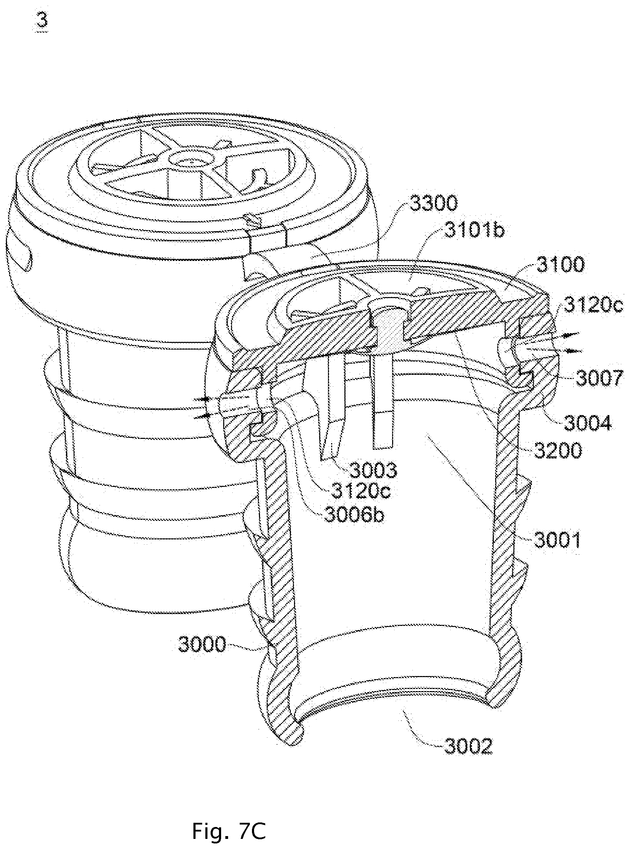

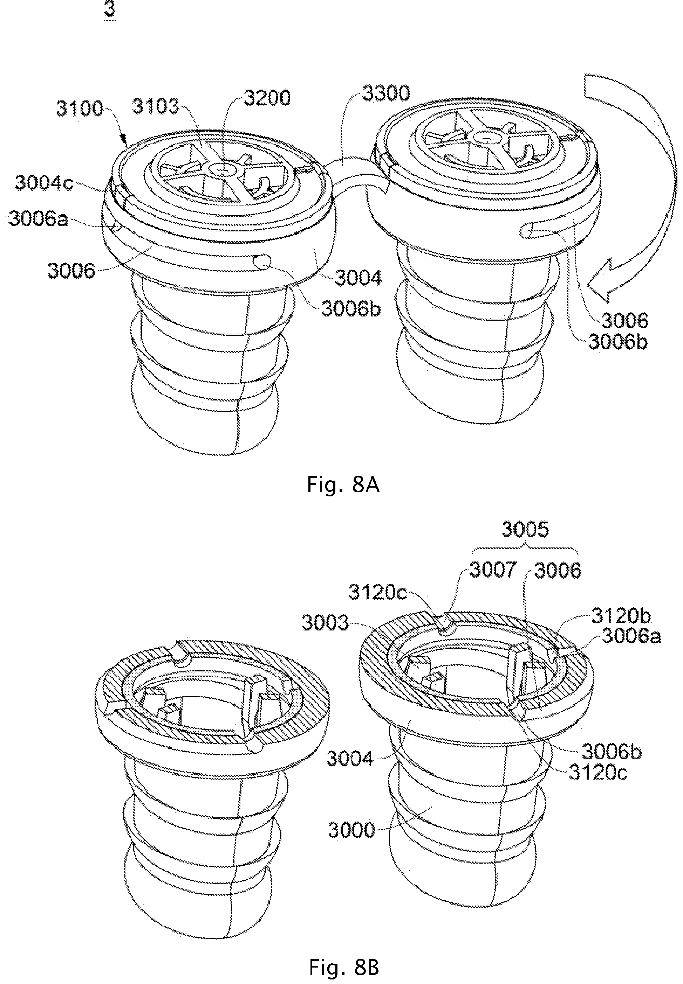

The nasal dilator with an EPAP valve insert 3 of the third embodiment includes a pair of nasal dilator unit 300. Each of the pair of nasal dilator unit 300 includes a hollow nasal dilator body 3000, a valve seat 3100 and a one-way flow resistance valve 3200. The nasal dilator with an EPAP valve insert 3 further includes a jointing piece 3300 for connecting the pair of nasal dilator unit 300. The hollow nasal dilator body 3000 has a first end opening 3001 and a second end opening 3002 opposite to the first end opening 3001. The hollow nasal dilator body 3000 from the first end opening 3001 extends longitudinally to the second end opening 3002. A plurality of flange 3000a is disposed longitudinally around an outer surrounding wall of the hollow nasal dilator body 3000 to enhance the degree of engagement between the hollow nasal dilator body 3000 and the nasal cavity, and supporting and dilating the nasal passage of the user. The hollow nasal dilator body 3000 is provided with a hollow valve seat supporting portion 3004 at the first end opening 3001 to assemble the valve seat 3100. Please refer to Fig. 7A, an inner diameter of the hollow valve seat supporting portion 3004 is larger than a diameter of the first end opening 3001 so that the hollow valve seat supporting portion 3004 can have a step surface 3004d at the first end opening 3001 and connecting the hollow nasal dilator body 3000. The inner surrounding wall of the hollow nasal dilator body 3000 is provided with at least a pair of stopper 3003 passing the first end opening 3001 and the step surface 3004d and extending longitudinally inside the hollow valve seat supporting portion 3004 and corresponding to the jointing piece 3300. Please refer to Fig. 6A and Fig. 8B, the hollow valve seat supporting portion 3004 is provided with a flow resistance structure unit 3005 having a slit channel 3006 and a through hole 3007. The slit channel 3006 has a first end 3006a corresponding to the stopper 3003 and a second end 3006b corresponding to the through hole 3007. The slit channel 3006 from the first end 3006a extends around the surrounding wall of the hollow valve seat supporting portion 3004 to the second end 3006b. The slit channel 3006 and the through hole 3007 pass through the surrounding wall of the hollow valve seat supporting portion 3004 to fluidly communicate inside of the hollow vale seat supporting portion 3004. A pair of engaging block 3004c is provided at a top surface of the hollow valve seat supporting portion 3004 to engage the valve seat 3100 so that the components of the nasal dilator unit 300 may be assembled firmly. The vale seat 3100 has a hollow annular upper portion 3110 and a hollow flow resistance adjustment unit 3120. The upper portion of the flow resistance adjustment unit 3120 is rotatingly assembled in the annular upper portion 3110, and the lower portion of the flow resistance adjustment unit 3120 protrudes from the bottom of the annular upper portion 3110. A pair of opposing recess 3110a is provided at an outer periphery of the annular upper portion 3110 to engage the pair of engaging block 3004c of the hollow valve seat supporting portion 3004. An arrow marking is provided at a top surface of the annular upper portion 3110 of the flow resistance adjustment unit 3120 corresponding to the recess 3110a to represent a rotation starting point of the flow resistance adjustment unit 3120 relative to the annular upper portion 3110. The surrounding wall of the lower portion of the flow resistance adjustment unit 3120 is provided with a through hole 3120b corresponding to the recess 3110a aligning with the arrow marking. A pair of through hole 3120c spaced from the through hole 3120b is provided at the surrounding wall of the lower portion of the flow resistance adjustment unit 3120. When the valve seat 3100 is assembled to the hollow valve seat supporting portion 3004, the lower portion of the flow resistance adjustment unit 3120 is positioned above the step surface 3004d of the hollow valve seat supporting portion 3004. Take the nasal dilator unit 3 at the right side as an example for explanation. When the arrow marking aligns with the recess 3110a of the annular upper portion 3110, the through hole 3120b completely fluidly communicates with the first end 3006a of the slit channel 3006 of the hollow valve seat supporting portion 3004, and one of the through holes 3120c completely fluidly communicates with the second end 3006b of the slit channel 3006, while the other through hole 3120c completely fluidly communicates with the through hole 3007 of the hollow valve seat supporting portion 3004. Please refer to Fig. 7B and Fig. 7C, under this circumstance, the three through holes 3120b, 3120c of the valve seat 3100 relative to the hollow seat valve supporting portion 3004 are completely opened to form three exhalation air flow exits of the nasal dilator with an EPAP valve insert 3. Moreover, referring to Fig. 8A, when the flow resistance adjustment unit 3120 is rotated along the arrow direction, the through hole 3120b of the flow resistance adjustment unit 3120 of the nasal dilator unit 300 at the right side departs from the first end 3006a of the hollow valve seat supporting portion 3004, resulting in the though hole 3120b is partially or completely blocked by the surrounding wall of the flow resistance adjustment unit 3120. And, the second end 3006b of the hollow valve seat supporting portion 3004 is partially or completely blocked by the surrounding wall of the flow resistance adjustment unit 3120. Under this circumstance, the number of the exhalation air flow exit of the nasal dilator with an EPAP valve insert 3 may decrease and/or the size of the air flow exit may be reduced, and hence the exhalation air flow resistance increases and a positive pressure inside the nasal cavity also increases. The exhalation air flow resistance may be adjusted depending on the user's demand by practicing the nasal dilator with an EPAP valve insert 3 of the third embodiment. As a consequence, the positive pressure inside the nasal cavity may be adjusted.

Please refer to Fig. 6A, the top surface of the flow resistance adjustment unit 3120 of the valve seat 3100 has an opening 3101b and a valve connecting part 3103 positioned at the opening 3101b. The one-way flow resistance valve 3200 has at least one radial cut 3201 and made of flexible material. The one-way flow resistance valve 3200 may be a check valve. The one-way flow resistance valve 3200 may be deformed when air flow pressure acts at the one-way flow resistance valve 3200. Moreover, upon action of the air flow pressure, the radial cut assists the one-way flow resistance valve 3200 deforms along the air flow direction.

Please refer to Fig. 7A, during the inhalation of the user, air flow directions are represented by arrows. The one-way flow resistance valve 3200 is in an open state to allow air flow entering the hollow nasal dilator body 3000 to let the user easily breathe. Meanwhile, air flow pressure acts at the one-way flow resistance valve 3200 to force the one-way flow resistance valve 3200 deformed to become an umbrella configuration toward the second end opening 3002 and resisting against the stoppers 3003. The height of the stoppers 3003 protruding the step surface 3004d may be varied so as to adjust an opening angle of the umbrella configuration of the one-way flow resistance valve 3200 to change a size of fluid channels between the one-way flow resistance valve 3200 and the hollow valve seat supporting portion 3004. Please refer to Fig. 7B, during the exhalation of the user, air flow directions are represented by arrows, the air flow from the second end opening 3002 of the hollow nasal dilator body 3000 flows to the first end opening 3001, and the one-way flow resistance valve 3200 is in a closed state to prevent the air flow from passing through it, and air flow pressure forces the one-way flow resistance valve 3200 returning to the original state from the umbrella configuration and resisting against the top of the flow resistance adjustment unit 3120 so as to close the opening 3 101b of the valve seat 3100. Please refer to Fig. 7A and Fig. 7B, meanwhile, air flow inside the hollow nasal dilator body 3000 only can pass through the through holes 3120b and 3120c of the hollow valve seat supporting portion 3004 to outside of the nasal cavity. As a consequence, flow resistance of air flow represented by arrows is increased relative to the flow resistance of air flow during the inhalation of the user. Therefore, a positive pressure is generated inside the upper airway of the user to facilitate the upper airway keep patency to stabilize soft tissue inside the oral cavity of the user to prevent the soft tissue from collapsing.

Moreover, the variances of the valve seat 2100 and the one-way flow resistance valve 2200 of the second embodiment may be used as variances of the valve seat 3100 and the one-way flow resistance valve 3200.

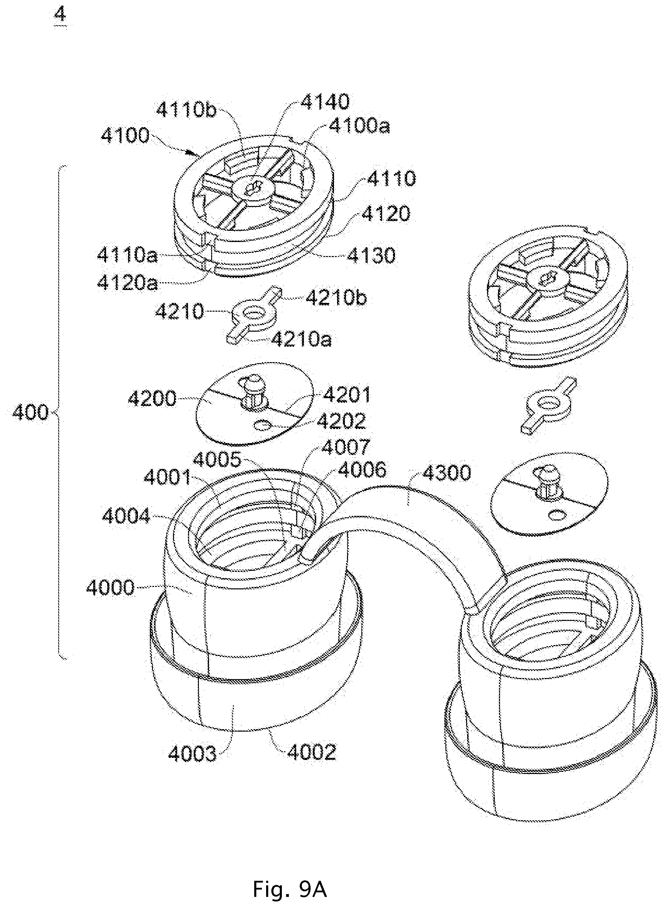

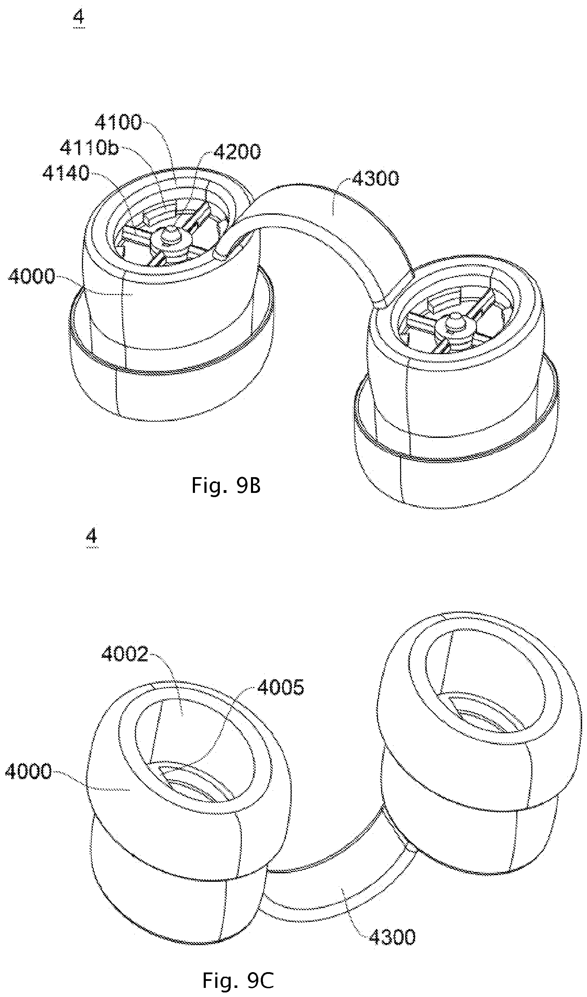

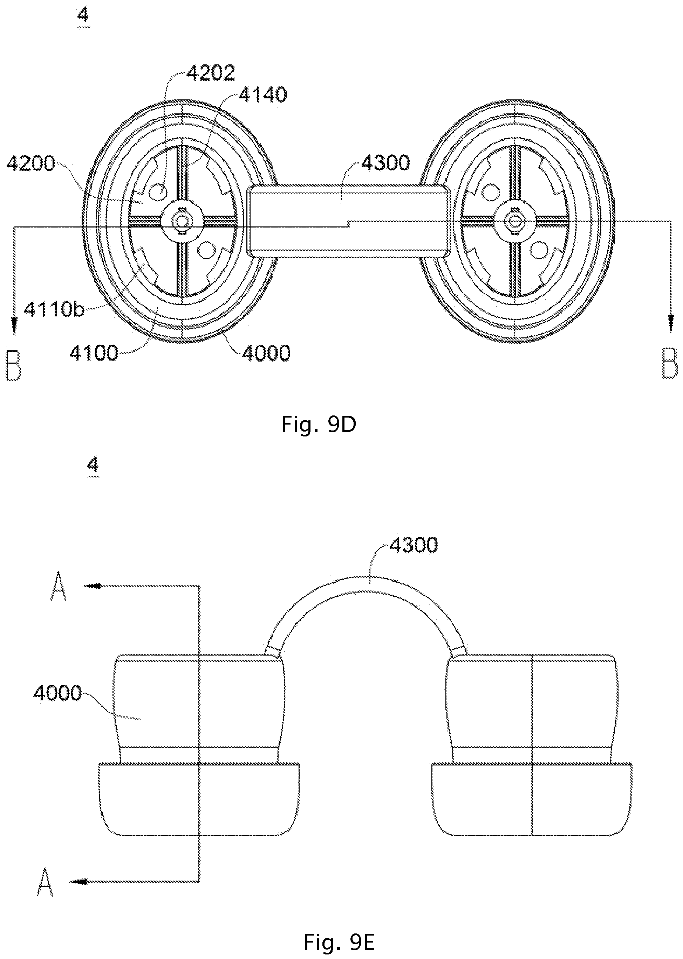

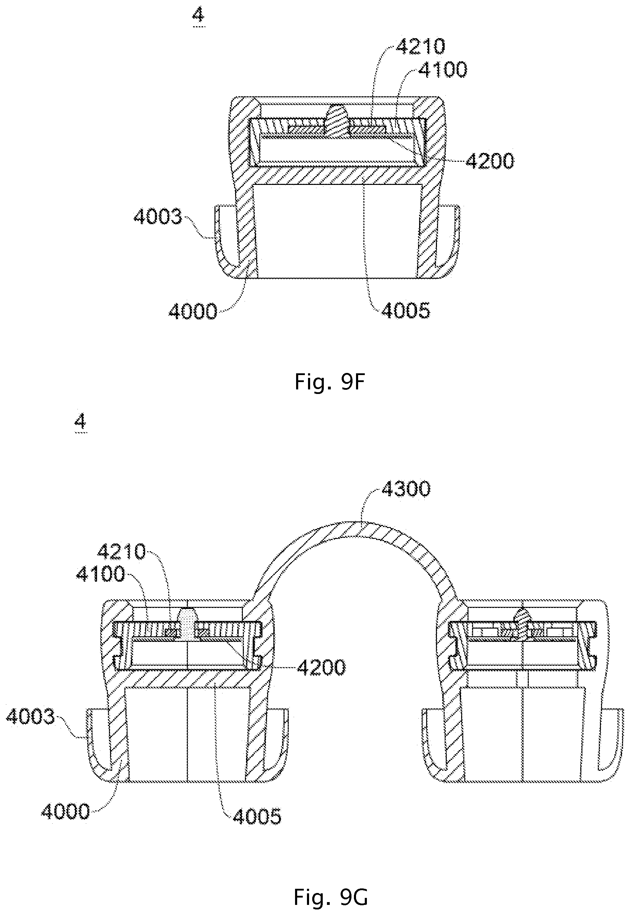

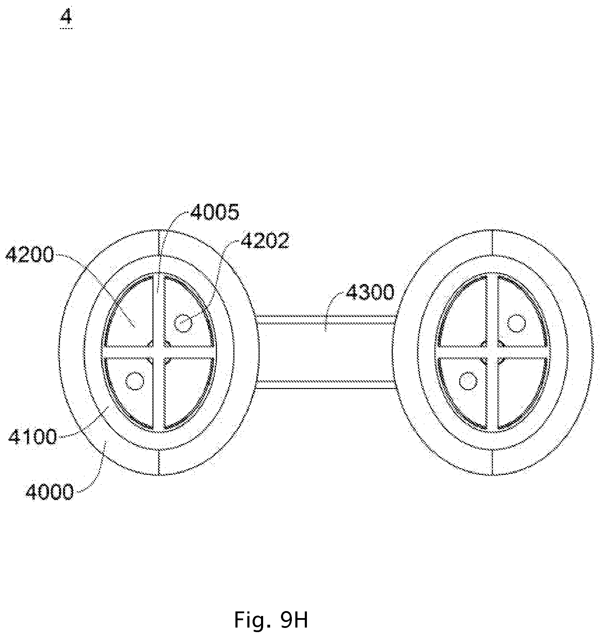

Fig. 9A through Fig. 9H are various schematic views of a nasal dilator with an EPAP valve insert 4 according to a fourth embodiment of this invention. Fig. 9A is a schematic perspective exploded view, Fig. 9B is a schematic perspective front view, Fig. 9C is a schematic perspective bottom view, Fig. 9D is a schematic top view, Fig. 9E is a schematic front view, Fig. 9F is a schematic cross-sectional view of Fig. 9E along the A-A cutting line, Fig. 9G is a schematic cross-sectional view of Fig. 9D along the B-B cutting line, and Fig. 9H is a schematic bottom view. The nasal dilator with an EPAP valve insert 4 of the fourth embodiment includes a pair of nasal dilator unit 400. Each of the pair of nasal dilator unit 400 includes a hollow nasal dilator body 4000, a valve seat 4100, a one-way flow resistance valve 4200 and a rotatable baffle 4210. The hollow nasal dilator body 4000 has a first end opening 4001 and a second end opening 4002 opposite to the first end opening 4001. The hollow nasal dilator body 4000 from the first end opening 4001 extends longitudinally to the second end opening 4002. A folded flange 4003 is disposed around an outer surrounding wall of the hollow nasal dilator body 3000 at the second end opening 4002 to enhance the degree of engagement between the hollow nasal dilator body 4000 and the nasal cavity, and supporting and dilating the nasal passage of the user. Please refer to Fig. 9A and Fig. 9H, an annular step surface 4004 is provided at an inner surrounding wall of the hollow nasal dilator body 4000 close to the first end opening 4001. A cross-shaped stent 4005 connects with the annular step surface 4004 to form a platform inside the hollow nasal dilator body 4000 in order that the cross-shaped stent 4005 may prevent the one-way flow resistance valve 4200 from dropping in the nasal cavity once the one-way flow resistance valve 4200 drops off from the valve seat 4100 during the inhalation of the user. A pair of first engaging block 4006 opposite to each other is provided at the inner surrounding wall of the hollow nasal dilator body 4000 and on the annular step surface 4004. A pair of second engaging block 4007 opposite to each other is provided at the inner surrounding wall of the hollow nasal dilator body 4000 close to the first end opening 4001 and corresponding to the pair of first engaging block 4006. The pair of first engaging block 4006 and the pair of second engaging block 4007 are used for engaging the valve seat 4100 so that the valve seat 4100 may be disposed inside the hollow nasal dilator body 4000 firmly, see Fig. 9B. Please refer to Fig. 9A, the valve seat 4100 has a hollow valve seat body having a first hollow annular portion 4110 and a second hollow annular portion 4120 as well as a hollow middle portion 4130 connecting with the first hollow annular portion 4110 and the second hollow annular potion 4120. The first hollow annular portion 4110, the second hollow annular portion 4120 and the hollow middle portion 4130 have a common opening 4100a. A pair of recess 4110a opposite to each other is provided at an outer surrounding wall of the first hollow annular portion 4110 for engaging the second engaging blocks 4007 provided at the inner surrounding wall of the hollow nasal dilator body 4000. A pair of recess 4120a opposite to each other is provided at an outer surrounding wall of the second hollow annular portion 4120 for engaging the first engaging blocks 4006 provided at the inner surrounding wall of the hollow nasal dilator body 4000. The first hollow annular part further comprises a valve connecting portion 4140 disposed at the common opening 4100a of the first hollow annular portion 4110 for assembling the one-way flow resistance valve 4200 and the rotatable baffle 4210. Several blocks 4110b spaced from the valve connecting portion 4140 may be provided at the inner surrounding wall of the first hollow annular portion 4110. The one-way flow resistance valve 4200 has at least a radial cut 4201 and a pair of air vent 4202 spaced from the radial cut 4201. An outer diameter of the one-way flow resistance valve 4200 is larger than a diameter of the common opening 4100a of the valve seat 4100. The one-way flow resistance valve 4200 may be a check valve. The one-way flow resistance valve 4200 may be made of flexible material so that the one-way flow resistance valve 4200 may be deformed under action of air flow pressure. Moreover, the radial cut 4201 assists the one-way flow resistance valve 4200 deformed along the air flow direction. The rotatable baffle 4210 has rib end portions 4210a, 4210b opposite to each other. The rib end portions 4210a, 4210b may block part of opening area of the air vent 4202 of the one-way flow resistance valve 4200 to adjust air flow resistance by way of rotating the rotatable baffle 4210.

Please refer to Fig. 9B, Fig. 9D and Fig. 9H, during the inhalation of the user, the one-way flow resistance valve 4200 is in an open state to allow air flow entering the hollow nasal dilator body 4000 to let the user easily breathe. Meanwhile, air flow pressure acts at the one-way flow resistance valve 4200 to force the one-way flow resistance valve 4200 deformed to become an umbrella configuration toward the second end opening 4002 so that a gap existing between the one-way flow resistance valve 4200 and the valve seat 4100 to form a fluid channel. The air vents 4202 of the one-way flow resistance valve 4200 also become fluid channels between the hollow nasal dilator body 4000 and the outside environment. During the exhalation of the user, the air flow from the second end opening 3002 of the hollow nasal dilator body 3000 flows to the first end opening 3001, and the one-way flow resistance valve 3200 is in a closed state to prevent the air flow from passing through it, and air flow pressure forces the one-way flow resistance valve 3200 returning to the original state from the umbrella configuration and resisting against the bottom of the first hollow annular portion 4110 of the valve seat 4100 to close the common opening 4100a of the valve seat 4100, as shown in Fig. 9G and Fig. 9F. Meanwhile, air flow inside the hollow nasal dilator body 4000 only can pass through the air vents 4202 of the one-way flow resistance valve 4200 to outside of the nasal cavity. As a consequence, the exhalation flow resistance is increased relative to the inhalation flow resistance. Therefore, a positive pressure is generated inside the upper airway of the user to facilitate the upper airway keep patency to stabilize soft tissue inside the oral cavity of the user to prevent the soft tissue from collapsing.

Fig. 15C is a variance of the one-way flow resistance valve 4200 of the fourth embodiment. In this variance, the one-way flow resistance valve 1550 has two radial cut lines 1552a, 1552b and two air vents 1554 spaced from the two radial cut lines 1552a, 1552b. Fig. 15D shows a schematic top view of the assembly of the valve seat 1540 and the one-way flow resistance valve 1550.

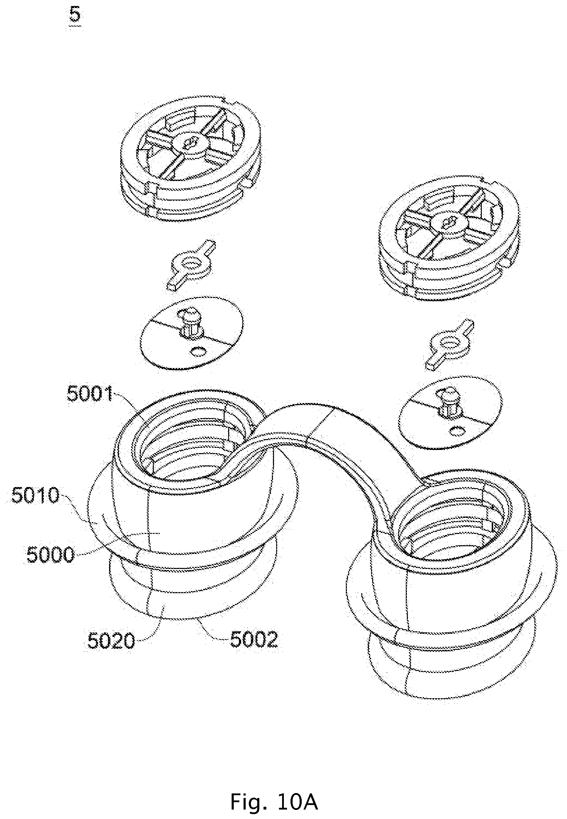

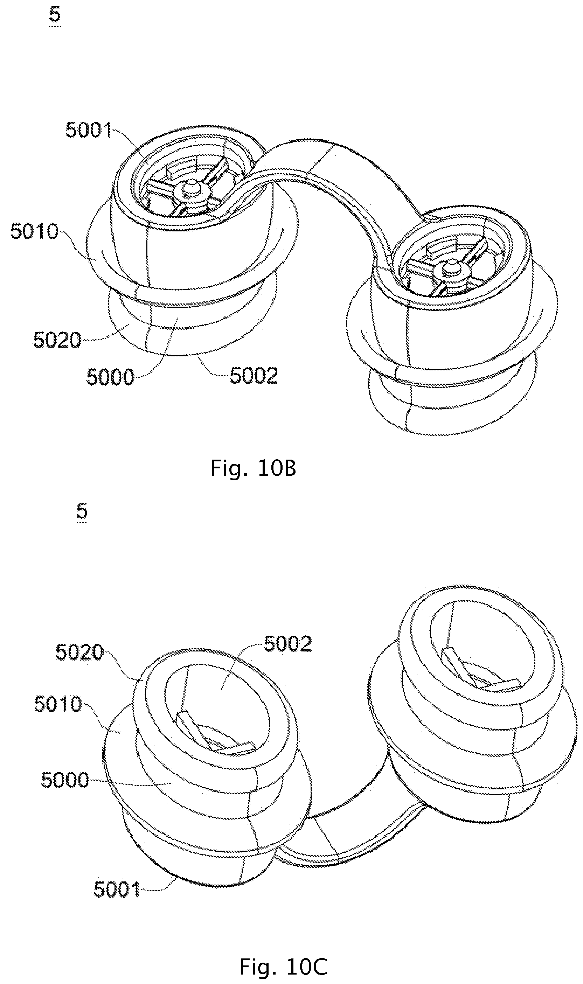

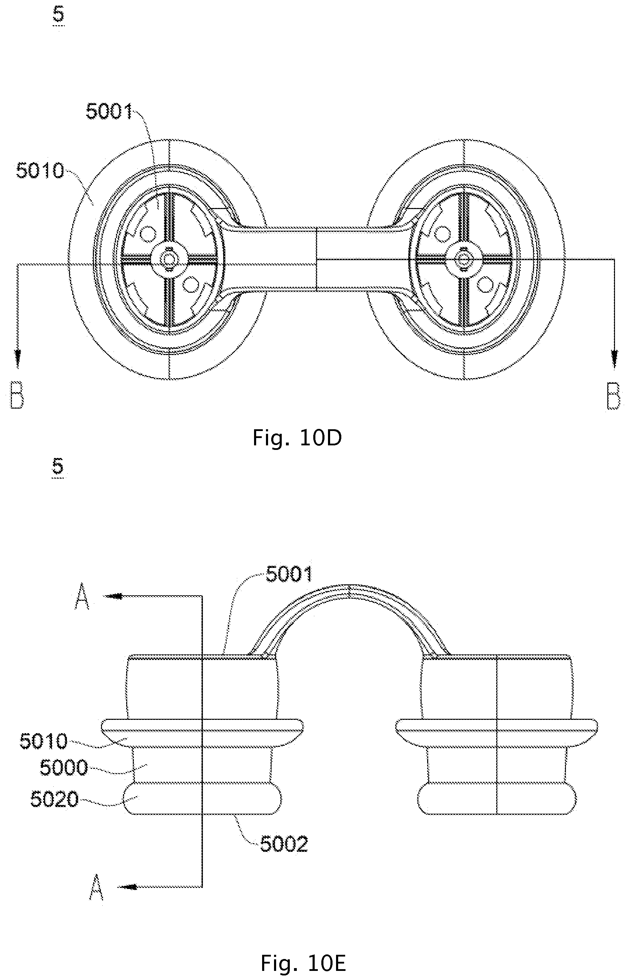

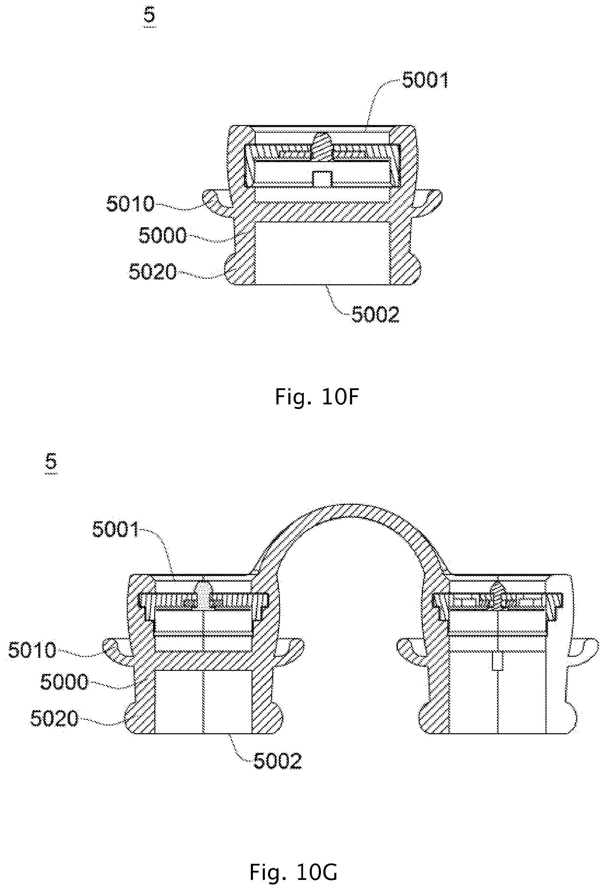

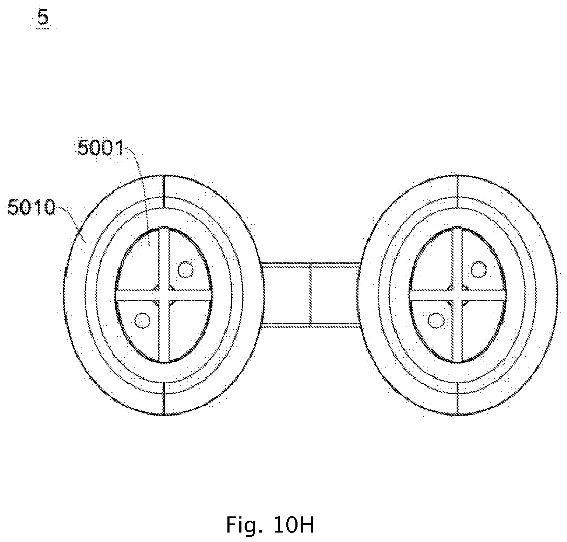

Fig. 10A through Fig. 10H are various schematic views of a nasal dilator with an EPAP valve insert 5 according to a fifth embodiment of this invention. Fig. 10A is a schematic perspective exploded view, Fig. 10B is a schematic perspective front view, Fig. 10C is a schematic perspective bottom view, Fig. 10D is a schematic top view, Fig. 10E is a schematic front view, Fig. 10F is a schematic cross-sectional view of Fig. 10E along the A-A cutting line, Fig. 10G is a schematic cross-sectional view of Fig. 10D along the B-B cutting line, and Fig. 10H is a schematic bottom view. The difference between the fifth embodiment and fourth embodiment only resides in a flange shape around an outer surrounding wall of the hollow nasal dilator body and its number. In the fifth embodiment, a first annular flange 5010 and a second annular flange 5020 are disposed around the outer surrounding wall of the hollow nasal dilator body 5000. An outer diameter of the first annular flange 5010 is larger than an outer diameter of the second annular flange 5020. The first annular flange 5010 is disposed around a middle portion of the outer surrounding wall of the hollow nasal dilator body 5000, while the second opening 5020 is disposed around the second opening 5002 so that the first annular flange 5010 is closer to the first end opening 5001 of the hollow nasal dilator body 5000 relative to the second annular flange 5020. When a user wears the nasal dilator with an EPAP valve insert 5, as needed, the portion of the hollow nasal dilator body 5000 from the first annular flange 5010 to the first end opening 5001 may be exposed outside the nasal cavity to let the first annular flange 5010 resisting against the nose. Or, as needed, the first annular flange 5010 placed inside the nasal cavity to enhance the degree of engagement between the hollow nasal dilator body 5000 and the nasal cavity, and supporting and dilating the nasal passage of the user.

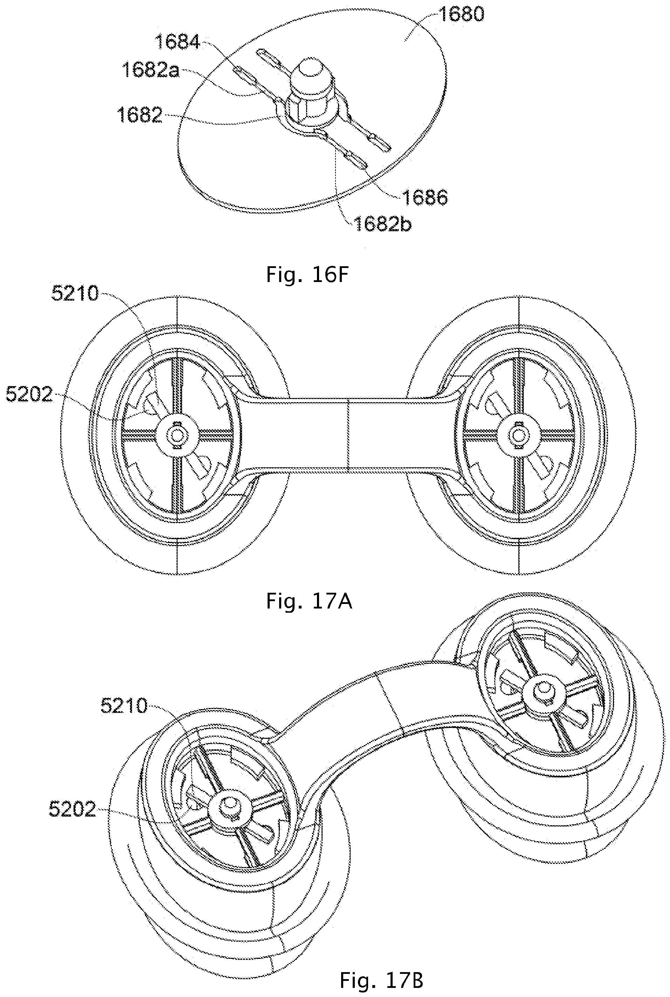

Please refer to Fig. 17A and Fig. 17B, by way of rotating the rotatable baffle 5210, air vents 5210 may be partially blocked to adjust air flow resistance for the fourth and fifth embodiments.

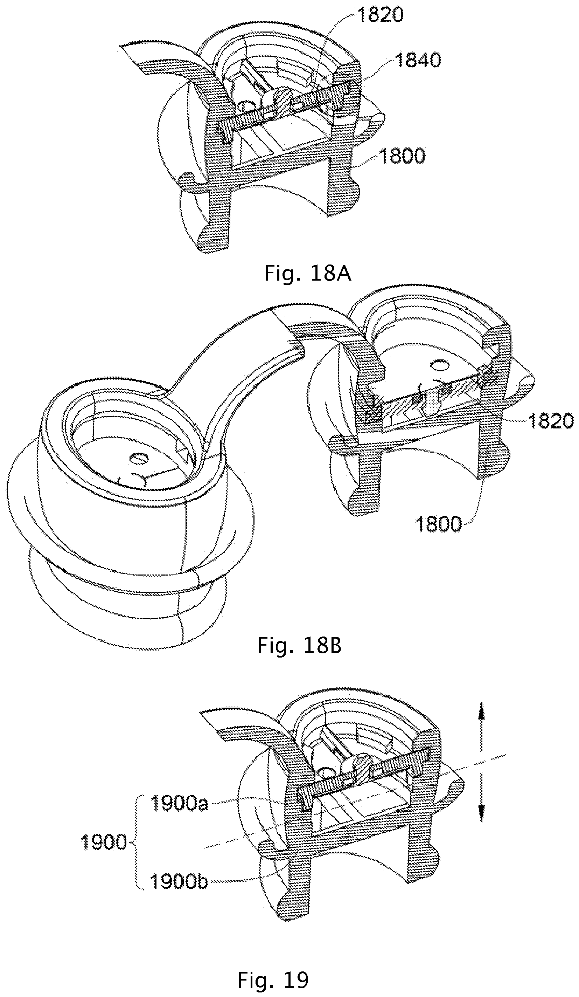

Besides, the engaging mechanism between the outer surrounding wall of the valve seat and the inner surrounding wall of the hollow nasal dilator body, such as an engaging mechanism composed of a recess and a protrusion, is not only beneficial to the connection between the valve seat and the hollow nasal dilator body, the engagement mechanism can also provide a foolproof mechanism to guide the assembly direction of the valve seat and the one-way flow resistance valve in the hollow nasal dilator body to ensure that the valve seat and the one-way flow resistance valve are correctly assembled into the hollow nasal dilator body. Please refer to Fig. 18A and Fig. 18B, Fig. 18A illustrates a valve seat 1820 via guidance of an engaging mechanism 1840 is correctly and smoothly assembled in a hollow nasal dilator body 1800, while Fig. 18B illustrates when the valve seat 1820 is assembled in the hollow nasal dilator body 1800 in an reverse direction, components of the engaging mechanism 1840 cannot match each other, and leading to an unsmooth assembling process which indicates the assembling direction may be incorrect.

Please refer to Fig. 19, the hollow nasal dilator body of this invention may have different materials, for example, one portion 1900a of the hollow nasal dilator body 1900 pointed by an upper arrow above the dotted line may be made of a hard material to enhance supporting strength of the hollow nasal dilator body 1900, while another portion 1900b of the hollow nasal dilator body 1900 pointed by a lower arrow below the dotted line may be made of a soft material to improve a user's wearing comfort.

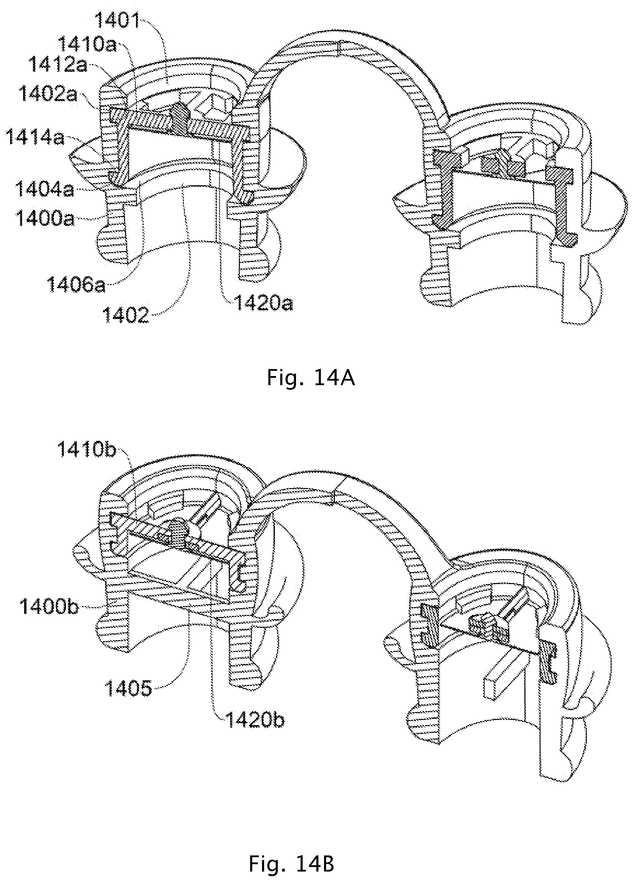

Fig. 14A is a schematic perspective cross-sectional view of a variance of the nasal dilator with an EPAP valve insert 5 of the fifth embodiment. In this variance, an engagement relationship between a valve seat 1410a and a hollow nasal dilator body 1400a is that a top outer peripheral edge and a bottom outer peripheral edge of the valve seat 1410a are respectively provided with a pair of opposing first protrusion 1412a and a pair of opposing second protrusion 1414a, and the inner surrounding wall of the hollow nasal dilator body 1400a is respectively provided with a pair of first recess 1402a and a pair of second recess 1404a corresponding to the first protrusions 1412a and the second protrusions 1414a of the valve seat 1410a, so as to facilitate the assembly of the valve seat 1410a in the hollow nasal dilator body 1400a. Besides, an annular step surface 1406a is provided at the inner surrounding wall of the hollow nasal dilator body 1400a under the second recesses 1414a so that a diameter of a top opening 1401 of the hollow nasal dilator body 1400a is larger than a diameter of an opening 1402 of the hollow nasal dilator body 1400a at the step surface 1406a such that the exhalation flow resistance may be further increased to increase the positive pressure inside the nasal cavity. The annular step surface 1406a may replace the cross stent. The step surface 1406a also can support a one-way flow resistance valve 1420a to prevent the one-way flow resistance valve 1420a from dropping in the nasal cavity.

Fig. 14B is a schematic perspective cross-sectional view of another variance of the nasal dilator with an EPAP valve insert 5 of the fifth embodiment. In this variance, a longitudinal length of a valve sear 1410b is shorter than that of the valve seat 1410a, but an engagement relationship between the valve seat 1410b and a hollow nasal dilator body 1400b is similar to that of the variance of Fig. 14A. For the variance of Fig. 14B, a cross stent 1405 is provided inside the hollow nasal dilator body 1400b and joining the inner surrounding wall of the hollow nasal dilator body 1400b to provide a support means for the one-way flow resistance valve 1420b to prevent the one-way flow resistance valve 1402b from dropping in the nasal cavity.

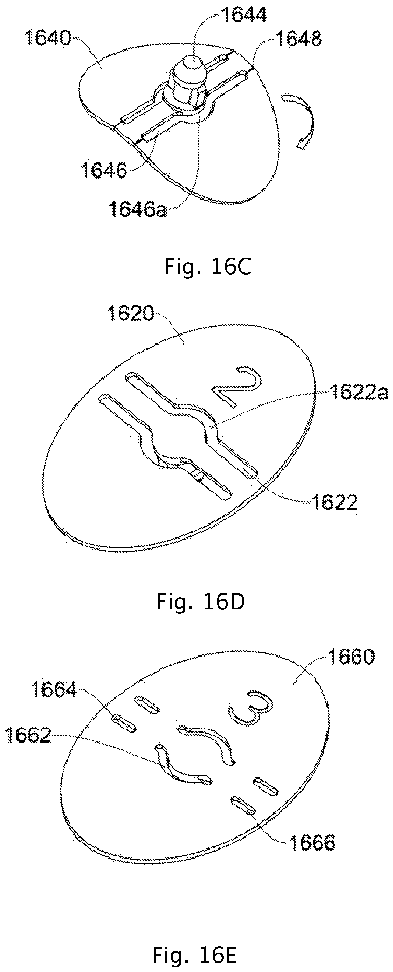

Fig. 16A is a schematic assembly view of a valve seat 1600 and a one-way flow resistance valve 1620 according to a variance of the nasal dilator with an EPAP valve insert 5 of the fifth embodiment. In this variance, the one-way flow resistance valve 1620 adopts a design concept in which an air vent and a radial cutting line are integrated into one. The one-way flow resistance valve 1620 has a central positioning post 1624 to let the one-way flow resistance valve 1620 to assemble to a valve connecting portion 1604 of the valve seat 1600. The one-way flow resistance valve 1620 has a pair of elongated slit type air vent 1622 opposite to each other. Each of the elongated slit type air vents 1622 has a curved middle portion 1622a around a periphery of the central positioning post 1624, and the elongated slit type air vent 1622 longitudinally extends from two ends of the curved middle portion 1622a toward the periphery of the one-way flow resistance valve 1620. The elongated slit type air vent 1622 integrates functions of an air vent and a cutting line. When air flow pressure acts at the one-way flow resistance valve 1620, the elongated slit type air vents 1622 assist the one-way flow resistance valve 1620 deflectively deformed, or return to the original state from the deformed state. During the inhalation of the user, the one-way flow resistance valve 1620 is in an open state to allow air flow entering the hollow nasal dilator body to let the user easily breathe. Meanwhile, air flow pressure acts at the one-way flow resistance valve 1620 to force the one-way flow resistance valve 1620 deformed to become an umbrella configuration toward the hollow nasal dilator body so that a gap existing between the one-way flow resistance valve 1620 and the valve seat 1600 to form a fluid channel. The elongated slit type air vents 1622 of the one-way flow resistance valve 1620 become fluid channels between the hollow nasal dilator body and outside environment. During the exhalation of the user, the air flow from the hollow nasal dilator body flows to the one-way flow resistance valve 1620, and the one-way flow resistance valve 1620 is in a closed state to prevent the air flow from passing through it, and air flow pressure forces the one-way flow resistance valve 1620 returning to the original state from the umbrella configuration to close an opening 1603 of the valve seat 1600. Meanwhile, air flow inside the hollow nasal dilator body only can pass the elongated slit type air vents 1622 of the one-way flow resistance valve 1620 to outside of the nasal cavity. As a consequence, the exhalation flow resistance is increased relative to the inhalation flow resistance. Therefore, a positive pressure is generated inside the upper airway of the user to facilitate the upper airway keep patency to stabilize soft tissue inside the oral cavity of the user to prevent the soft tissue from collapsing. Moreover, the rotatable baffle 4210 shown in Fig. 9A is omitted in this variance.

Fig. 16C, Fig. 16E and Fig. 16F show different variances of the one-way flow resistance valve 1620, in which Fig. 16C and Fig. 16F are schematic perspective views, Fig. 16E shows a schematic bottom view. Fig. 16C shows a one-way flow resistance valve 1640 having a pair of elongated slit type air vent 1646 opposite to each other and a central positioning post 1644 to assist the one-way flow resistance valve to assemble to a valve connection part of a valve seat. Each of the elongated slit type air vents 1646 has a curved middle portion 1646a around a periphery of the central positioning post 1644, and the elongated slit type air vent 1646 longitudinally extends from two ends of the curved middle portion 1646a toward the periphery of the one-way flow resistance valve 1640. A cutting line 1648 formed between the elongated slit type air vent 1646 and the periphery of the one-way flow resistance valve 1640. The cutting lines 1648 allow the one-way flow resistance valve 1640 more easily deflected to enlarge a gap between the one-way flow resistance valve 1640 and the valve seat during the inhalation of the user to let the user more easily breathe. A one-way flow resistance valve 1660 of Fig. 16E is modifying the elongated slit type air vent 1622 of Fig. 16B to three individual sub-slit type air vents 1662, 1664, 1666 which are spaced to each other and arranged along a longitudinal direction. A one-way flow resistance valve 1680 of Fig. 16F also has three individual sub-slit type air vents 1682, 1684, 1686 which are spaced to each other and arranged along a longitudinal direction. A V-shaped cut 1682a connects between the sub-slit type air vents 1682, 1684, and a V-shaped cut 1682b connects between the sub-slit type air vents 1682 and 1686. These V-shaped cuts 1682a, 1682b allow the one-way flow resistance valve 1680 more easily deformed under action of air flow pressure.

Besides, a number marking may be provided at a bottom side of the one-way flow resistance valve of this invention to represent a model number of the one-way flow resistance valve, for example, the one-way flow resistance valve 1620 may be marked a number "2" at its bottom side, the one-way flow resistance valve 1660 may be marked a number "3" at its bottom side, so that the model number of the one-way flow resistance valve may be easily and quickly recognized.

Moreover, the one-way flow resistance valves shown in Fig. 16B, Fig. 16C, Fig. 16E and Fig. 16F may be variances of the one-way flow resistance valves of the fourth embodiment shown in Fig. 9 and the fifth embodiment shown in Fig. 10.

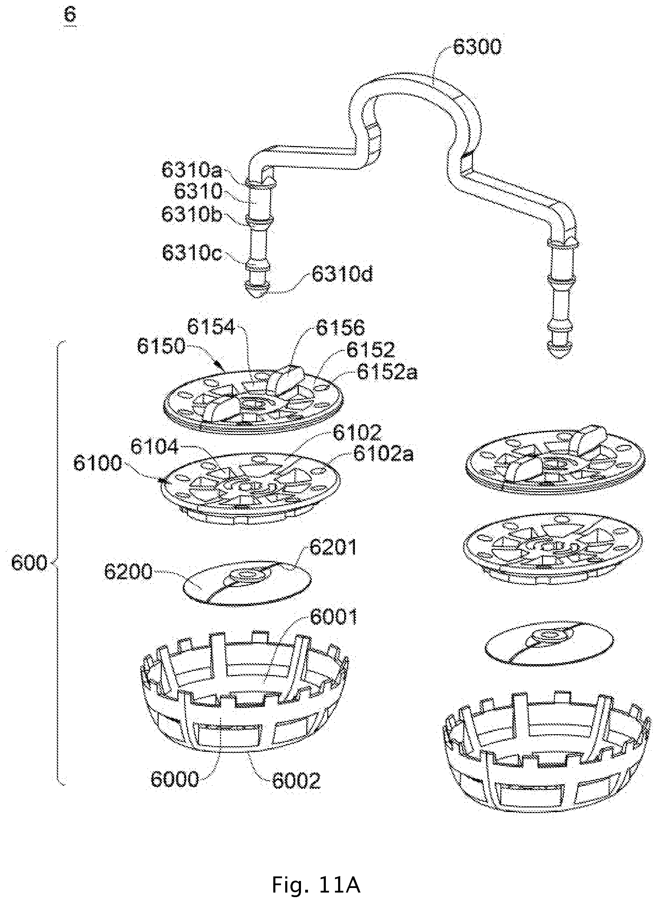

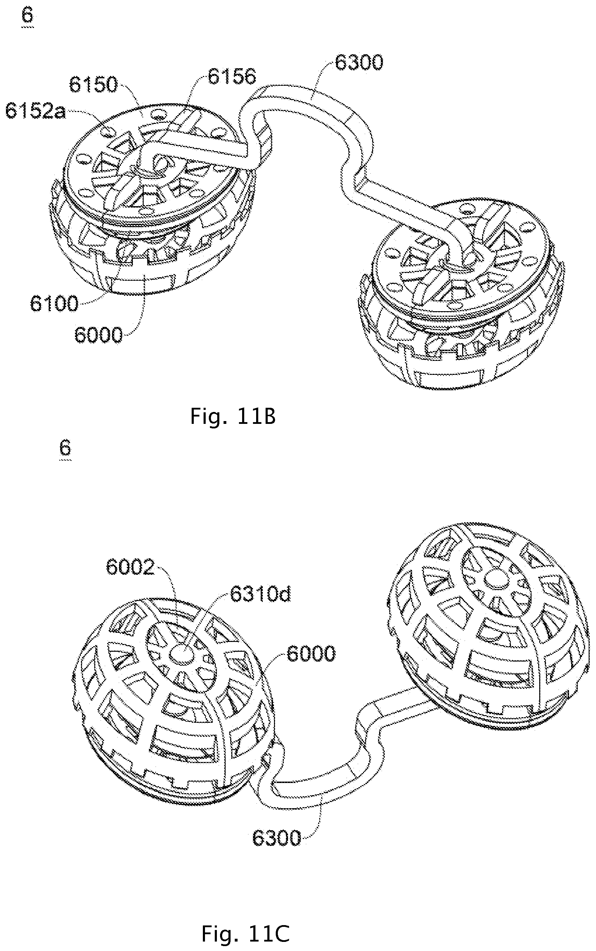

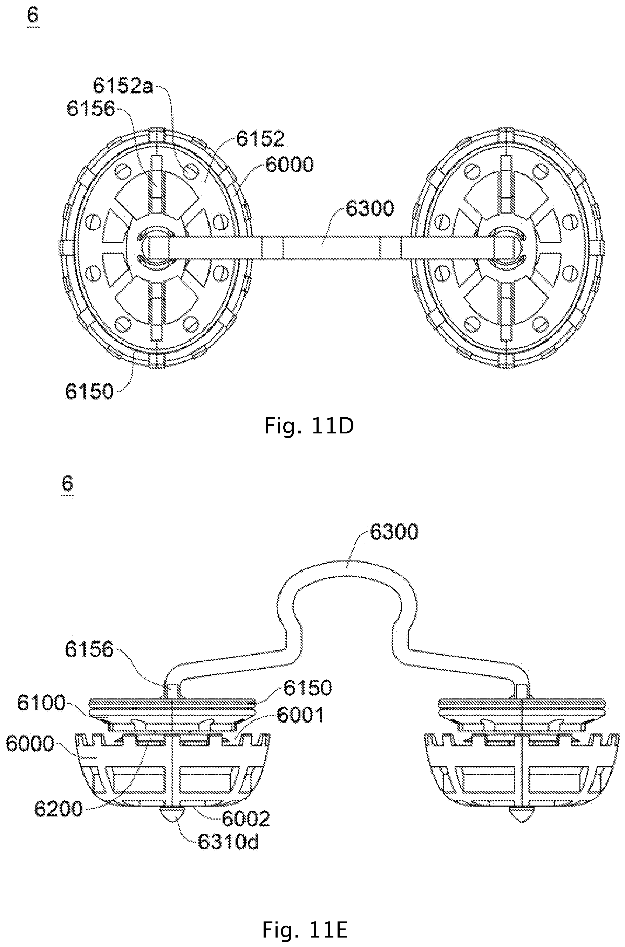

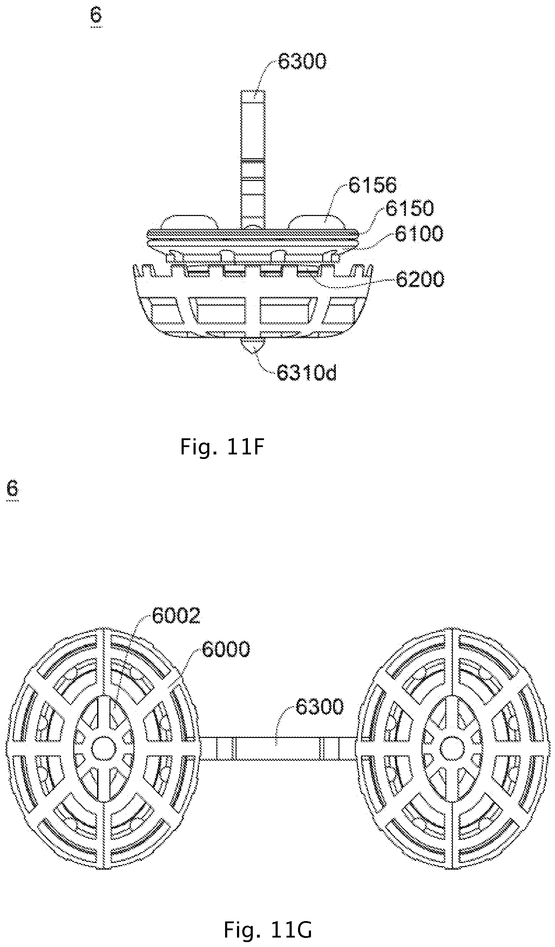

Fig. 11A through Fig. 11G are various schematic views of a nasal dilator with an EPAP valve insert 6 according to a sixth embodiment of this invention. Fig. 11A is a schematic perspective exploded view, Fig. 11B is a schematic perspective front view, Fig. 11C is a schematic perspective bottom view, Fig. 11D is a schematic top view, Fig. 11E is a schematic front view, and Fig. 11F is a schematic side view. The nasal dilator with an EPAP valve insert 6 includes a pair of nasal dilator unit 600. Each of the pair of nasal dilator unit 600 includes a hollow carved nasal dilator body 6000, a valve seat 6100, a flow resistance adjustment element 6150 and a one-way flow resistance valve 6200. The nasal dilator with an EPAP valve insert 6 further comprises a jointing piece 6300 having a pair of supporting post 6310 opposite to each other. Each of the supporting post 6310 is provided with protrusions 6310a, 6310b, 6310c, 6310d spaced from each other and arranged longitudinally around an outer surrounding wall of the supporting post 6310 to respectively position the flow resistance adjustment element 6150, the valve seat 6100, the one-way flow resistance valve 6200 and the hollow carved nasal dilator body 6000 to serially connect them to the jointing piece 6300 so that the components of the nasal dilator unit 600 may be assembled together. The hollow carved nasal dilator body 6000 has a top opening 6001 and a hollow carved bottom 6002. From a direction that the hollow carved bottom 6002 toward the top opening 6001, the one-way flow resistance valve 6200 and the valve seat 6100 are assembled in the hollow carved nasal dilator body 6000 close to the top opening 6001, while the flow resistance adjustment element 6150 is assembled at the outside of the hollow carved nasal dilator body 6000 close to the top opening 6001. The valve seat 6100 has a periphery 6102 to define a hollow carved cavity 6104 and a plurality of through hole 6102a annularly arranged on the periphery of the valve seat 6100 fluidly communicates inside of the hollow carved nasal dilator body 6000. An outer diameter of the one-way flow resistance valve 6200 is substantially equal to an inner diameter of the periphery 6102 of the valve seat 6100 so that the one-way flow resistance valve 6200 at its original state may completely cover the inner diameter of the periphery 6102 but does not bock the through holes 6102a.The flow resistance adjustment element 6150 has a periphery 6152 to define a hollow carved cavity 6154. A plurality of through holes 6152a are annularly disposed on the periphery 6152 and each of the through holes 6152a corresponds to one of the through holes 6102a of the valve seat 6100 so that fluid channels are established among the through holes 6102a, 6152a and the hollow nasal dilator body 6000. The flow resistance adjustment element 6150 may have a pair opposing rotation portion 6156. A size of the fluid channels among through holes 6102a, 6152a and the hollow caved nasal dilator body 6000 may be adjusted by rotating the rotation portions 6156 to rotate the flow resistance adjustment element 6150. The one-way flow resistance valve 6200 has at least one radial cut line 6201 and may be practiced by a check valve. The one-way flow resistance valve 6200 may be made of flexible material. The one-way flow resistance valve 6200 is deformed upon action of the air flow pressure. Also, the radial cut line 6201 assists the one-way flow resistance valve 6200 deformed along the air flow direction upon action of the air flow pressure.

During the inhalation of the user, the one-way flow resistance valve 6200 is in an open state to allow air flow from the outside environment entering the hollow nasal dilator body 6000 to let the user easily breathe. Meanwhile, air flow pressure acts at the one-way flow resistance valve 6200 to force the one-way flow resistance valve 6200 deformed to become an umbrella configuration toward the hollow carved bottom 6002 to form a gap between the one-way flow resistance valve 6200 and the valve seat 6100 so that air flow from the outside environment may pass the one-way flow resistance valve 6200, the gap between the one-way flow resistance valve 6200 and the valve seat 6100 and the through holes 6102a, 6152a to enter inside of the hollow carved nasal dilator body 6000 during the inhalation of the user. During the exhalation of the user, air flow from the hollow carved bottom 6002 of the hollow carved nasal dilator body 6000 flows to the top opening 6001, and the one-way flow resistance valve 6200 is in a closed state to prevent air flow from passing it. The air flow pressure forces the one-way flow resistance valve 6200 returning to its original state from the umbrella configuration and completely cover the inner diameter of the flow resistance adjustment element 6150 such that there is no gap between the one-way flow resistance valve 6200 and the valve seat 6150. Meanwhile, air flow inside the hollow carved nasal dilator body 6000 only can pass the through holes 6102a, 6152a to the outside of the nasal cavity so that the exhalation flow resistance of the user is increased relative to the inhalation flow resistance of the user. Therefore, a positive pressure is generated inside the upper airway of the user to facilitate the upper airway keep patency to stabilize soft tissue inside the oral cavity of the user to prevent the soft tissue from collapsing. Moreover, the exhalation flow resistance of the user and the positive pressure inside the nasal cavity may be adjusted by rotating the rotation portions 6156 to rotate the flow resistance adjustment element 6150 to adjust the size of the fluid channel among through holes 6102a, 6152a and the hollow caved nasal dilator body 6000.

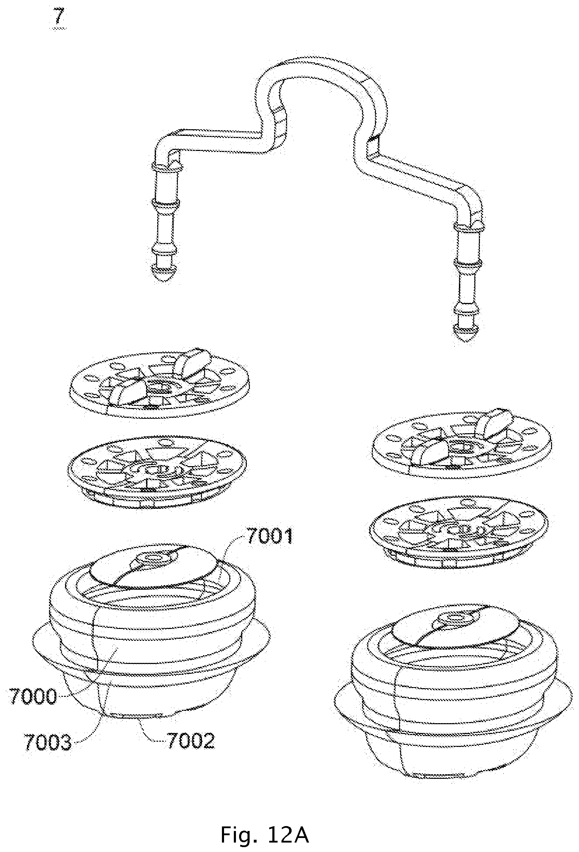

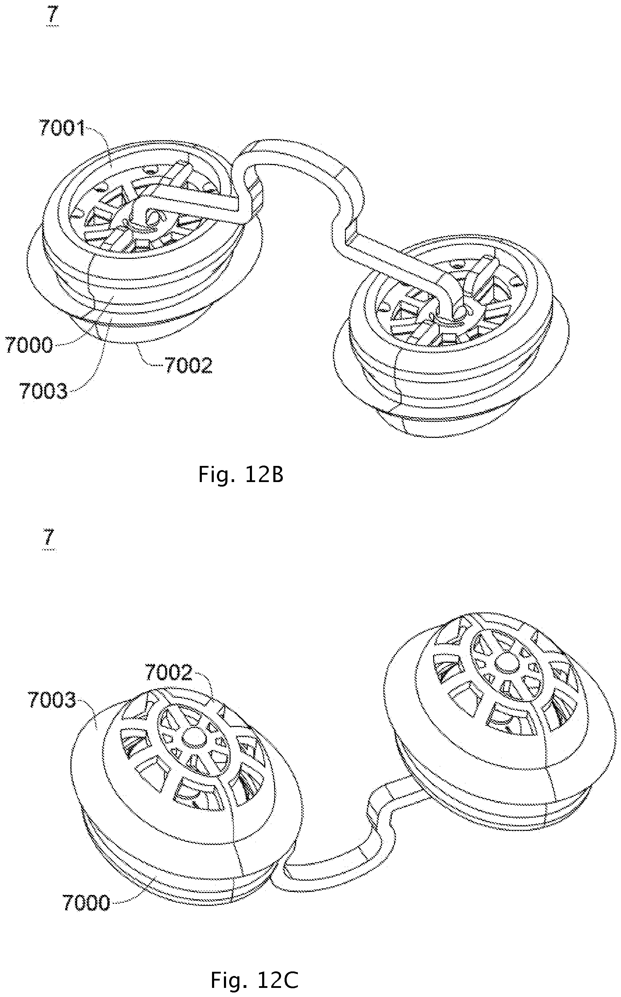

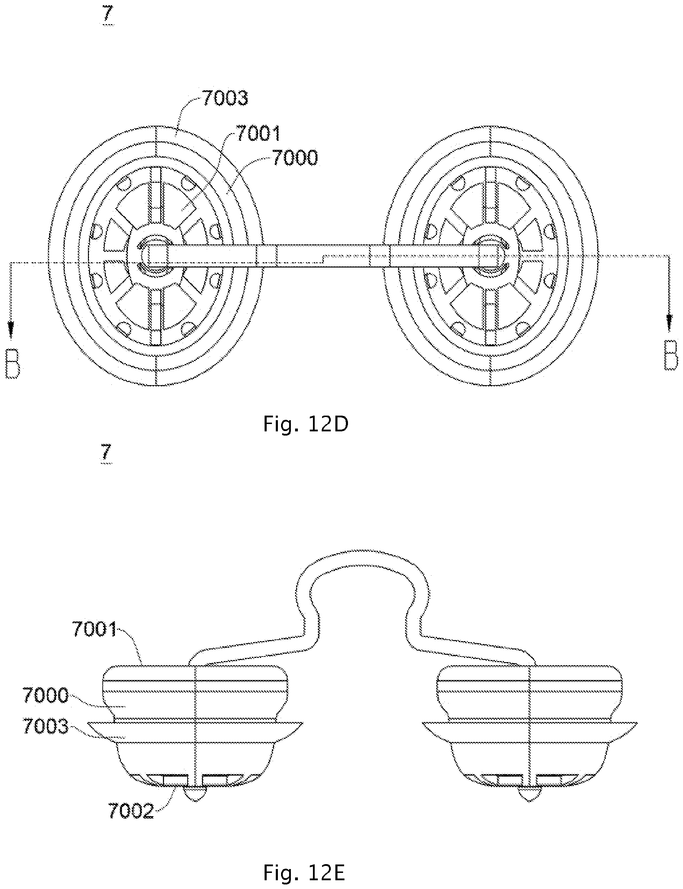

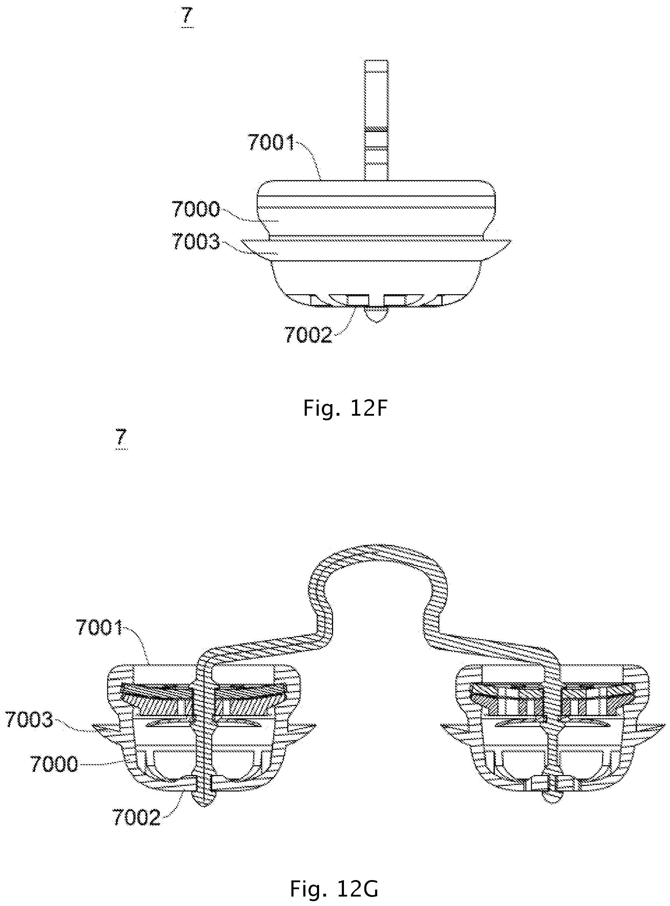

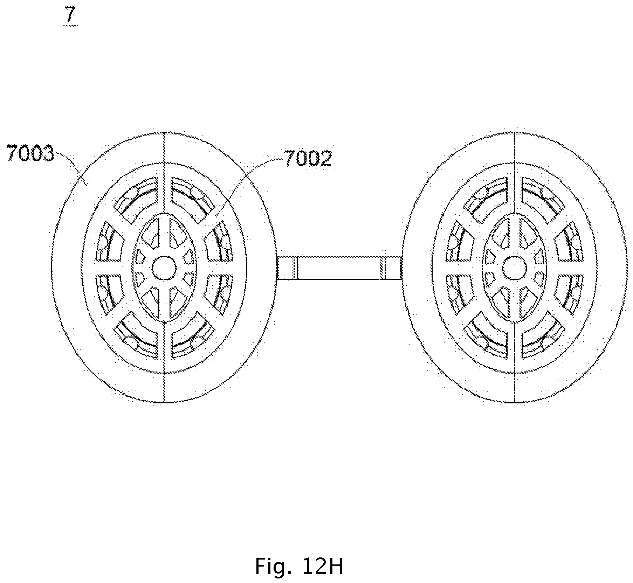

Fig. 12A through Fig. 12H are various schematic views of a nasal dilator with an EPAP valve insert 7 according to a seventh embodiment of this invention. Fig. 12A is a schematic perspective exploded view, Fig. 12B is a schematic perspective front view, Fig. 12C is a schematic perspective bottom view, Fig. 12D is a schematic top view, Fig. 12E is a schematic front view, Fig. 12F is a schematic side view, Fig. 12G is a schematic cross-sectional view of Fig. 12D along a B-B cutting line, and Fig. 12H is a schematic bottom view. The difference between the seventh embodiment and the sixth embodiment resides in the structure of the hollow nasal dilator body. In the seventh embodiment, the hollow nasal dilator body 7000 has a top opening 7001 and a hollow carved bottom 7002, and the hollow nasal dilator body 7000 longitudinally extends from the hollow carved bottom 7002 to the top opening 7001. An annular flange 7003 is provided around an outer surrounding of a middle portion of the hollow nasal dilator body 7000. When the user wears the nasal dilator with an EPAP valve insert 7, as needed, a body portion of the hollow nasal dilator body 7000 from the annular flange 7003 to the top opening 7001 is exposed outside the nasal cavity, and the annular flange 7003 resists against the outer part of the nose. Or, as needed, the annular flange 7003 may be placed inside the nasal cavity to increase the degree of engagement between the hollow nasal dilator body 7000 and the nasal cavity to support and dilate the nasal passage of the user.

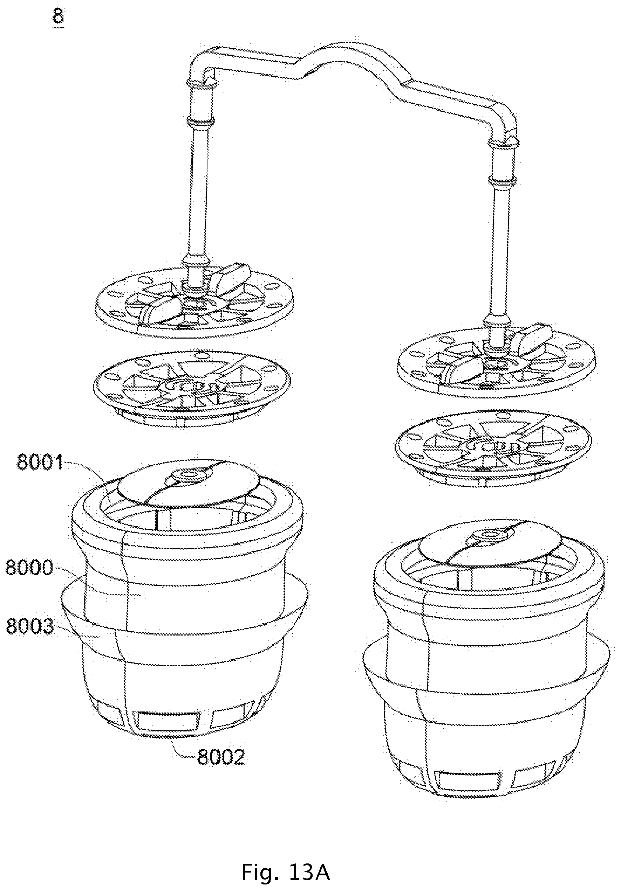

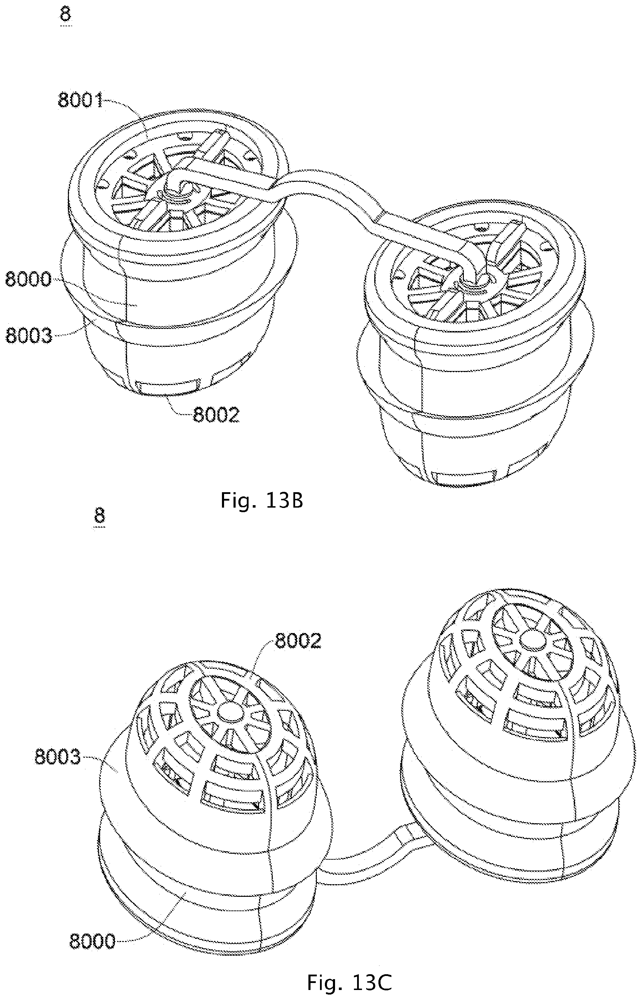

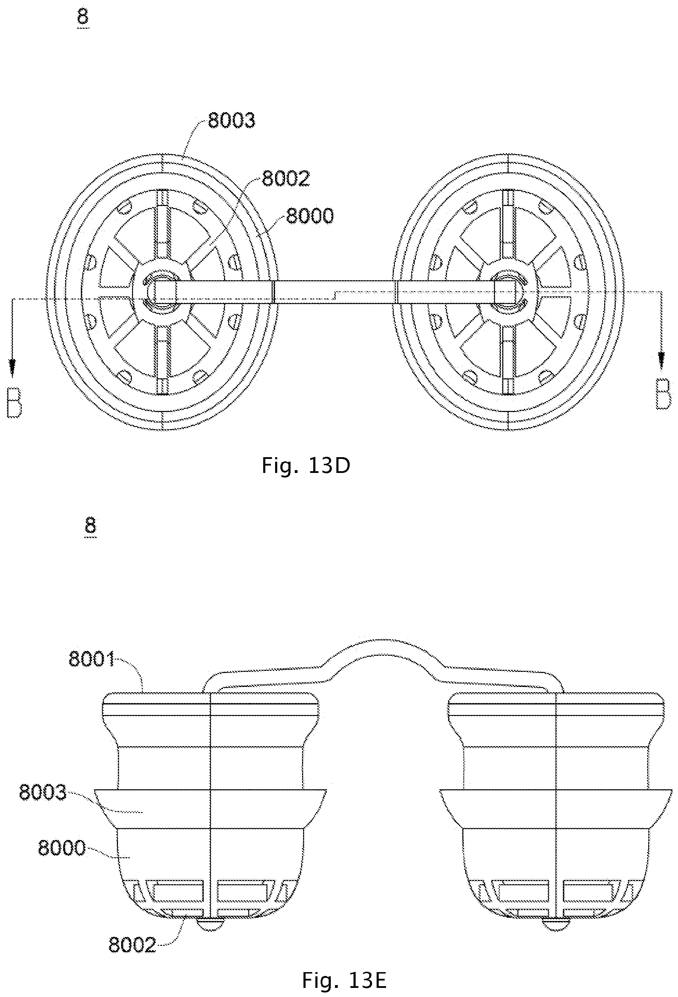

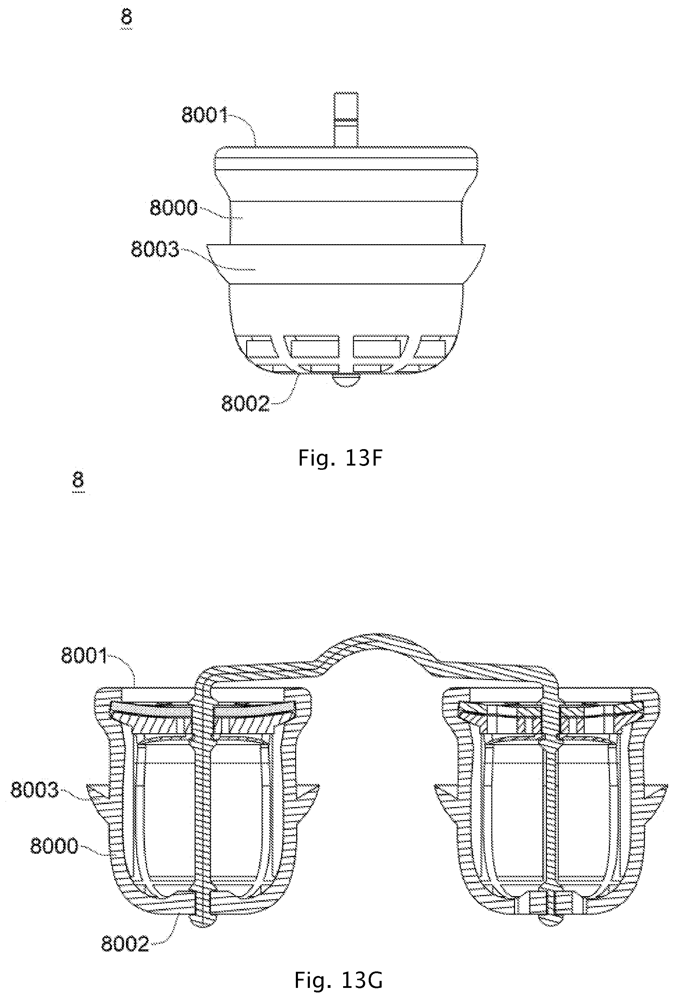

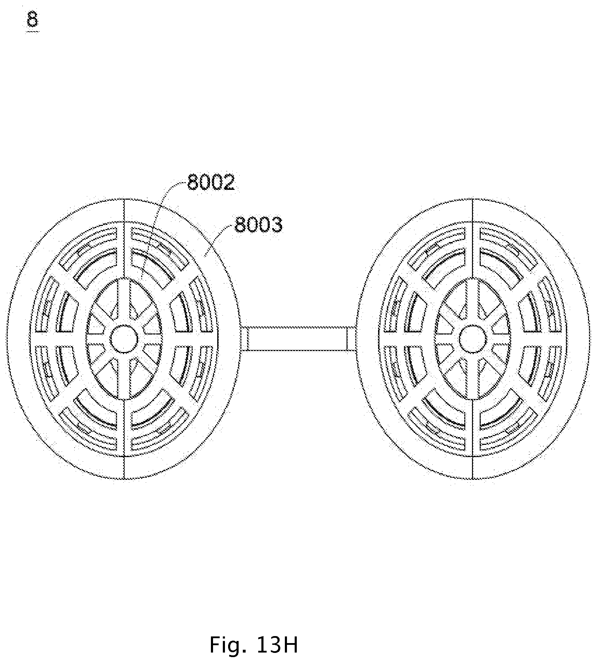

Fig. 13A through Fig. 13H are various schematic views of a nasal dilator with an EPAP valve insert 8 according to an eighth embodiment of this invention. Fig. 13A is a schematic perspective exploded view, Fig. 13B is a schematic perspective front view, Fig. 13C is a schematic perspective bottom view, Fig. 13D is a schematic top view, Fig. 13E is a schematic front view, Fig. 13F is a schematic side view, Fig. 13G is a schematic cross-sectional view of Fig. 13D along a B-B cutting line, and Fig. 13H is a schematic bottom view. The difference between the eighth embodiment and the sixth embodiment resides in the structure of the hollow nasal dilator body 8000. In the eighth embodiment, the hollow nasal dilator body 8000 has a top opening 8001 and a hollow carved bottom 8002, and the hollow nasal dilator body 8000 longitudinally extends from the hollow carved bottom 8002 to the top opening 8001. An outer surrounding of the hollow nasal dilator body 8000 near the hollow carved bottom 8002 is also hollow carved. An annular flange 8003 is provided around an outer surrounding of a middle portion of the hollow nasal dilator body 8000. When the user wears the nasal dilator with an EPAP valve insert 8, as needed, a body portion of the hollow nasal dilator body 8000 from the annular flange 8003 to the top opening 8001 is exposed outside the nasal cavity, and the annular flange 8003 resists against the outer part of the nose. Or, as needed, the annular flange 8003 may be placed inside the nasal cavity to increase the degree of engagement between the hollow nasal dilator body 8000 and the nasal cavity to support and dilate the nasal passage of the user.

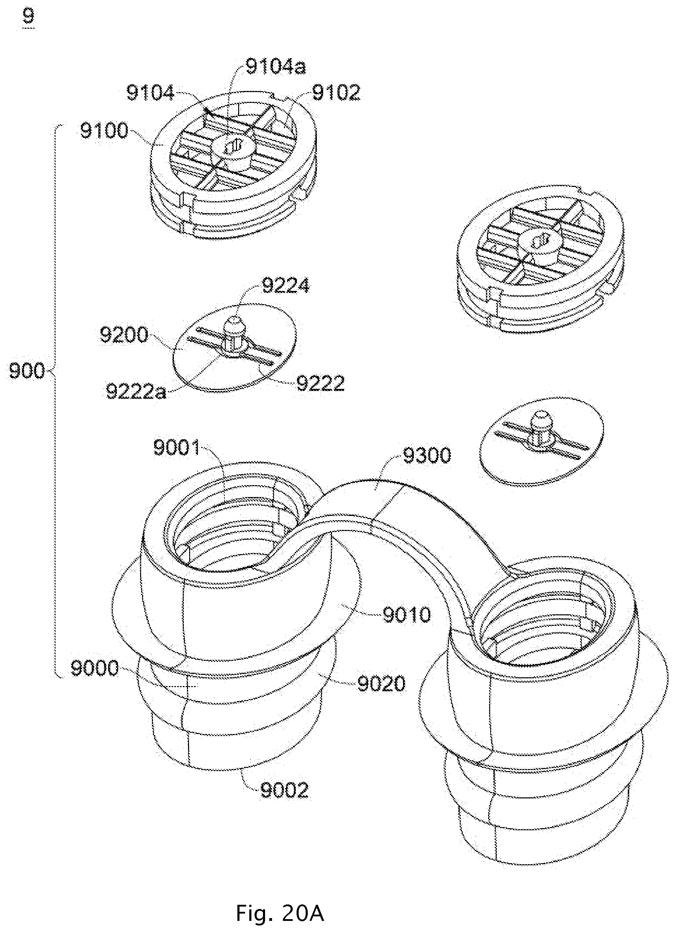

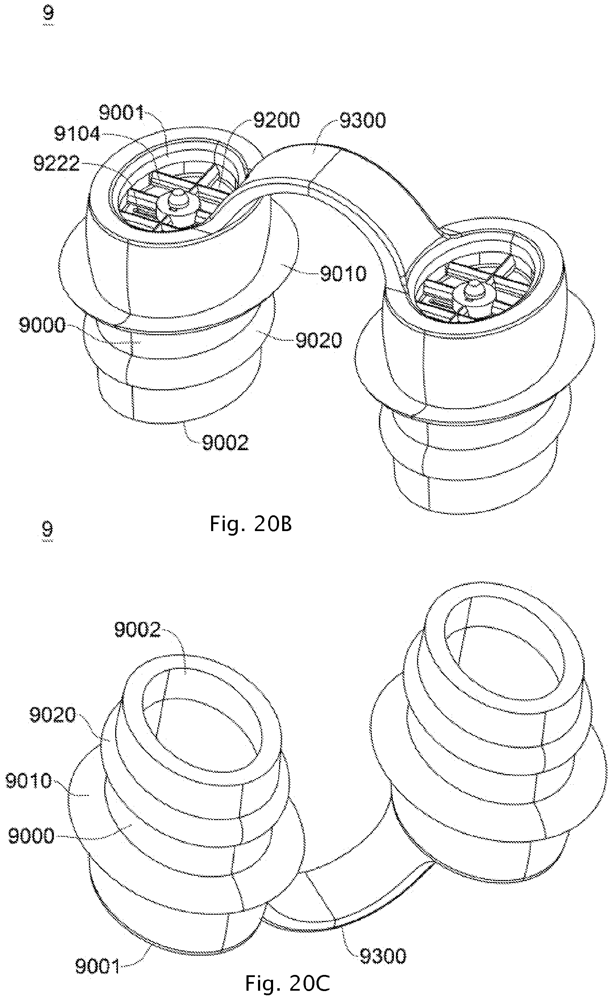

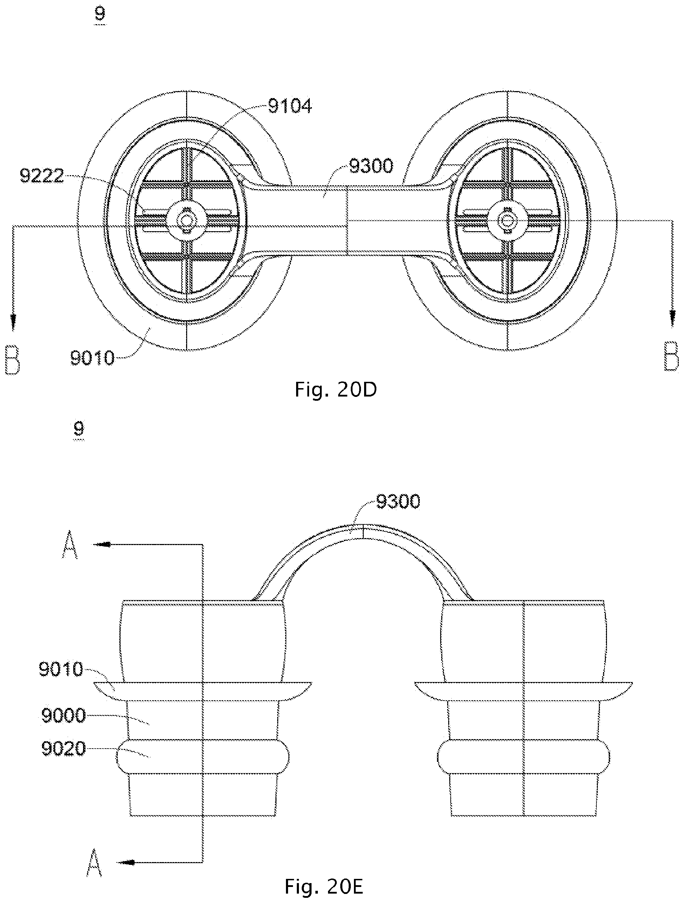

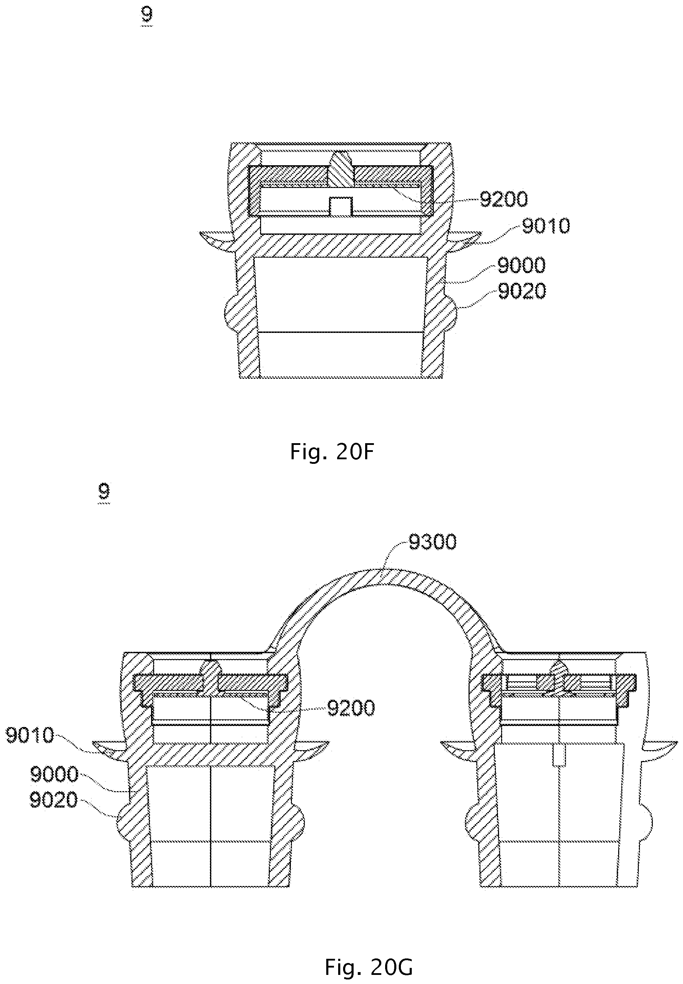

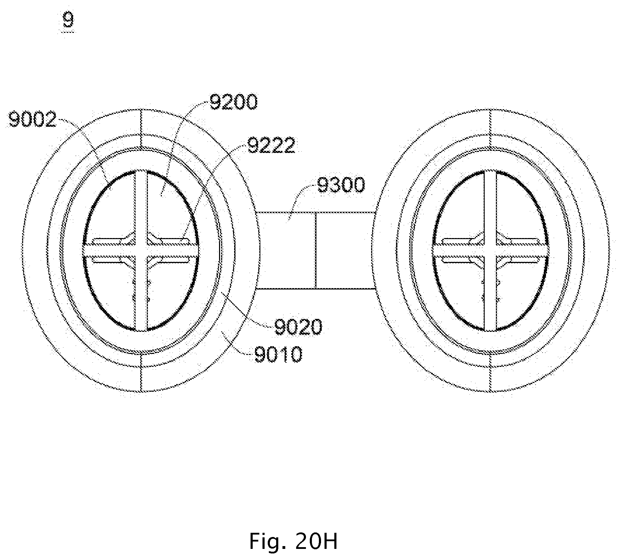

Fig. 20A through Fig. 20H are various schematic views of a nasal dilator with an EPAP valve insert 9 according to a ninth embodiment of this invention. Fig. 20A is a schematic perspective exploded view, Fig. 20B is a schematic perspective front view, Fig. 20C is a schematic perspective bottom view, Fig. 20D is a schematic top view, Fig. 20E is a schematic front view, Fig. 20F is a schematic cross-sectional view of Fig. 20E along a A-A cutting line, Fig. 20G is a schematic cross-sectional view of Fig. 20D along a B-B cutting line and Fig. 20H is a schematic bottom view. The difference between the ninth embodiment and the fifth embodiment of Fig. 10 resides in the structures of the valve seat and the one-way flow resistance valve. The nasal dilator with an EPAP valve insert 9 of the ninth embodiment includes a pair of nasal dilator unit 900. Each of the pair of the nasal dilator unit 900 includes a hollow nasal dilator body 9000, a valve seat 9100 and a one-way flow resistance valve 9200. The nasal dilator with an EPAP valve insert 9 further includes a jointing piece 9300 for connecting the pair of the nasal dilator unit 900. The hollow nasal dilator body 9000 has a first end opening 9001 and a second end opening 9002 opposite to each other. An outer surrounding of the hollow nasal dilator body 9000 is provided with a first annular flange 9010 and a second annular flange 9020 spaced from each other between the first end opening 9001 and the second end opening 9002 to increase the degree of engagement between the hollow nasal dilator body 9000 and the nasal cavity, and supporting and dilating the nasal passage of the user. The valve seat 9100 has a hollow valve seat body having a through hole 9102, a valve connecting portion 9104 connecting an inner surrounding wall of the hollow valve seat body and crossing the through hole 9102. The valve connecting portion 9104 has a fence structure. A middle positioning post 9104a of the fence structure may connect the one-way flow resistance valve 9200. The one-way flow resistance valve 9200 adopts a design concept in which an air vent and a cutting line are integrated into one. The one-way flow resistance valve 9200 has a central positioning post 9224 to assist the one-way flow resistance valve connected to the valve connecting portion 9104 of the valve seat 9100, and the valve seat 9100 and the one-way flow resistance valve 9200 are assembled inside the hollow nasal dilator body 9000 close to the first end opening 9001. The one-way flow resistance valve 9200 has a pair of elongated slit vent 9222 opposite to each other. Each of the elongated sit vents 9222 has an arc-shaped middle section 9222a around the central positioning post 9224, and the elongated slit vent 9222 longitudinally extends from two ends of the arc-shaped middle section 9222a toward a periphery of the one-way flow resistance valve 9200. The elongated slit vents 9222 integrate functions of an air vent and a cutting line. The elongated slit vents 9222 assist the one-way flow resistance valve 9200 deflectively deformed upon action of air flow pressure at the one-way flow resistance valve 9200, or assisting the one-way flow resistance valve returning to its original state. An outer diameter of the one-way flow resistance valve 9200 is larger than the top opening 9102 of the valve seat 9100. The one-way flow resistance valve 9200 may be practiced by a check valve. The one-way flow resistance valve 9200 may be made of flexible material so that the one-way flow resistance valve may be deformed upon action of air flow pressure. Moreover, the elongated slit vents 9222 may assist the one-way flow resistance valve 9200 deformed along the air flow direction.

During the inhalation of the user, the one-way flow resistance valve 9200 is in an open state to allow air flow from the outside environment entering the hollow nasal dilator body 9000 to let the user easily breathe. Meanwhile, air flow pressure acts at the one-way flow resistance valve 9200 to force the one-way flow resistance valve 9200 deformed to become an umbrella configuration toward the second end opening 9002 to form a gap as a fluid channel between the one-way flow resistance valve 9200 and the valve seat 9100. The elongated slit vents 9222 of the one-way flow resistance valve 9200 also form fluid channels between the outside environment and the hollow nasal dilator body 9000. During the exhalation of the user, air flow from the second end opening 9002 of the hollow nasal dilator body 9000 flows to the first end opening 9001, and the one-way flow resistance valve 9200 is in a closed state to prevent air flow from passing it. The air flow pressure forces the one-way flow resistance valve 9200 returning to its original state from the umbrella configuration and closing the through hole 9102 of the valve seat 9100, as shown in Fig. 20F and Fig. 20G. Meanwhile, air flow inside the hollow nasal dilator body 9000 only can pass the elongated slit vents 9222 of the one-way flow resistance valve 9200 to the outside of the nasal cavity so that the exhalation flow resistance of the user is increased relative to the inhalation flow resistance of the user. Therefore, a positive pressure is generated inside the upper airway of the user to facilitate the upper airway keep patency to stabilize soft tissue inside the oral cavity of the user to prevent the soft tissue from collapsing. Besides, during exhalation of the user, the fence structure of the valve connecting portion 9104 of the valve seat 9100 further prevents the interface between an outer periphery of the one-way flow resistance valve 9200 and an inner surrounding wall of the valve seat 9100 from air leakage to facilitate keep positive pressure inside the hollow nasal dilator body 9000.

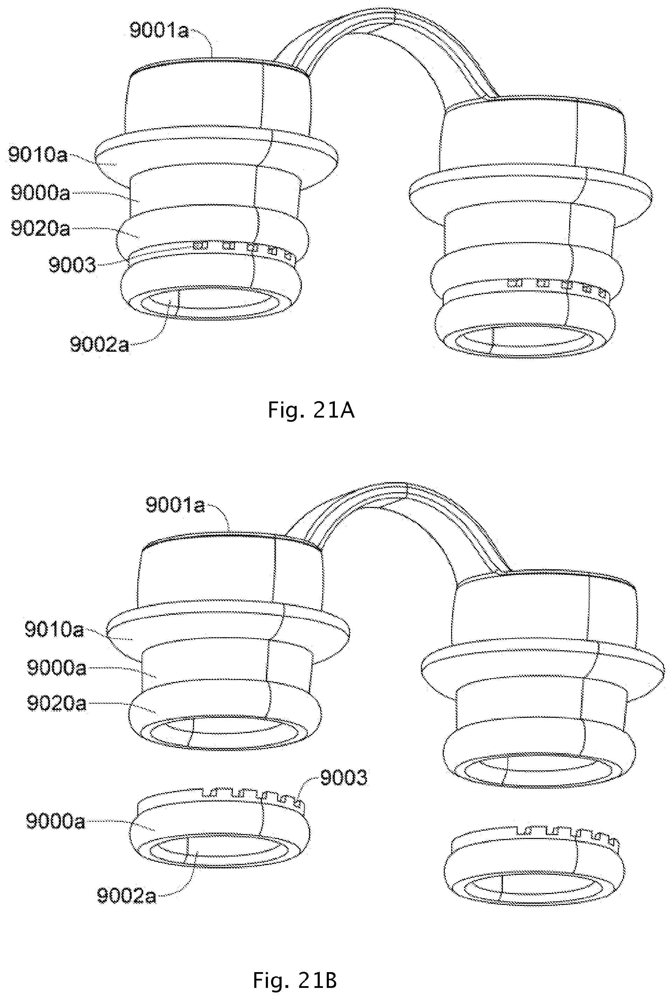

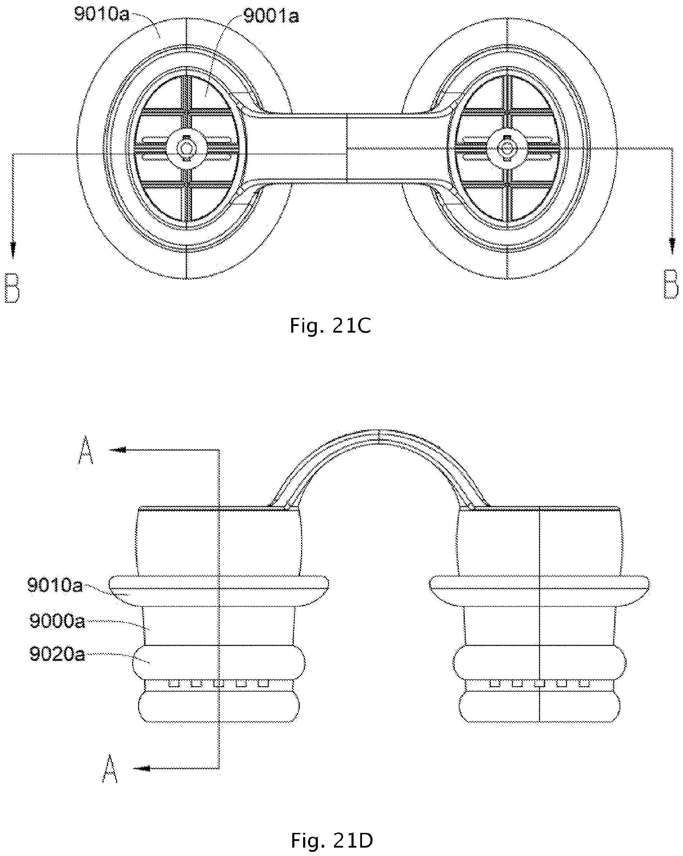

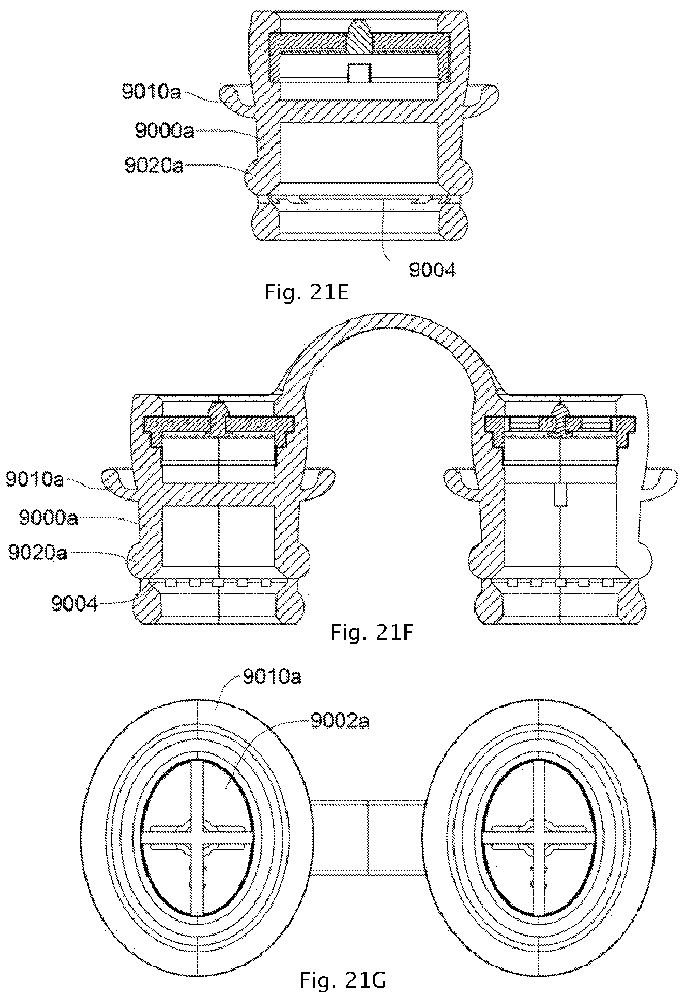

Fig. 21 is a first variance of the nasal dilator with an EPAP valve insert 9 of the ninth embodiment shown in Fig. 20. Fig. 21A is a schematic perspective view, Fig. 21B is a schematic perspective exploded view, Fig. 21C is a schematic bottom view, Fig. 21D is a schematic perspective front view, Fig. 21E is a schematic cross-sectional view of Fig. 21D along a A-A cutting line, Fig. 21F is a schematic cross-sectional view of Fig. 21C along a B-B cutting line and Fig. 21G is a schematic bottom view. In the first variance, a hollow nasal dilator body 9000a has a first end opening 9001a and a second end opening 9002a opposite to each other. The hollow nasal dilator body 9000a from the firs end opening 9001a longitudinally extends to the second end opening 9002a. A first annular flange 9010a and a second annular flange 9020a spaced from each other are provided around an outer surrounding wall of the hollow nasal dilator body 9000a between the first end opening 9001a and the second end opening 9002a. A plurality of perforation 9003 are provided around the outer surrounding wall of the hollow nasal dilator body 9000a underneath the second annular flange 9020a, and these perforations 9003 are serially connected by an easy-to-remove means 9004, see Fig. 21E and Fig. 21F. In the first variance, a portion of the hollow nasal dilator body 9000a underneath the second annular flange 9020a may be removed by way of the easy-to-remove means 9004 to be adaptable for a shorter nose.

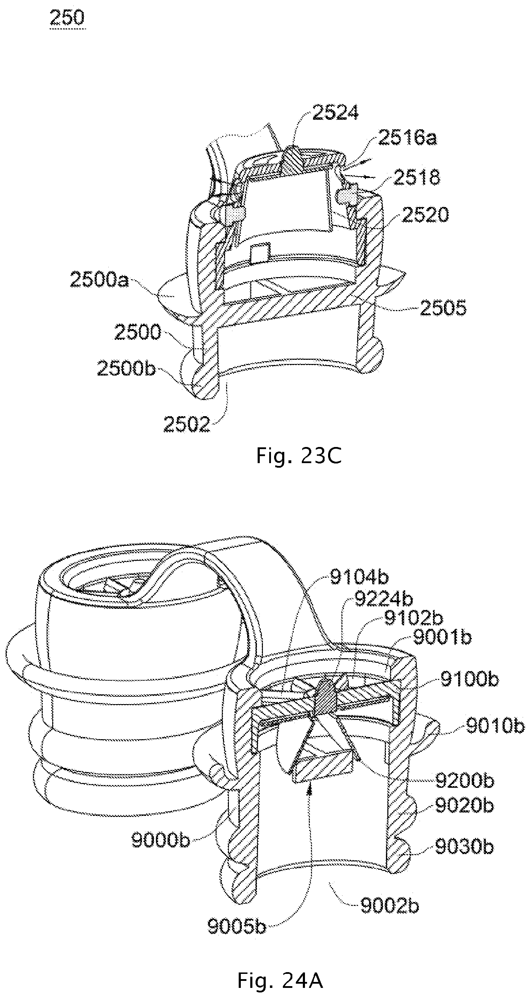

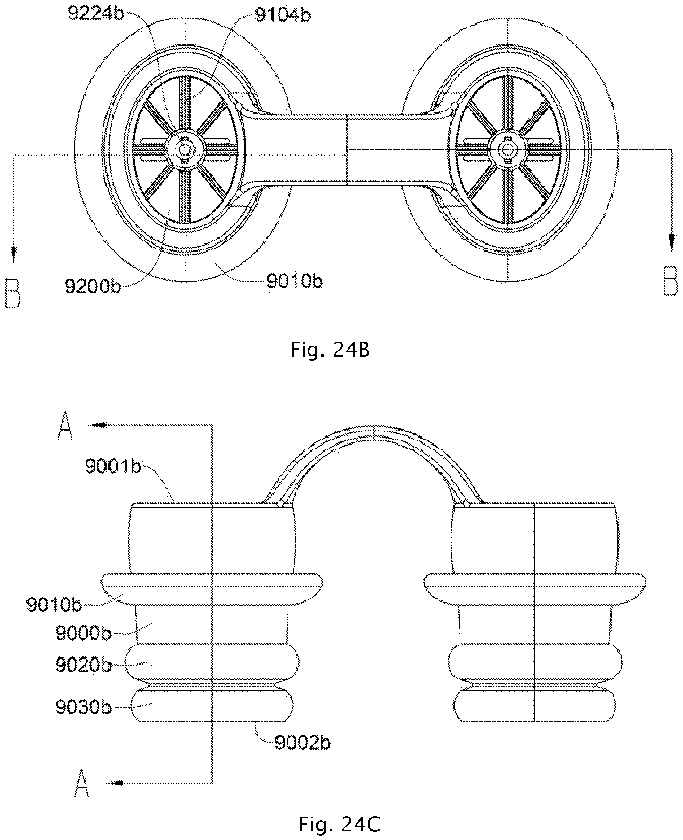

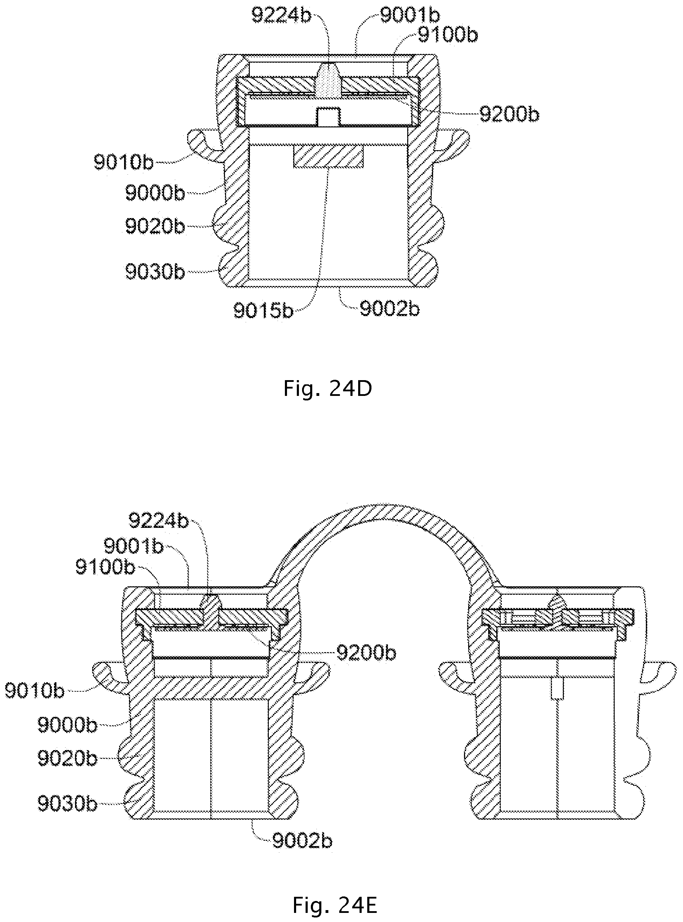

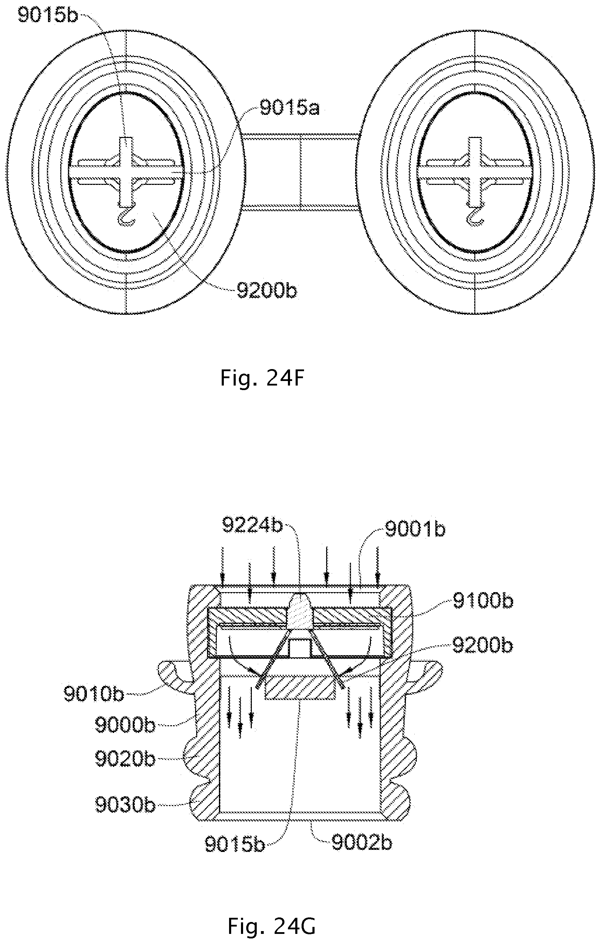

Fig. 24 is a second variance of the nasal dilator with an EPAP valve insert 9 of the ninth embodiment shown in Fig. 20. Fig. 24A is a schematic perspective view, Fig. 24B is a schematic top view, Fig. 24C is a schematic front view, Fig. 24D is a schematic cross-sectional view of Fig. 24C along a A-A cutting line, Fig. 24E is a schematic cross-sectional view of Fig. 24B along a B-B cutting line and Fig. 24F is a schematic bottom view. In the second variance, a hollow nasal dilator body 9000b has a first end opening 9001b and a second end opening 9002b opposite to each other. The hollow nasal dilator body 9000b from the first end opening 9001b longitudinally extends to the second end opening 9002b. A first annular flange 9010b, a second annular flange 9020b and a third annular flange 9030b spaced from each other are provided around an outer surrounding wall of the hollow nasal dilator boy 9000b between the first end opening 9001b and the second end opening 9002b to increase the degree of engagement between the hollow nasal dilator body 9000b and the nasal cavity. The valve seat 9100b has a hollow valve seat body having a through hole 9102b, a valve connecting portion 9104b connecting an inner surrounding wall of the hollow valve seat body and crossing the through hole 9102b. The valve connecting portion 9104b has a fence structure in a radial configuration, as shown in Fig. 24B. The one-way flow resistance valve 9200b is similar to the one-way flow resistance valve 9200 of the ninth embodiment, having a middle positioning post 9104a to connect the valve connecting portion 9104b of the valve seat 9100b. The valve seat 9100b and the one-way flow resistance valve 9200b are assembled inside of the hollow nasal dilator body 9000b close to the first end opening 9001b. Besides, the one-way flow resistance valve 9200b may be replaced by the variances of the one-way flow resistance valve shown in Fig. 16C, Fig. 16E and Fig. 16F. The hollow nasal dilator body 9000b is provided with a cross stent 9005b connecting an inner surrounding wall of the hollow nasal dilator body 9000b corresponding to the first annular flange 9100b. The cross stent 9005b has a first rib 9015a and a second rib 9015b. A length of the first rib 9015a is longer than that of the second rib 9015b, and the first rib 9015a crosses an inner diameter of the hollow nasal dilator body 9000b, while the second rib 9015b keeps a distance from the inner surrounding wall of the hollow nasal dilator body 9000b, as shown in Fig. 24F. Other structures of the second variance are similar to that of the nasal dilator with an EPAP valve insert 9.

Please refer to Fig. 24G, during the exhalation of the user, the one-way flow resistance valve 9200b is deflected toward the second end opening 9002b under action of air flow pressure, and the second rib 9015b of the cross stent 9005b may assist to increase the deflection of the one-way flow resistance valve 9200b so as to facilitate elimination of the inhalation flow resistance. Besides, the second rib 9015b may be as an umbrella stopper to effectively reduce the flapping sound of the valve piece of the one-way flow resistance valve 9200b.

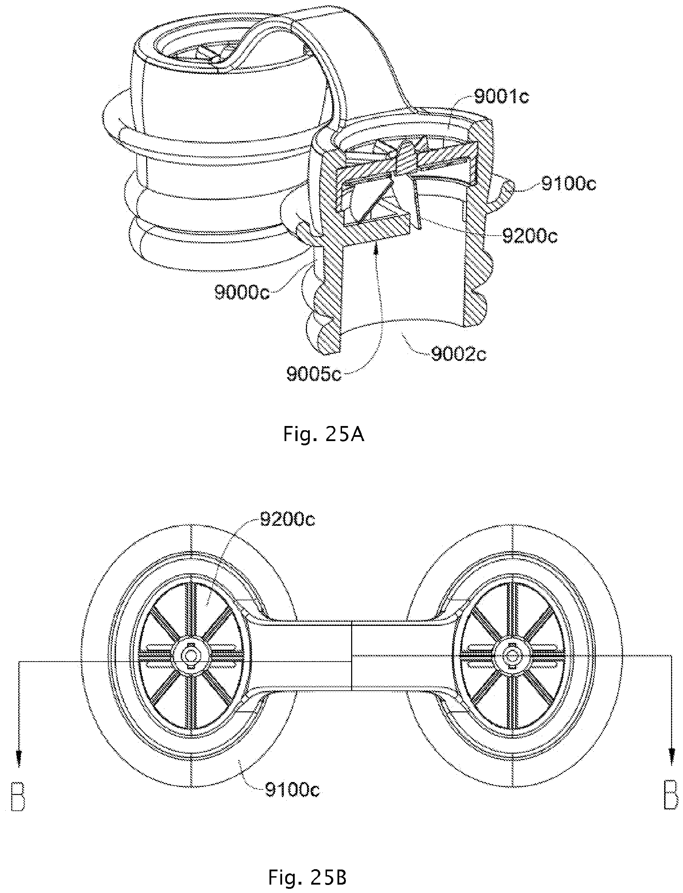

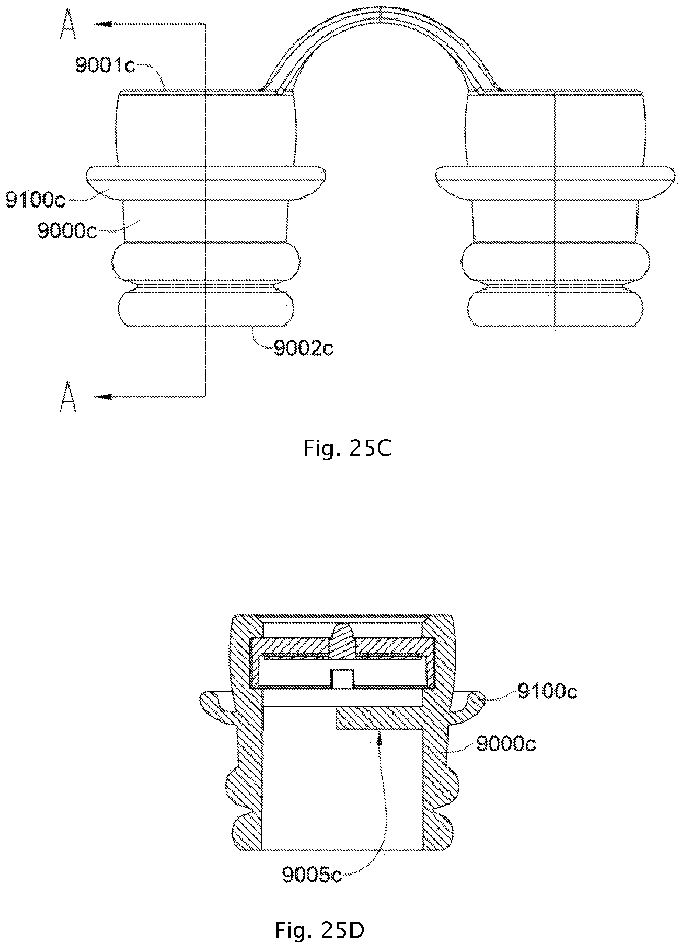

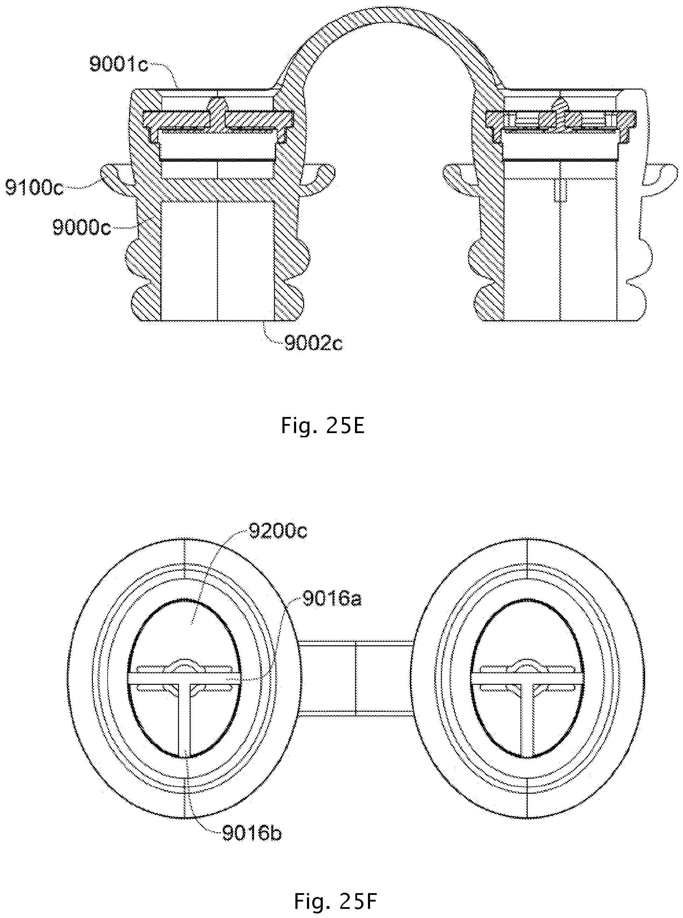

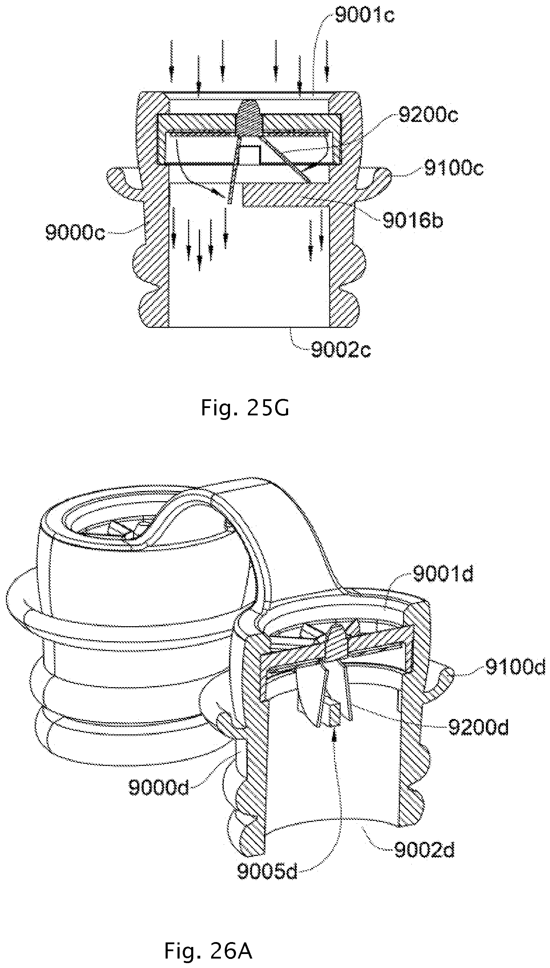

Fig. 25 is a third variance of the nasal dilator with an EPAP valve insert 9 of the ninth embodiment shown in Fig. 20. Fig. 25A is a schematic perspective partly cross-sectional view, Fig. 25B is a schematic top view, Fig. 25C is a schematic front view, Fig. 25D is a schematic cross-sectional view of Fig. 25C along a A-A cutting line, Fig. 25E is a schematic cross-sectional view of Fig. 25B along a B-B cutting line and Fig. 25F is a schematic bottom view. The difference between the third variance and the second variance resides in a hollow nasal dilator body 9000c of the third variance has a T stent 9005c connecting an inner surrounding wall of the hollow nasal dilator body 9000c corresponding to a first annular flange 9100c. The T stent 9005c has a first rib 9016a and a second rib 9016b. The first rib 9016a crosses an inner diameter of the hollow nasal dilator body 9000c and connecting the inner surrounding wall of the hollow nasal dilator body 9000c. One end of the second rib 9016b connects the inner surrounding wall of the hollow nasal dilator body 9000c and the other end of the second rib 9016b connects a middle portion of the first rib 9016a, as shown in Fig. 25F.

Please refer to Fig. 25G, during the inhalation of the user, the one-way flow resistance valve 9200c is deflected toward the second end opening 9002c under action of air flow pressure and the second rib 9016b of the T stent 9005c assists to increase deflection of the one-way flow resistance valve 9200c toward the second end opening 9002c to facilitate elimination of the inhalation flow resistance. Besides, the second rib 9016b may be as an umbrella stopper to effectively reduce flapping sound of the valve piece of the one-way flow resistance valve 9200c.

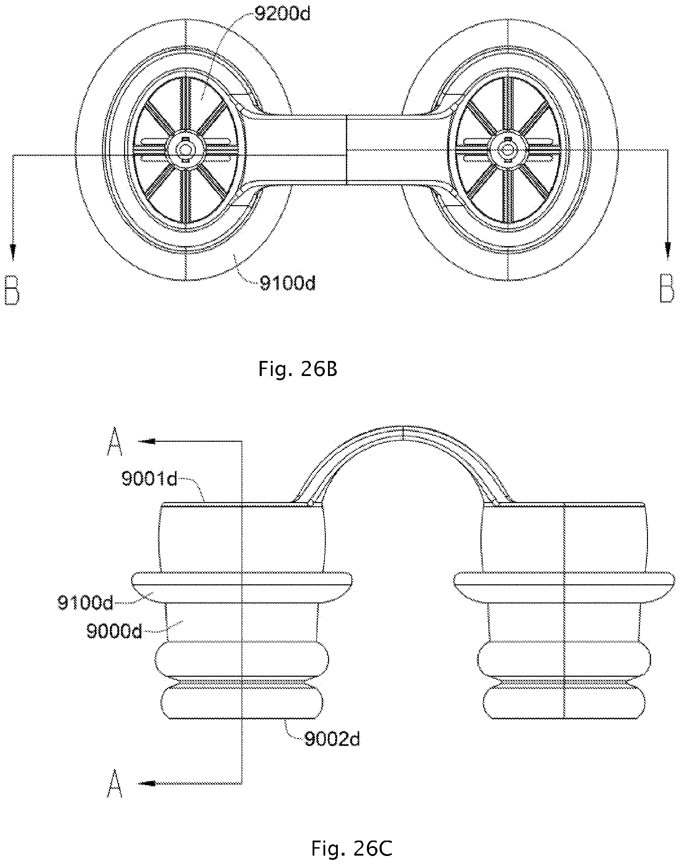

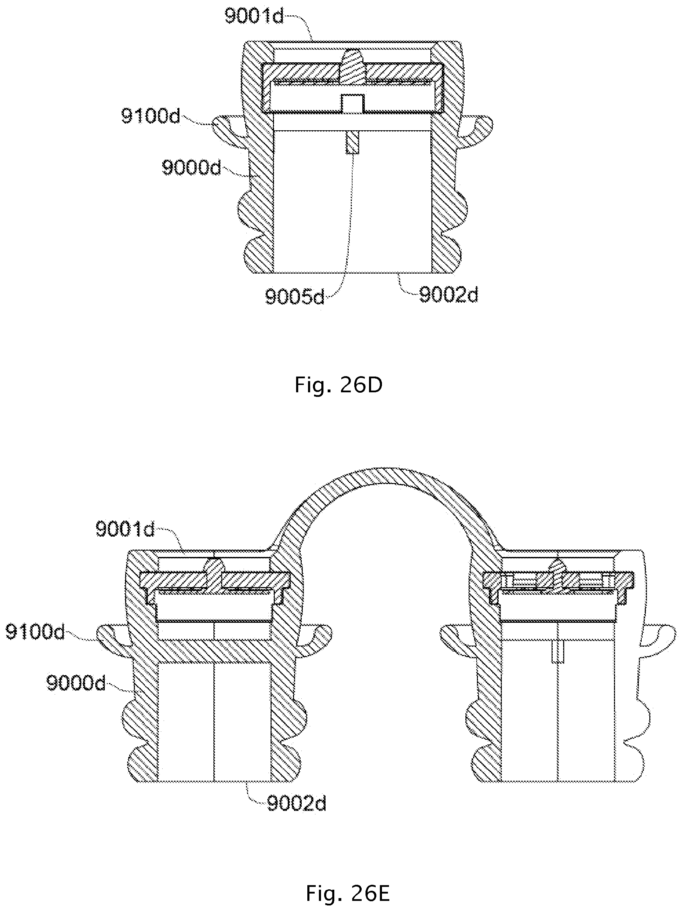

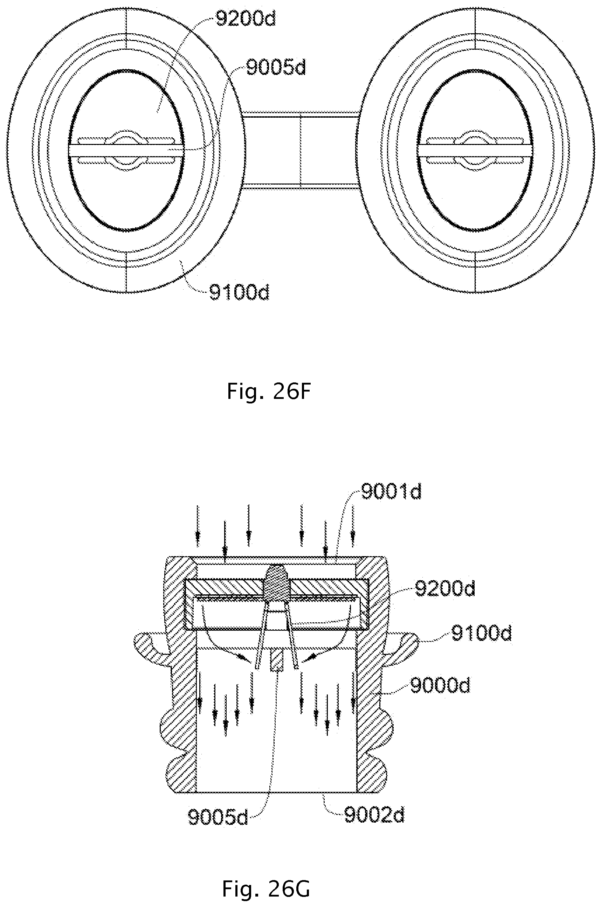

Fig. 26 is a fourth variance of the nasal dilator with an EPAP valve insert 9 of the ninth embodiment shown in Fig. 20. Fig. 26A is a schematic perspective partly cross-sectional view, Fig. 26B is a schematic top view, Fig. 26C is a schematic front view, Fig. 26D is a schematic cross-sectional view of Fig. 26C along a A-A cutting line, Fig. 26E is a schematic cross-sectional view of Fig. 26B along a B-B cutting line and Fig. 26F is a schematic bottom view. The difference between the fourth variance and the second variance resides in a hollow nasal dilator body 9000d has a single rib stent 9005d at an inner surrounding wall of the hollow nasal dilator body 9000d corresponding to a first annular flange 9100d. The single rib stent 9005d crosses an inner diameter of the hollow nasal dilator body 9000d and its two opposite ends connecting the inner surrounding wall of the hollow nasal dilator body 9000d, as shown in Fig. 26F.

Please refer to Fig. 26G, during the inhalation of the user, a one-way flow resistance valve 9200d is deflected toward the second end opening 9002d of the hollow nasal dilator body 9200d under action of air flow pressure, and the single rib stent 9005d assists to increase deflection of the one-way flow resistance valve 9200d toward the second end opening 9002d to facilitate elimination of the inhalation flow resistance. Besides, the single rib stent 9005d may be as an umbrella stopper to effectively reduce flapping sound of the valve piece of the one-way flow resistance valve 9200d.

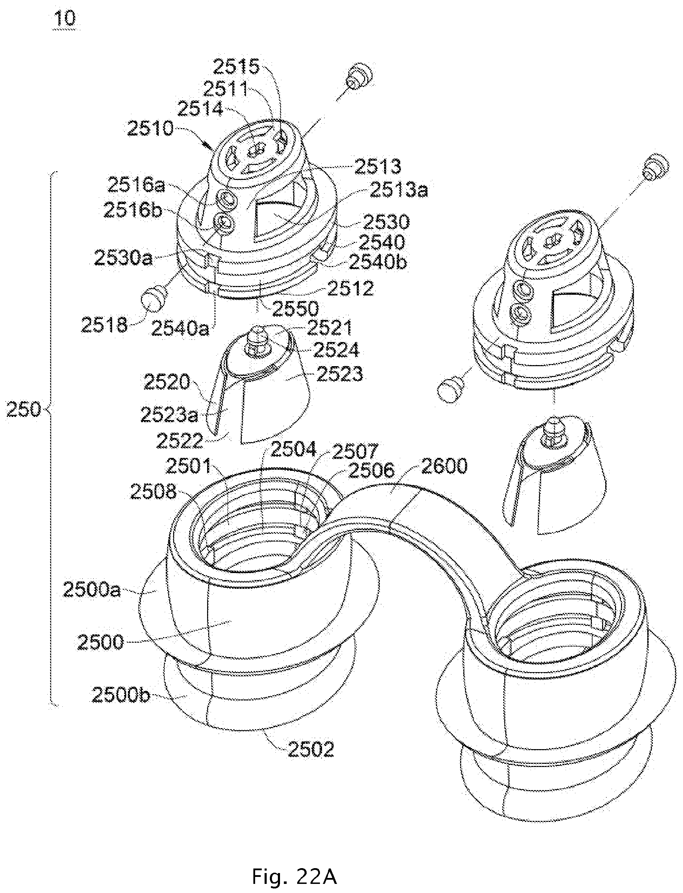

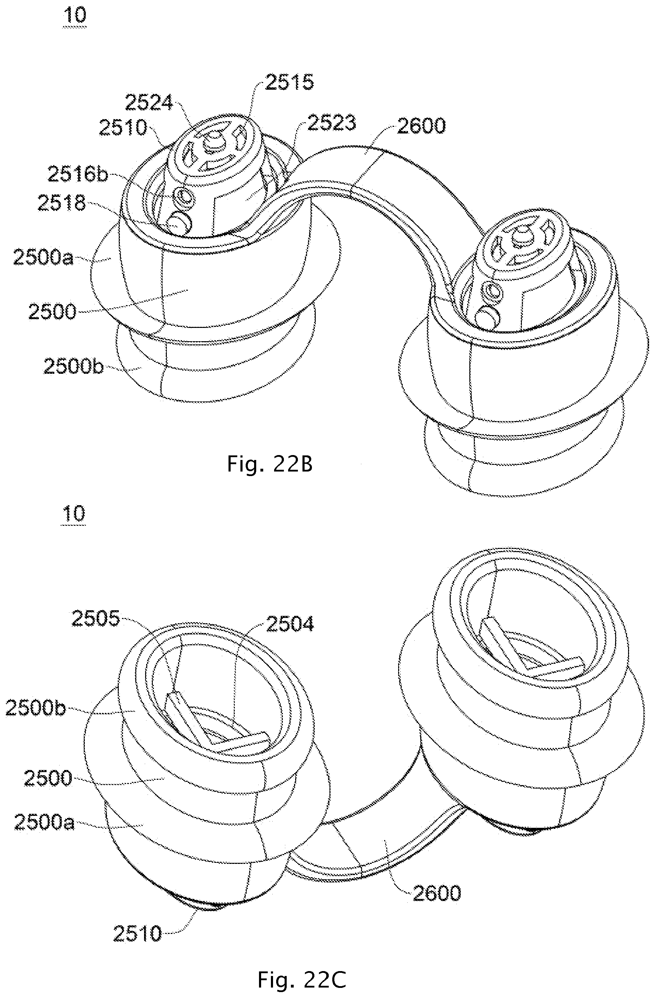

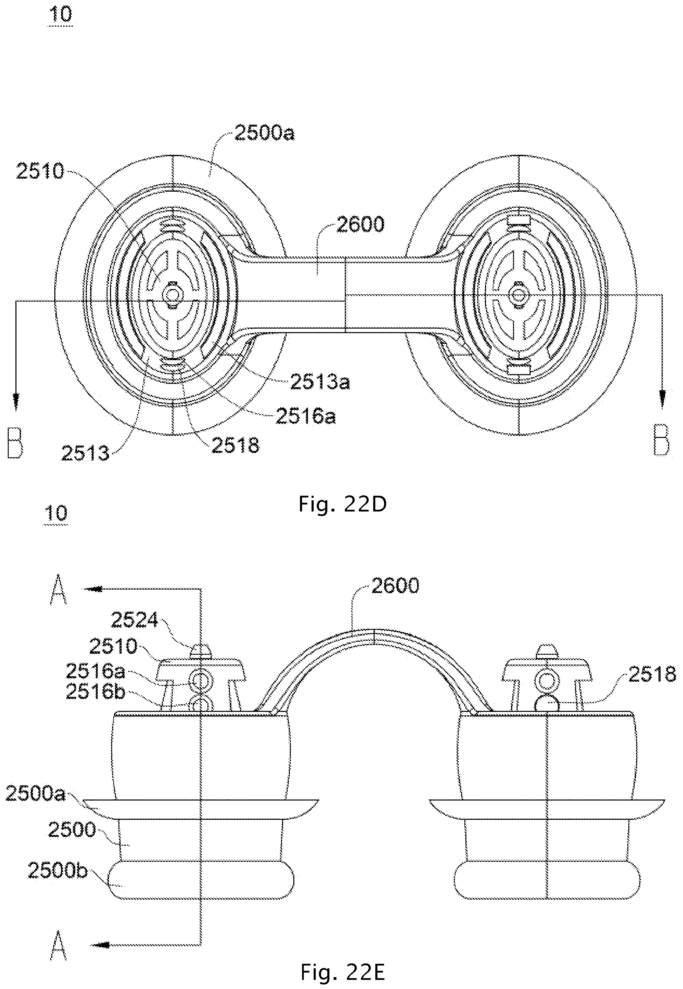

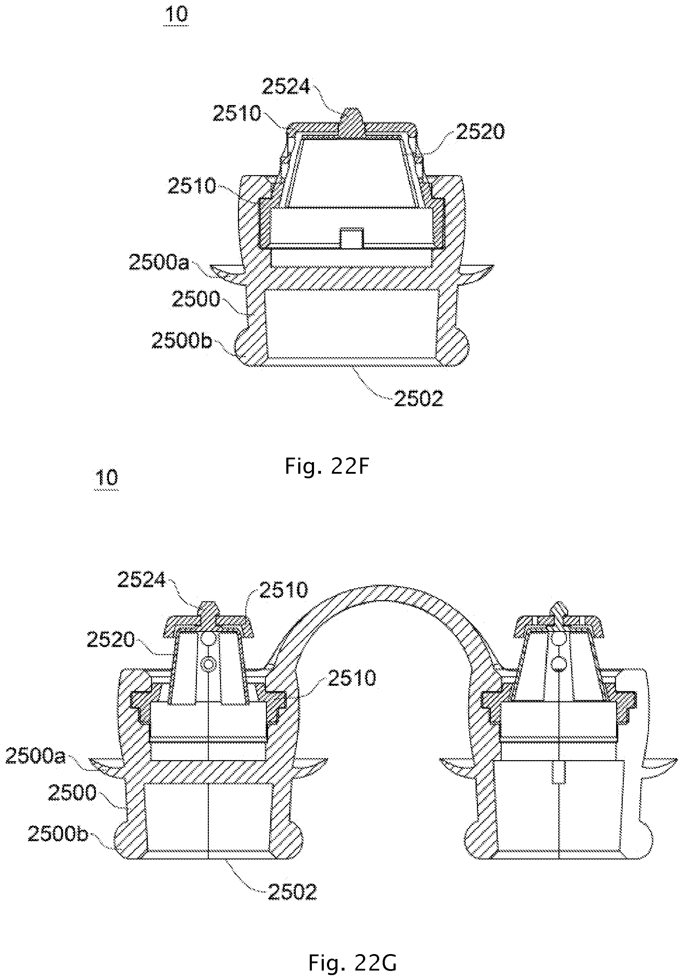

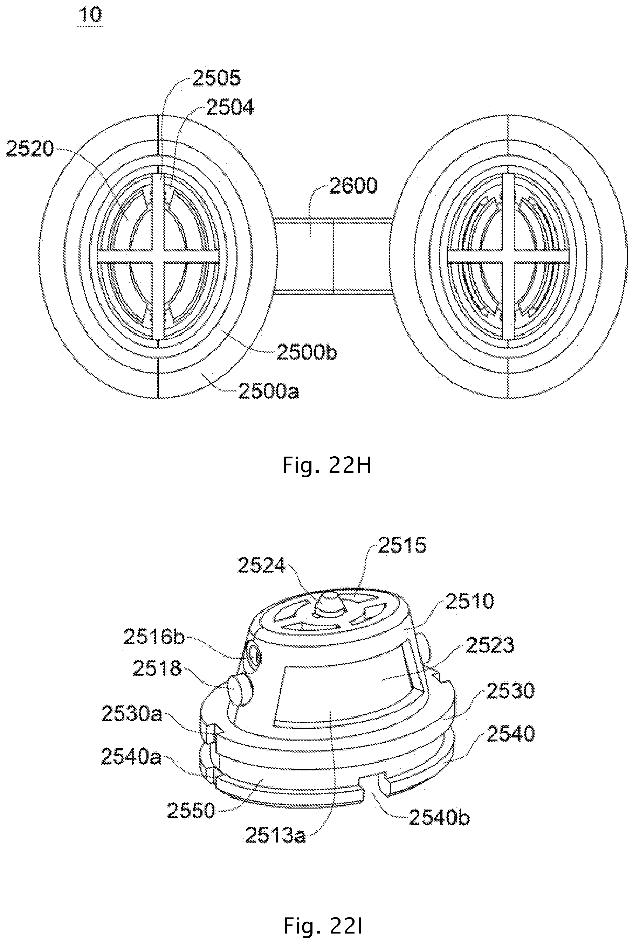

Fig. 22A through Fig. 22H are various schematic views of a nasal dilator with an EPAP valve insert 10 according to a tenth embodiment of this invention. Fig. 22A is a schematic perspective exploded view, Fig. 22B is a schematic perspective front view, Fig. 22C is a schematic perspective bottom view, Fig. 22D is a schematic top view, Fig. 22E is a schematic front view, Fig. 22F is a schematic cross-sectional view of Fig. 22E along a A-A cutting line, Fig. 22G is a schematic cross-sectional view of Fig. 22D along a B-B cutting line and Fig. 22H is a schematic bottom view. The nasal dilator with an EPAP valve insert 10 includes a pair of nasal dilator unit 250. Each of the pair of nasal dilator unit 250 includes a hollow nasal dilator body 2500, a valve seat 2510, and a one-way flow resistance valve in a three-dimensional trapezoid configuration 2520. The nasal dilator with an EPAP valve insert 10 further comprises a jointing piece 2600 for connecting the pair of nasal dilator unit 250. The hollow nasal dilator body 2500 has a first end opening 2501 and a second end opening 2502 opposite to each other. The hollow nasal dilator body 2500 from the first end opening 2501 longitudinally extends to the second end opening 2502. A first annular flange 2500a is provided around a middle portion of an outer surrounding wall of the hollow nasal dilator body 2500 and a second annular flange 2500b is provided around the outer surrounding wall at the second end opening 2502. An outer diameter of the first annular flange 2500a is larger than that of the second annular flange 2500b. When the user wears the nasal dilator with an EPAP valve insert 10, as needed, a portion of the hollow nasal dilator body 2500 from the first annular flange 2500a to the first end opening 2501 may be exposed outside the user's nose and let the first annular flange 2500a resisting against an outer portion of the user's nose. Or, as needed, the first annular flange 2500a may be placed inside the nasal cavity to increase the degree of engagement between the hollow nasal dilator body 2500 and the nasal cavity. Please refer to Fig. 22C and Fig. 22H, an annular step surface 2504 is provided around an inner surrounding wall of the hollow nasal dilator body 2500 and at a height level slightly higher than the first annular flange 2500a, and a cross stent 2505 is immediately disposed under the annular step surface 2504 so that the cross stent 2505 is approximately at the same height level as the first annular flange 2500a and connecting the inner surrounding wall of the hollow nasal dilator body 2500 to form a plate inside the hollow nasal dilator body 2500 in order that the one-way flow resistance valve 2500 is dropped off from the valve seat 2510 during the inhalation of the user, the cross stent 2505 may prevent the one-way flow resistance valve 2520 from dropping in the nasal cavity. A pair of first engaging block 2506 is provided at the inner surrounding wall of the hollow nasal dilator body 2500 and on a top of the annular step surface 2504. A pair of second engaging block 2507 is provided at the inner surrounding wall of the hollow nasal dilator body 2500 near the first end opening 2501 and corresponding to the first engaging block 2506. The first engaging blocks 2506 and the second engaging blocks 2507 engagely connect the valve seat 2510 to assist the valve seat 2510 firmly assembled to the hollow nasal dilator body 2500. Please refer to Fig. 22A, the valve seat 2510 has a hollow valve seat body in a three-dimensional trapezoid configuration and a profile of an inner wall of the hollow valve seat body is mated with the shape of the one-way flow resistance valve 2520. The hollow valve seat body has a top side 2511 and a bottom side 2512 opposite to the top side 2511 and a three-dimensional conical middle portion 2513 provided between the top side 2511 and the bottom side 2512. The top side 2511 has a valve connecting portion 2514 at its center to connect the one-way flow resistance valve 2520. A plurality of first air vent 2515 are provided along a periphery of the top side 2511, and air from outside environment may enter the valve seat 2510 through the first air vents 2515. The three-dimensional conical middle portion 2513 is provided with a pair of trapezoid through holes 2513a opposite to each other, and a pair of second air vent 2516a, 2516b longitudinally arranged at a partial outer surrounding wall of the three-dimensional conical middle portion 2513. A pair of third air vent (not shown) longitudinally arranged are provided at another outer surrounding wall of the three-dimensional conical middle portion 2513 corresponding to the second air vents 2516a, 2516b. One or more of the second air vents 2516a, 2516b may be blocked with a plug 2518 to facilitate adjustment of air flow resistance of the nasal dilator with an EPAP valve insert 10. The trapezoid through holes 2513 a, the second air vents 2516a, 2516b and the third air vents annularly provided around the three-dimensional conical middle portion 2513. The hollow valve seat body has a first hollow annular piece 2530 and a second hollow annular piece 2540 provided at its bottom side 2512 and a hollow middle portion 2550 connecting the first hollow annular piece 2530 and a second hollow annular piece 2540. A pair of opposing recess 2530a is provided at an outer surrounding wall of the first hollow annular piece 2530 for engaging the second engaging blocks 2507 at the inner surrounding wall of the hollow nasal dilator body 2500. A pair of opposing recess 2540a is provided at an outer surrounding wall of the second hollow annular piece 2540 for engaging the first engaging blocks 2506 at the inner surrounding wall of the hollow nasal dilator body 2500. A pair of opposing recess 2540b spaced from the recesses 2540a is provided at the outer surrounding wall of the second hollow annular piece 2540. A pair of protrusion 2508 are provided inside the hollow nasal dilator body 2500 at the top of the annular step surface 2504 for engaging the pair of the recess 2540b. According to the tenth embodiment, the recesses 2530a, 2540a, 2540b of the valve seat 2510 and the engaging blocks 2506, 2507 and the protrusions 2508 provided at the inner surrounding wall of the hollow nasal dilator body 2500 constitute a foolproof mechanism to assist the valve seat 2510 and the one-way flow resistance valve 2520 to correctly assemble inside the hollow nasal dilator body 2500. The one-way flow resistance valve 2520 has a top side 2521, a bottom side 2522 and a hollow conical middle portion 2325 provided between the top side 2521 and the bottom side 2522. The top side 2521 has an engaging part 2524 for engagely connecting the valve connecting portion 2514. The bottom side 2522 forms an opening. The hollow conical middle portion 2523 is provided with an opposing trapezoid opening 2523a from the top side 2521 longitudinally extending to the bottom side 2522. The one-way flow resistance valve 2520 may be a check valve. The one-way flow resistance valve 2520 may be made of flexible material to facilitate deformation of the one-way flow resistance valve 2520 under action of air flow pressure. Fig. 22I shows a schematic perspective view of an assembly of the valve seat 2510 and the one-way flow resistance valve 2520, the trapezoid openings 2523a of the one-way flow resistance valve 2520 are toward the air vents 2516a, 2516b, while the trapezoid through holes 2513a of the valve seat 2510 are toward parts of the hollow conic middle portion 2523 of the one-way flow resistance valve 2520. In other words, the trapezoid openings 2523a fluid communicate with the second air vents 2516a, 2516b, while the parts of the hollow conic middle portion 2523 deformably cover the trapezoid through holes 2513a.

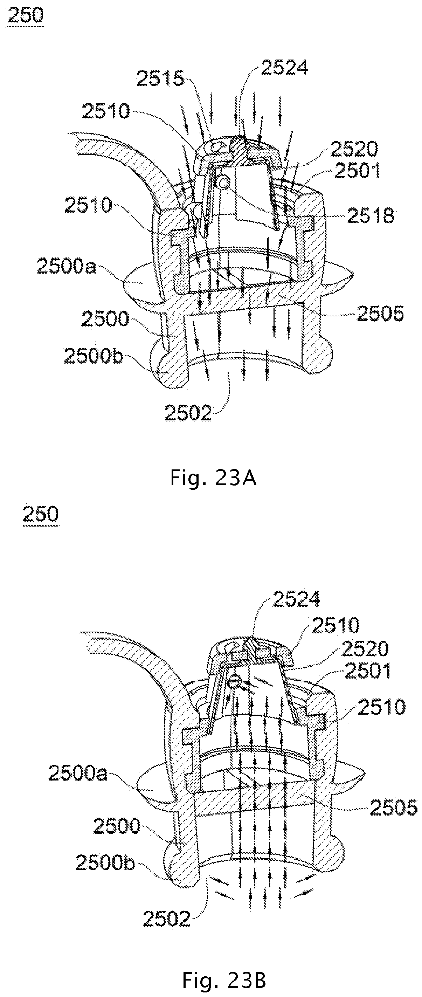

Please refer to Fig. 23A, Fig. 23B and Fig. 23C, during the inhalation of the user, as shown in Fig. 23A, the one-way flow resistance valve 2520 is in an open state to allow air flow pass it and entering the hollow nasal dilator body 2500 to let the user easily breathe. Meanwhile, air flow pressure acts at the one-way flow resistance valve 2520 to force the one-way flow resistance valve 2520 deformed toward a direction away from the top side 2511 and the three-dimensional conical middle portion 2513 of the valve seat 2510 so as to form gaps between the one-way flow resistance valve 2520 and the valve seat 2510 to establish fluid channels communicating the outside environment. During the exhalation of the user, air flow from the second end opening 2502 of the hollow nasal dilator body 2500 flows to the first end opening 2501, and the one-way flow resistance valve 2520 is in a closed state to prevent air flow from passing it. The air flow pressure forces the one-way flow resistance valve 2520 returning to its original state from the deformation and resisting against the inner surrounding wall of the valve seat 2510 to close the first air vents 2515 and the trapezoid through holes 2513a, as shown in Fig. 23B and Fig. 23C. Meanwhile, air flow inside the hollow nasal dilator body 2500 only can pass the second air vents 2516a unblocked by the plug 2518 and flowing outside of the nasal cavity so that the exhalation flow resistance of the user is increased relative to the inhalation flow resistance of the user. Therefore, a positive pressure is generated inside the upper airway of the user to facilitate the upper airway keep patency to stabilize soft tissue inside the oral cavity of the user to prevent the soft tissue from collapsing.

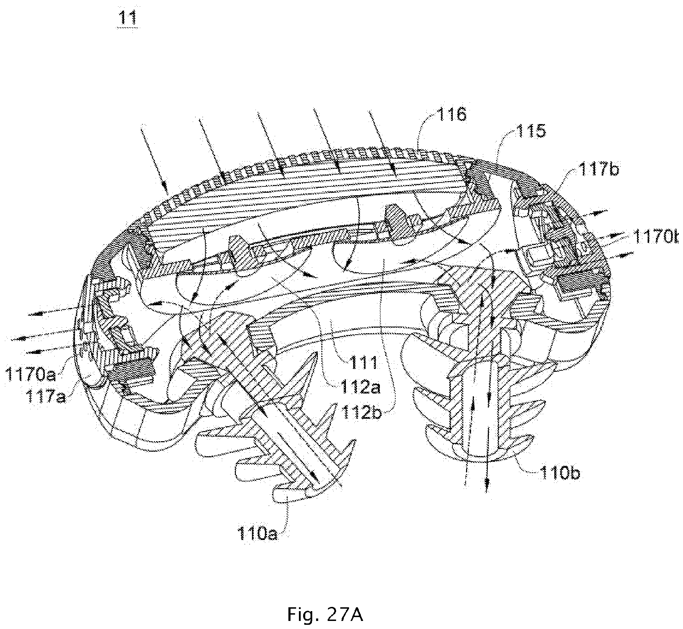

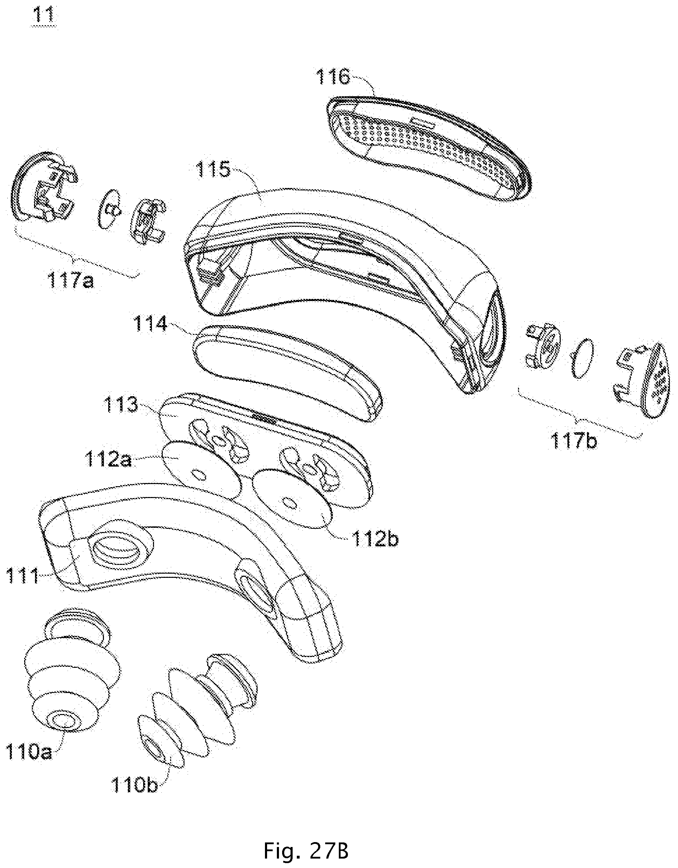

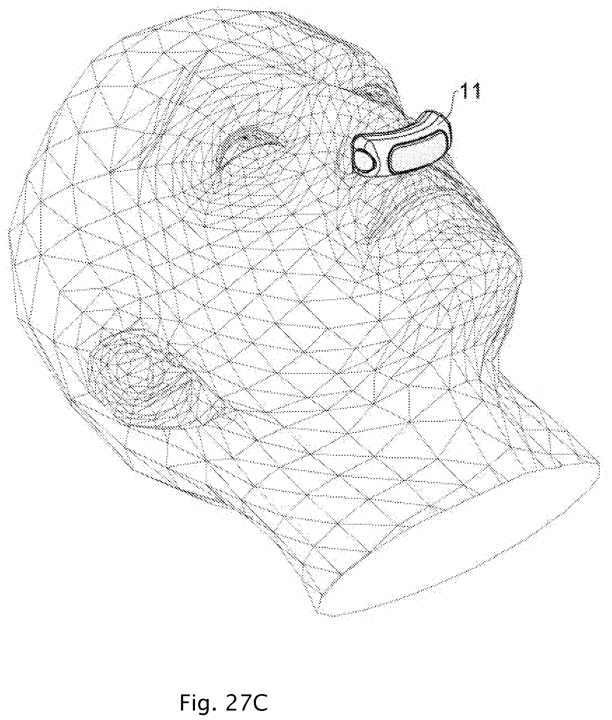

Fig. 27A is a schematic cross-sectional view of a nasal dilator with an EPAP valve insert 11 according to an eleventh embodiment of this invention. Fig. 27B is a schematic perspective exploded view of Fig. 27A. Fig. 27C shows a schematic use state of the nasal dilator with an EPAP valve insert 11 of the eleventh embodiment. The nasal dilator with an EPAP valve insert 11 includes a first hollow nasal dilator body 110a, a second hollow nasal dilator body 110b, a rear cover 111, a first one-way flow resistance valve 112a, a second one-way flow resistance valve 112b, a valve seat 113, a filter 114, a front cover 115, a mesh cover 116, a first exit flow adjuster 117a and a second exit flow adjuster 117b. Each of the first exit flow adjuster 117a and the second exit flow adjuster 117b has a one-way valve to allow air flow from the first hollow nasal dilator body 110a and the second hollow nasal dilator body 110b flows to outside environment. During the inhalation of the user, the first one-way flow resistance valve 112a and the second one-way flow resistance valve 112b allow air flow passing them. As air flow direction represented by solid arrows shown in Fig. 27A, air flow from the outside environment passing the mesh cover 116 and the front cover 115 enters the nasal dilator with an EPAP valve insert 11 and passing the first one-way flow resistance valve 112a and the second one-way flow resistance valve 112b assembled to the valve seat 113 after filtered by the filter 114, then respectively flows inside the first hollow nasal dilator body 110a and the second hollow nasal dilator body 110b to let the user easily breathe. During the exhalation of the user, the first one-way flow resistance valve 112a and the second one-way flow resistance valve 112b prevent air flow from passing them so that air flow inside the first hollow nasal dilator body 110a and the second hollow nasal dilator body 110b only can pass the first exit flow adjuster 117a and the second exit flow adjuster 117b and flowing outside of the nasal cavity, as air flow direction represented by dotted arrows shown in Fig. 27A, so that the exhalation flow resistance of the user is increased relative to the inhalation flow resistance of the user. Therefore, a positive pressure is generated inside the upper airway of the user to facilitate the upper airway keep patency to stabilize soft tissue inside the oral cavity of the user to prevent the soft tissue from collapsing. Moreover, a size of an opening 1170a and an opening 1170b may be adjusted by rotating the first exit flow adjuster 117a and the second exit flow adjuster 117b so that the exhalation flow resistance may be adjusted. Then, the positive pressure inside the first hollow nasal dilator body 110a and the second hollow nasal dilator body 110b may be adjusted.

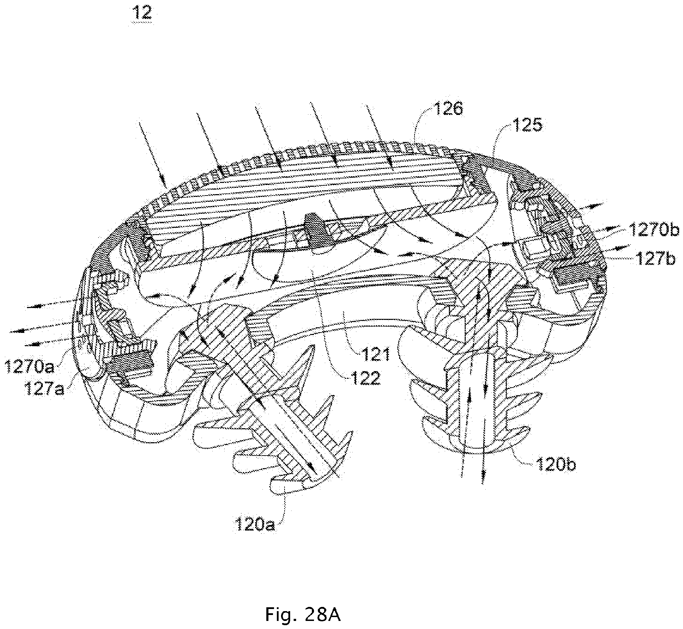

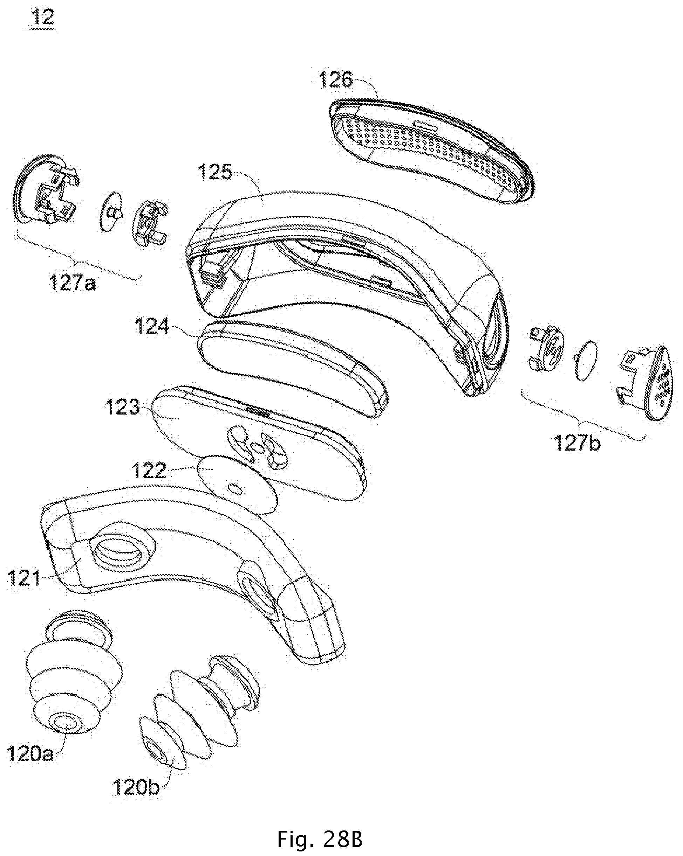

Fig. 28 is a variance of the eleventh embodiment, in which Fig. 28A is a schematic cross-sectional view of this variance and Fig. 28B is a schematic exploded view of Fig. 28A. The difference between this variance and the eleventh embodiment resides in a common one-way flow resistance valve replaces the two one-way flow resistance valves respectively used in the left and right nasal cavities. In this variance, a nasal dilator with an EPAP valve insert 12 includes a first hollow nasal dilator body 120a, a second hollow nasal dilator body 120b, a rear cover 121, a common one-way flow resistance valve 122, a valve seat 123, a filter 124, a front cover 125, a mesh cover 126, a first exit flow adjuster 127a and a second exit flow adjuster 127b. Each of the first exit flow adjuster 127a and the second exit flow adjuster 127b has a one-way valve to allow air flow from the first hollow nasal dilator body 120a and the second hollow nasal dilator body 120b flows to outside environment. During the inhalation of the user, the common one-way flow resistance valve 122 allows air flow passing it. As air flow direction represented by solid arrows shown in Fig. 28A, air flow from the outside environment passing the mesh cover 126 and the front cover 125 enters the nasal dilator with an EPAP valve insert 12 and passing the common one-way flow resistance valve 122 assembled to the valve seat 123 after filtered by the filter 124, then respectively flows inside the first hollow nasal dilator body 120a and the second hollow nasal dilator body 120b to let the user easily breathe. During the exhalation of the user, the common one-way flow resistance valve 122 prevents air flow from passing it so that air flow inside the first hollow nasal dilator body 120a and the second hollow nasal dilator body 120b only can pass the first exit flow adjuster 127a and the second exit flow adjuster 127b and flowing outside of the nasal cavity, as air flow direction represented by dotted arrows shown in Fig. 28A, so that the exhalation flow resistance of the user is increased relative to the inhalation flow resistance of the user. Therefore, a positive pressure is generated inside the upper airway of the user to facilitate the upper airway keep patency to stabilize soft tissue inside the oral cavity of the user to prevent the soft tissue from collapsing. Moreover, a size of an opening 1270a and an opening 1270b may be adjusted by rotating the first exit flow adjuster 127a and the second exit flow adjuster 127b so that the exhalation flow resistance may be adjusted. Then, the positive pressure inside the first hollow nasal dilator body 120a and the second hollow nasal dilator body 120b may be adjusted.

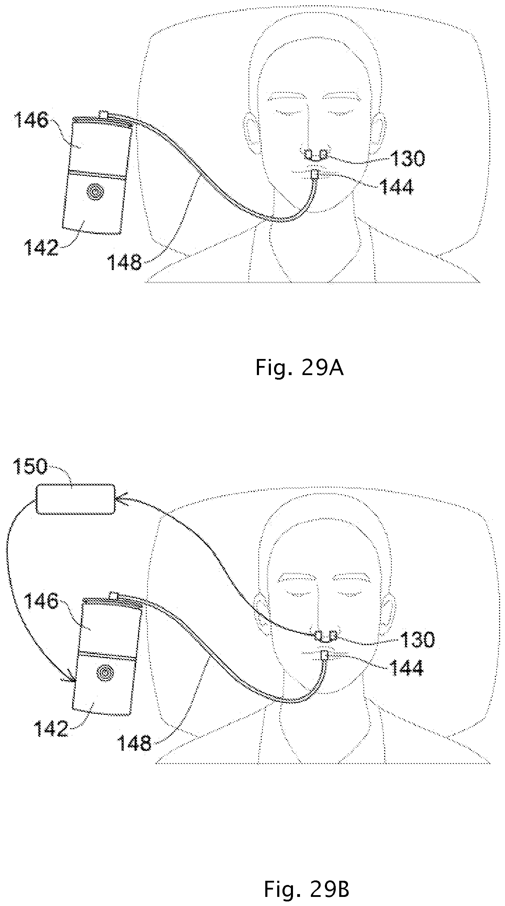

Fig. 29A and Fig. 29B are applications of a nasal dilator with an EPAP valve insert 130 of this invention. The nasal dilator with an EPAP valve insert 130 is combinable with a negative pressure sleep breathing therapy system for treating snoring and obstructive sleep apnea. Please refer to Fig. 29A, the negative pressure sleep breathing therapy system includes an electric console 142, an oral interface 144 and a saliva reservoir 146. The electric console 142 may provide negative pressure through a tube 148 and the oral interface 144 to deliver in an oral cavity of a user, and the saliva reservoir 146 collects saliva extracted from the oral cavity of the user via the tube 148. The negative pressure sleep breathing therapy system of US Patent No. 10,166,140 owned by the applicant of this invention may be adopted in this invention. And, the oral interfaces of US Patent Published No. 20140190489A1 and US Patent Published No. 20200352776A1 owned by the applicant of this invention may be used as the oral interface 144. Moreover, please refer to Fig. 29B, a sensing unit 150 though the nasal dilator with an EPAP valve insert 130 detects at least one user's physiological signal including temperature, humidity, air flow quantity, air flow pressure, breathing pattern and snoring, also may detect vibration and chemical materials inside the user's nasal passage. The sensing unit 150 transmits the detected physiological signal to the electric console 142 for computation with an algorithm, and the electric console 142 accordingly adjusts the negative pressure provided and then via the oral interface144 to control the pressure inside the user's oral cavity.

The above-mentioned embodiments of the present invention are exemplary and not intended to limit the scope of the present invention. Various variations or modifications made without departing from the spirit of this invention and achieving equivalent effects shall fall within the scope of claims of this invention. Please note after reading the above disclosure of this invention, a person skilled in the art of this invention should be able to easily understand that the various components of the above embodiments, such as the valve seat, the one-way flow resistance valve, the hollow nasal dilator body, etc. can be removed from the nasal dilator of this invention so that different valve seats can be replaced with each other, different one-way flow resistance valves can be replaced with each other, and different hollow nasal dilator bodies can be replaced with each other to realize the technical means of this invention and the desired effects. Furthermore, the hollow nasal dilator body of this invention can support and dilate the user's nasal passage, and the outer surrounding wall of the hollow nasal dilator body can be provided with at least one annular flange to further dilate the user's nasal passage and increase the degree of engagement between the hollow nasal dilator body and the nasal cavity. In addition, this invention may have various one-way flow resistance units that can provide different flow resistances by matching various valve seats with various one-way flow resistance valves. In order to facilitate quick and easy identification and selection of one-way flow resistance units that can provide the required airflow resistance, this invention further provides one-way flow resistance units with different flow resistances having their different colors. Or, different color marks are made on these one-way flow resistance units with different flow resistances to facilitate mapping different color codes to the one-way flow resistance units with different flow resistances, so that users can choose the one-way flow resistance unit they want according to the color code.

## Claims

1. A nasal dilator for treating breathing problem, comprising:
a hollow nasal dilator body having a first end opening and a second end opening opposing the first end opening, wherein the hollow nasal dilator body longitudinally extends from the first end opening to the second end opening and being adapted for insertion into a user's nostril with the second end opening placed within the user's nasal passage so that the hollow nasal dilator body supports and dilates the nasal passage; and
a one-way flow resistance unit assembled to the hollow nasal dilator body and in proximity to the first end opening and away from the second end opening, wherein the one-way flow resistance unit creates an exhalation flow resistance higher than an inhalation flow resistance such that an expiratory positive airway pressure is generated within the nasal passage.

2. The nasal dilator of claim 1, wherein the one-way flow resistance unit is removable and interchangeable from the nasal dilator.

3. The nasal dilator of claim 1, wherein the one-way flow resistance unit is able to adjust a ratio of the exhalation flow resistance to the inhalation flow resistance.

4. The nasal dilator of claim 1, wherein the one-way flow resistance unit comprises a one-way flow resistance valve and a valve seat, the one-way flow resistance valve is fastened in the valve seat, and the valve seat is assembled to the hollow nasal dilator body.

5. The nasal dilator of claim 4, wherein the one-way flow resistance valve has at least one radial cut.

6. The nasal dilator of claim 5, wherein the one-way flow resistance valve has at least one vent spaced from the radial cut.

7. The nasal dilator of claim 4, wherein the one-way flow resistance valve has at least one slit vent in a radial direction.

8. The nasal dilator of claim 7, wherein the one-way flow resistance valve has a cut between the slit vent and a periphery of the one-way flow resistance valve.

9. The nasal dilator of claim 7, wherein the one-way flow resistance valve has a multiple of slit vents spaced from each other and arranged along at least one radial direction.

10. The nasal dilator of claim 7, wherein the one-way flow resistance valve has a cut between at least one pair of the neighboring slit vents.

11. The nasal dilator of claim 4, wherein each of the one-way flow resistance valve and the valve seat is in a shape of a three-dimensional trapezoid.

12. The nasal dilator of claim 1, wherein the hollow nasal dilator body has at least one lateral vent in proximity to the first end opening.

13. The nasal dilator of claim 4, wherein the hollow nasal dilator body has a flow resistance structure unit in proximity to the first end opening.

14. The nasal dilator of claim 13, wherein the valve seat has a rotatable flow resistance adjustment unit with capability to change relative orientation between the flow resistance adjustment unit and the flow resistance structure unit to adjust the exhalation flow resistance.

15. The nasal dilator of claim 6, further comprising a rotatable baffle assembled between the valve seat and the one-way flow resistance valve for blocking a partial area of the vent.

16. The nasal dilator of claim 4, further comprising at least one stopper positioned at an inner surface of the hollow nasal dilator body in proximity to the first end opening to confine a bending angle of the one-way flow resistance valve upon a force from an inhalation flow.

17. The nasal dilator of claim 4, further comprising a stent assembled under the one-way flow resistance valve and connected to an inner surrounding wall of the hollow nasal dilator body, and the stent crosses a part or the whole of an inner radius of the hollow nasal dilator body.

18. The nasal dilator of claim 17, wherein the stent has one of the following configurations: cross shape, X shape, T shape and a singular rib.

19. The nasal dilator of claim 1, wherein the one-way flow resistance unit is provided with an individual color code based on its individual flow resistance.

20. The nasal dilator of claim 1, wherein an easy-to-remove means is formed along an inner surrounding wall of the hollow nasal dilator body in proximity to the second end opening to facilitate removal of a part of the hollow nasal dilator body via the easy-to-remove means.

21. The nasal dilator of claim 1, further comprising at least one sensing unit for detecting temperature, humidity, air flow pressure, breathing pattern, vibration, snoring and chemical materials inside the user's nasal passage.

22. A nasal dilator assembly, comprising two nasal dilators as claimed in claim 1 and a jointing piece connected between the two nasal dilators.

23. The nasal dilator assembly of claim 22, wherein the one-way flow resistance unit of each of the nasal dilator is able to adjust a ratio of the exhalation flow resistance to the inhalation flow resistance.

24. A sleep breathing therapy system, comprising:
a nasal dilator assembly as claimed in claim 22;
an electric console;
an oral interface; and
a sensing unit;
wherein the nasal dilator assembly is worn by a user's nose in order that an expiratory positive airway pressure is generated inside the user's nasal passage, the electric console provides a negative pressure via the oral interface delivering to the user's oral cavity such that a negative pressure environment is generated inside the user's oral cavity, the sensing unit detects at least one user's physiological signal including temperature, humidity, air flow pressure, breathing pattern, vibration, snoring and chemical materials inside the user's nasal passage, and the sensing unit transmits the detected physiological signal to the electric console for computation with an algorithm, and the electric console accordingly adjusts the negative pressure provided and then via the oral interface to control the pressure inside the user's oral cavity.
